(19) Europäisches Patentamt
European Patent Office
Office européen des brevets

(11) **EP 4 679 087 A1**

(12) **EUROPEAN PATENT APPLICATION**
published in accordance with Art. 153(4) EPC

(43) Date of publication:
**14.01.2026 Bulletin 2026/03**

(21) Application number: **24770769.8**

(22) Date of filing: **08.03.2024**

(51) International Patent Classification (IPC):
**G01N 33/50** (2006.01)

(52) Cooperative Patent Classification (CPC):
**G01N 33/50**

(86) International application number:
**PCT/JP2024/009043**

(87) International publication number:
**WO 2024/190663 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.03.2023 JP 2023038035**

(71) Applicant: **MEIJI CO., LTD**
**Chuo-ku**
**Tokyo 104-8306 (JP)**

(72) Inventors:
• **YASUDA Miyabi**
  **Hachioji-shi, Tokyo 192-0919 (JP)**
• **MORIFUJI Masashi**
  **Hachioji-shi, Tokyo 192-0919 (JP)**
• **TAKAHASHI Saori**
  **Hachioji-shi, Tokyo 192-0919 (JP)**

(74) Representative: **TBK**
**Bavariaring 4-6**
**80336 München (DE)**

(54) **BIOMARKER AND USE THEREOF**

(57)    An object of the present invention is to provide a method for evaluating intestinal barrier function in a subject, and in order to achieve the object, the present invention provides a method for evaluating intestinal barrier function in a subject, the method including the steps of: (1a) measuring a biomarker in a specimen derived from the subject to acquire a measurement value for the biomarker; (1b) comparing the measurement value acquired in step 1a with a reference value to acquire a comparison result; and (1c) evaluating intestinal barrier function in the subject on the basis of the comparison result acquired in step 1b, wherein the specimen is dry feces or wet feces, wherein when the specimen is dry feces, the biomarker includes one or more substances selected from Group A, and wherein when the specimen is wet feces, the biomarker includes one or more substances selected from Group B.

**Description**

TECHNICAL FIELD

The present invention relates to a biomarker and use thereof.

Specifically, the present invention relates to a method for evaluating intestinal barrier function in a subject, a biomarker for the evaluation, use of a biomarker for the evaluation, a kit for the evaluation, and a computer program for allowing a computer to execute the method.

The present invention also relates to a method for diagnosing a disease or symptom caused by reduction in intestinal barrier function in a subject, a biomarker for the diagnosis, use of a biomarker for the diagnosis, a kit for the diagnosis, and a computer program for allowing a computer to execute the method.

The present invention also relates to a method for treating a disease or symptom caused by reduction in intestinal barrier function in a subject.

The present invention also relates to a method for evaluating a subject's risk of developing a disease or symptom caused by reduction in intestinal barrier function, or probability of the subject being affected by the disease or symptom, a biomarker for the evaluation, use of a biomarker for the evaluation, a kit for the evaluation, and a computer program for allowing a computer to execute the method.

The present invention also relates to a method for acquiring data for diagnosing a disease or symptom caused by reduction in intestinal barrier function in a subject, a biomarker for the acquisition, use of a biomarker for the acquisition, a kit for the acquisition, and a computer program for allowing a computer to execute the method.

The present invention also relates to a method for screening for a substance or composition having an improving effect on intestinal barrier function or a preventing or ameliorating effect on a disease or symptom caused by reduction in intestinal barrier function, a biomarker for the screening, use of a biomarker for the screening, a kit for the screening, and a computer program for allowing a computer to execute the method.

The present invention also relates to a method for evaluating a candidate substance or candidate composition for an improving effect on intestinal barrier function or a preventing or ameliorating effect on a disease or symptom caused by reduction in intestinal barrier function, a biomarker for the evaluation, use of a biomarker for the evaluation, a kit for the evaluation, and a computer program for allowing a computer to execute the method.

The present invention also relates to a method for producing a food product or pharmaceutical composition.

BACKGROUND ART

The intestinal lumen is a space surrounded by a single layer of mucosal epithelial cells, and the intestinal lumen (the outside of the living body) and the inside of the living body are partitioned from each other via mucosal epithelial cells. Exposed to foreign bodies (e.g., harmful microorganisms, toxins, allergens) incorporated from the ambient environment into the intestinal lumen, the intestinal tract is an important organ in terms not only of absorption of nutrients but also of defense. The intestinal barrier function is a function to prevent foreign bodies (e.g., harmful microorganisms, toxins, allergens) incorporated from the ambient environment into the intestinal lumen from intruding into the living body.

The intestinal barrier function involves a physical barrier and a chemical barrier. The physical barrier is a barrier that serves literally as a physical obstacle to prevent the intrusion of foreign bodies. The physical barrier includes a mucus layer covering intestinal epithelial cells, glycocalyx, which is an assembly of sugar chains present on the surfaces of intestinal epithelial cells, and a tight junction structure, which is a cell-cell adhesion apparatus for intestinal epithelial cells. The chemical barrier is a molecular group that inhibits foreign body intrusion by exerting antibacterial activity, neutralization activity, and the like through causing chemical change to foreign bodies. The molecular group includes antibacterial peptides (e.g., defensin family molecules, Reg3 family molecules, lactoferrin, lysozyme) produced from intestinal epithelial cells such as Paneth cells. M cells, which incorporate an antigen and present it to dendritic cells, and immunoglobulin A (IgA), which is secreted into the intestinal tract to prevent foreign body intrusion by exerting neutralization activity, are each a kind of chemical barrier.

Reduction in intestinal barrier function causes various diseases or symptoms (e.g., enteritis, allergic diseases, psychiatric diseases, non-alcoholic fatty liver diseases, type II diabetes mellitus, metabolic syndrome, adiposity).

Lactulose/mannitol test (sugar tolerance test) is known as a testing method for intestinal barrier function. Intestinal permeability is tested in the lactulose/mannitol test, and increase in intestinal permeability is indicative of deterioration of intestinal barrier function (in particular, physical barrier functions including the tight junction structure). The lactulose/mannitol test has drawbacks including forcing test subjects to have long confinement time and fasting time and laying burdens on the digestive tracts of test subjects by sugar alcohol to be ingested.

Blood zonulin levels (Non Patent Literature 1), which are known to relate to intestinal barrier function, are used for a testing method for evaluating intestinal barrier function in some cases, but blood zonulin levels vary from day to day, and the use has problems including low reproducibility (Non Patent Literature 2).

No biomarker that enables simple and highly accurate evaluation of intestinal barrier function has been known.

CITATION LIST

NON PATENT LITERATURE

Non Patent Literature 1: Fasana A et al., Zonulin, "a newly discovered modulator of intestinal permeability, and its expression in coeliac disease.", THE LANCET., 2000; 355: pp. 1518-1519
Non Patent Literature 2: Aristo Vojdani et al., "Fluctuation of zonulin levels in blood vs stability of antibodies.", World Journal of Gastroenterology., 2017; 23(31): pp. 5669-5679

SUMMARY OF INVENTION

TECHNICAL PROBLEM

An object of the present invention is to provide a method for evaluating intestinal barrier function in a subject, a biomarker for the evaluation, use of a biomarker for the evaluation, a kit for the evaluation, and a computer program for allowing a computer to execute the method.
Another object of the present invention is to provide a method for diagnosing a disease or symptom caused by reduction in intestinal barrier function in a subject, a biomarker for the diagnosis, use of a biomarker for the diagnosis, a kit for the diagnosis, and a computer program for allowing a computer to execute the method.
Another object of the present invention is to provide a method for treating a disease or symptom caused by reduction in intestinal barrier function in a subject.
Another object of the present invention is to provide a method for evaluating a subject's risk of developing a disease or symptom caused by reduction in intestinal barrier function, or probability of the subject being affected by the disease or symptom in a subject, a biomarker for the evaluation, use of a biomarker for the evaluation, a kit for the evaluation, and a computer program for allowing a computer to execute the method.
Another object of the present invention is to provide a method for acquiring data for diagnosing a disease or symptom caused by reduction in intestinal barrier function in a subject, a biomarker for the acquisition, use of a biomarker for the acquisition, a kit for the acquisition, and a computer program for allowing a computer to execute the method.
Another object of the present invention is to provide a method for screening for a substance or composition having an improving effect on intestinal barrier function or a preventing or ameliorating effect on a disease or symptom caused by reduction in intestinal barrier function, a biomarker for the screening, use of a biomarker for the screening, a kit for the screening, and a computer program for allowing a computer to execute the method.
Another object of the present invention is to provide a method for evaluating a candidate substance or candidate composition for an improving effect on intestinal barrier function or a preventing or ameliorating effect on a disease or symptom caused by reduction in intestinal barrier function, a biomarker for the evaluation, use of a biomarker for the evaluation, a kit for the evaluation, and a computer program for allowing a computer to execute the method.
Another object of the present invention is to provide a method for producing a food product or a pharmaceutical composition.

SOLUTION TO PROBLEM

In the present invention, Group A consists of substances listed in Table 1.

[Table 1-1]

| Table 1 (Group A) | |
| --- | --- |
| No. | Substance Name |
| 1 | Formylanthranilic acid |
| 2 | Diethanolamine |
| 3 | Glycerol |
| 4 | 5,6-Dihydroxyindole |
| 5 | Formiminoglutamic acid |

(continued)

| Table 1 (Group A) | |
|---|---|
| No. | Substance Name |
| 6 | Leucine |
| 7 | Myristoleic acid |
| 8 | 3-Aminopropane-1,2-diol |
| 9 | 4-Guanidinobutyric acid |
| 10 | Phenlyalanine |
| 11 | 2'-Deoxyadenosine and 5'-Deoxyadenosine |
| 12 | Lipoamide |
| 13 | 3-Guanidinopropionic acid |
| 14 | Acetanilide |
| 15 | Histidine |
| 16 | $\gamma$-glutamyl-2-aminobutyric acid |
| 17 | Methionine sulfoxide |
| 18 | Phenylhydrazine |
| 19 | Tryptophan |
| 20 | Tyrosine |
| 21 | Hydroxyindole |
| 22 | Alanine |
| 23 | p-Aminobenzoic acid |
| 24 | Penicillamine |
| 25 | 2-Cyanopyridine |
| 26 | Glutamine |
| 27 | 2,4-Diaminobutyric acid |
| 28 | Trigonelline |
| 29 | 2-Methylserine |
| 30 | Glycylglycine (Gly-Gly) |
| 31 | N,N-Dimethylhistidine |
| 32 | Valine |
| 33 | N-Acetylphenylalanine |
| 34 | Isoleucine |
| 35 | Methionine |
| 36 | $\gamma$-glutamyl-valyl-glycine (y-Glu-Val-Gly) |
| 37 | N-Acetylaspartic acid |
| 38 | Threonic acid |

[Table 1-2]

| Table 1 (Continued) | |
|---|---|
| No. | Substance Name |
| 39 | Alloisoleucine |

(continued)

| Table 1 (Continued) | |
|---|---|
| No. | Substance Name |
| 40 | Arginine ethyl ester |
| 41 | 2,6-Diaminopimelic acid |
| 42 | Glucose 6-phosphate |
| 43 | Adenosine |
| 44 | Mucic acid |
| 45 | Thymidine |
| 46 | Glycocholic acid |
| 47 | 2-Deoxyribose 1-phosphate |
| 48 | Isoleucyl-prolyl-proline (Ile-Pro-Pro) |
| 49 | Sedoheptulose 7-phosphate |
| 50 | Fumaric acid |
| 51 | N-Acetylmethionine |
| 52 | Fructose 6-phosphate |
| 53 | Glutamylglutamic acid (Glu-Glu) |
| 54 | Cysteine |
| 55 | N-Acetylglucosamine 1-phosphate |
| 56 | Ribose 5-phosphate |
| 57 | Glucose 1-phosphate |
| 58 | 3'-Cytidylic acid (3'-CMP) and 2'-Cytidylic acid (2'-CMP) |
| 59 | Dihydroxyacetone phosphate |
| 60 | 4-Oxovaleric acid |
| 61 | 2-Deoxyglucose 6-phosphate |
| 62 | Glucosamine 6-phosphate |
| 63 | 3-Methyladipic acid |
| 64 | 2-Hydroxyquinoline |
| 65 | 7-(tert-butyl)-4-imino-1,2,3,4,5,6-hexahydropyrido[3,4-d]pyridazine-1,5-dione) |
| 66 | 2,4-dihydroxyheptadec-16-en-1-yl acetate |
| 67 | α-Aspartylphenylalanine |
| 68 | N1-[4-(1,3-Oxazol-5-yl)phenyl]cyclopropane-1-carboxamide |
| 69 | 1-(2,4-Dihydroxyquinolin-3-yl)ethan-1-one |
| 70 | 4-(Diethylamino)salicylaldehyde |
| 71 | Atenolol |
| 72 | 5-Hydroxyindole |

[Table 1-3]

| Table 1 (Continued) | |
|---|---|
| No. | Substance Name |
| 73 | 6-Methylquinoline |

(continued)

| Table 1 (Continued) | |
|---|---|
| No. | Substance Name |
| 74 | Guanine |
| 75 | Carboxyibuprofen |
| 76 | 3-Isopropylmalic acid |
| 77 | Bis(methylbenzylidene)sorbitol |
| 78 | 2-Furoic acid |
| 79 | Orotic acid |
| 80 | 2-Acetamido-N-β-aspartyl-2-deoxyhexopyranosylamine |
| 81 | Xanthosine |
| 82 | 1-propyl-1H-benzo[d]imidazole hydrobromide |
| 83 | 4-Phenolsulfonic acid |
| 84 | Xanthohumol |
| 85 | Laurolactam |
| 86 | Adenine |
| 87 | 2'-Deoxyguanosine |
| 88 | 6-Hydroxypicolinic acid |
| 89 | D-(-)-Quinic acid |
| 90 | 4-{[(4,6-Dimethoxypyrimidin-2-yl)amino]methylidene}-2-phenyl-4,5-dihydro-1,3-oxazol-5-one |
| 91 | 4-[(6E)-3-Hydroxy-8,10-dimethyl-2-(methylamino)-6-dodecen-1-yl]phenol |
| 92 | Ethyl 2-cyano-3-(tetrahydro-3-thiophenylamino)acrylate |
| 93 | 3-Succinoylpyridine |
| 94 | Proline |
| 95 | L-threo-3-Phenylserine |
| 96 | (2,4-Diamino-5-pyrimidinyl)(3,4,5-trimethoxyphenyl)methanone |
| 97 | 3-Morpholino-4-tetrahydro-1H-pyrrol-1-ylcyclobut-3-ene-1,2-dione |
| 98 | 16-Hydroxyhexadecanoic acid |
| 99 | Syringic acid |
| 100 | Tranexamic acid |
| 101 | 4-(tert-butyl)phenyl 3,5-dimethylisoxazole-4-carboxylate |

[Table 1-4]

| Table 1 (Continued) | |
|---|---|
| No. | Substance Name |
| 102 | 3-Methylhistidine |
| 103 | 5-Methoxyindole |
| 104 | Perillic acid |
| 105 | 6-Methyl[1,2,4]triazolo[4,3-b]pyridazin-8-ol |
| 106 | Piperonylonitrile |
| 107 | N 1-(5-Chloro-2-hydroxyphenyl)-2-(morpholinomethyl)benzamide |

(continued)

| Table 1 (Continued) | |
|---|---|
| No. | Substance Name |
| 108 | N-Methylhistamine |
| 109 | Dibutylone |
| 110 | (-)-Epigallocatechin |
| 111 | Myriocin |
| 112 | 4-Indolecarbaldehyde |
| 113 | Uracil |
| 114 | Dihydrothymine |
| 115 | 3-Methylglutaric acid |
| 116 | 1,5-Anhydro-D-glucitol |
| 117 | Valethamate |
| 118 | DL-Arginine |
| 119 | (R)-Equol |
| 120 | Theobromine |
| 121 | 1,5-Isoquinolinediol |
| 122 | 12-Hydroxydodecanoic acid |
| 123 | 1,3,5-trihydroxy-4-{[(2E)-3-(3-hydroxy-4-methoxyphenyl)prop-2-enoyl]oxy}cyclohexane-1-carboxylic acid |
| 124 | 3-Hydroxypicolinic acid |
| 125 | Theophylline |
| 126 | 3,4-Dihydroxybenzaldehyde |
| 127 | Acetylcholine |
| 128 | Tridecylic acid |

In the present invention, Group B consists of substances listed in Table 2.

[Table 2-1]

| Table 2 (Group B) | |
|---|---|
| No. | Substance Name |
| 1 | Formylanthranilic acid |
| 2 | Glycerol |
| 3 | Formiminoglutamic acid |
| 4 | 5,6-Dihydroxyindole |
| 5 | 3-Aminopropane-1,2-diol |
| 6 | 4-Guanidinobutyric acid |
| 7 | 2'-Deoxyadenosine and 5'-Deoxyadenosine |
| 8 | Acetanilide |
| 9 | Pyrophosphate |
| 10 | Myristoleic acid |
| 11 | Diethanolamine |
| 12 | Thiaproline |

(continued)

| Table 2 (Group B) | |
|---|---|
| No. | Substance Name |
| 13 | Hydroxyindole |
| 14 | 2,6-Diaminopimelic acid |
| 15 | p-Aminobenzoic acid |
| 16 | Lipoamide |
| 17 | Phenylhydrazine |
| 18 | 3-Guanidinopropionic acid |
| 19 | N-Acetylaspartic acid |
| 20 | 2,4-Diaminobutyric acid |
| 21 | γ-glutamyl-2-aminobutyric acid |
| 22 | Cysteine |
| 23 | Glycerol 3-phosphate |
| 24 | Glucose 6-phosphate |
| 25 | Mucic acid |
| 26 | Trigonelline |
| 27 | N-Carbamoylaspartic acid |
| 28 | Threonic acid |
| 29 | Glycocholic acid |
| 30 | Isobutyric acid and Butyric acid |
| 31 | 2-Deoxyribose 1-phosphate |
| 32 | Arginine ethyl ester |
| 33 | N-Methylglutamic acid |
| 34 | Pterin |
| 35 | N-Acetylphenylalanine |
| 36 | Ribulose 5-phosphate |
| 37 | Fructose 6-phosphate |
| 38 | Sedoheptulose 7-phosphate |

[Table 2-2]

| Table 2 (Continued) | |
|---|---|
| No. | Substance Name |
| 39 | N-Acetylglucosamine 1-phosphate |
| 40 | Ribose 5-phosphate |
| 41 | 3'-Cytidylic acid (3'-CMP) and 2'-Cytidylic acid (2'-CMP) |
| 42 | Dihydroxyacetone phosphate |
| 43 | 4-Oxovaleric acid |
| 44 | Propranolol |
| 45 | 2-Deoxyglucose 6-phosphate |
| 46 | 6-Methylquinoline |

(continued)

| Table 2 (Continued) | |
|---|---|
| No. | Substance Name |
| 47 | N1-[4-(1,3-Oxazol-5-yl)phenyl]cyclopropane-1-carboxamide |
| 48 | 2-Hydroxyquinoline |
| 49 | 3-Methyladipic acid |
| 50 | L-threo-3-Phenylserine |
| 51 | 4-(Diethylamino)salicylaldehyde |
| 52 | 5-Methoxyindole |
| 53 | 7-(tert-butyl)-4-imino-1,2,3,4,5,6-hexahydropyrido[3,4-d]pyridazine-1,5-dione |
| 54 | 4-Indolecarbaldehyde |
| 55 | Carboxyibuprofen |
| 56 | Piperonylonitrile |
| 57 | 5-Hydroxyindole |
| 58 | Orotic acid |
| 59 | Glucose 1-phosphate |
| 60 | L-Alanyl-L-proline |
| 61 | 2-Acetamido-N-β-aspartyl-2-deoxyhexopyranosylamine |
| 62 | α-Aspartylphenylalanine |
| 63 | 6-Hydroxymelatonin |
| 64 | Xanthosine |
| 65 | Atenolol |
| 66 | (R)-Equol |
| 67 | 2,4-dihydroxyheptadec-16-en-1-yl acetate |
| 68 | Guanine |
| 69 | 1-(4-methyl-2-pyridyl)pyrrolidine-2,5-dione |
| 70 | Laurolactam |
| 71 | Olmesartan |

[Table 2-3]

| Table 2 (Continued) | |
|---|---|
| No. | Substance Name |
| 72 | Adenine |
| 73 | Uracil |
| 74 | 6-Methyl[1,2,4]triazolo[4,3-b]pyridazin-8-ol |
| 75 | 1-propyl-1H-benzo[d]imidazole hydrobromide |
| 76 | Pseudouridine |
| 77 | D-(-)-Quinic acid |
| 78 | Acetylcholine |
| 79 | 3-Isopropylmalic acid |
| 80 | 2-methoxy-5-(1H-1,2,4-triazol-5-yl)-4-(trifluoromethyl)pyridine |

(continued)

| Table 2 (Continued) | |
| --- | --- |
| No. | Substance Name |
| 81 | 3-Amino-2-naphthoic acid |
| 82 | Ornithine |
| 83 | 2'-Deoxyguanosine |
| 84 | Bis(methylbenzylidene)sorbitol |
| 85 | 2-Furoic acid |
| 86 | 4-(tert-butyl)phenyl 3,5-dimethylisoxazole-4-carboxylate |
| 87 | Pyridoxamine |
| 88 | Tridecylic acid |
| 89 | 3-Methylhistidine |
| 90 | Hexanoic acid |
| 91 | Azobenzene |
| 92 | (-)-Epigallocatechin |
| 93 | 3-(2-thienyl)-1,2,4-oxadiazole-5-carbohydrazide |
| 94 | DL-Lactic Acid |
| 95 | 6-Hydroxypicolinic acid |
| 96 | Proline |
| 97 | 1,3,5-trihydroxy-4-{[(2E)-3-(3-hydroxy-4-methoxyphenyl)prop-2-enoyl]oxy}cyclohexane-1-carboxylic acid |
| 98 | Prolylglycine |
| 99 | 4-[(6E)-3-Hydroxy-8,10-dimethyl-2-(methylamino)-6-dodecen-1-yl]phenol |

The present invention encompasses the following inventions.

[1] A method for evaluating intestinal barrier function in a subject, the method including the steps of:

(1a) measuring a biomarker in a specimen derived from the subject to acquire a measurement value for the biomarker;
(1b) comparing the measurement value acquired in step 1a with a reference value to acquire a comparison result; and
(1c) evaluating intestinal barrier function in the subject on the basis of the comparison result acquired in step 1b,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[2] A method for diagnosing a disease or symptom caused by reduction in intestinal barrier function in a subject, the method including the steps of:

(2a) measuring a biomarker in a specimen derived from the subject to acquire a measurement value for the biomarker;
(2b) comparing the measurement value acquired in step 2a with a reference value to acquire a comparison result; and
(2c) diagnosing the disease or symptom on the basis of the comparison result acquired in step 2b,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[3] A method for treating a disease or symptom caused by reduction in intestinal barrier function in a subject, the method including the steps of:

(3a) measuring a biomarker in a specimen derived from the subject to acquire a measurement value for the biomarker;
(3b) comparing the measurement value acquired in step 3a with a reference value to acquire a comparison result;
(3c) diagnosing the disease or symptom on the basis of the comparison result acquired in step 3b; and
(3d) administering a substance or composition for treating the disease or symptom to the subject if the subject is diagnosed to be affected by the disease or symptom,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[4] A method for evaluating a subject's risk of developing a disease or symptom caused by reduction in intestinal barrier function or probability of the subject being affected by the disease or symptom, the method including the steps of:

(4a) measuring a biomarker in a specimen derived from the subject to acquire a measurement value for the biomarker;
(4b) comparing the measurement value acquired in step 4a with a reference value to acquire a comparison result; and
(4c) evaluating the risk or the probability on the basis of the comparison result acquired in step 4b,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[5] A method for acquiring data for diagnosing a disease or symptom caused by reduction in intestinal barrier function in a subject, the method including the steps of:

(5a) measuring a biomarker in a specimen derived from the subject to acquire a measurement value for the biomarker; and
(5b) comparing the measurement value acquired in step 5a with a reference value to acquire a comparison result as the data,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[6] A method for screening for a substance or composition having an improving effect on intestinal barrier function or a preventing or ameliorating effect on a disease or symptom caused by reduction in intestinal barrier function, the method including the steps of:

(6a) administering a candidate substance or candidate composition to a subject having reduced intestinal barrier function;
(6b) measuring a biomarker in a specimen obtained from the subject after administration of the candidate substance or candidate composition to acquire a measurement value for the biomarker;
(6c) comparing the measurement value acquired in step 6b with a reference value to acquire a comparison result; and
(6d) evaluating the candidate substance or candidate composition for the improving effect on intestinal barrier function or the preventing or ameliorating effect on the disease or symptom on the basis of the comparison result acquired in step 6c,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[7] A method for evaluating a candidate substance or candidate composition for an improving effect on intestinal barrier function or a preventing or ameliorating effect on a disease or symptom caused by reduction in intestinal barrier function, the method including the steps of:

> (7a) administering the candidate substance or candidate composition to a subject having reduced intestinal barrier function;
> (7b) measuring a biomarker in a specimen obtained from the subject after administration of the candidate substance or candidate composition to acquire a measurement value for the biomarker;
> (7c) comparing the measurement value acquired in step 7b with a reference value to acquire a comparison result; and
> (7d) evaluating the candidate substance or candidate composition for the improving effect on intestinal barrier function or the preventing or ameliorating effect on the disease or symptom on the basis of the comparison result acquired in step 7c,
> wherein the specimen is dry feces or wet feces,
> wherein when the specimen is dry feces, the biomarker includes one or more substances selected from Group A, and
> wherein when the specimen is wet feces, the biomarker includes one or more substances selected from Group B.

[8] The method according to any one of [1] to [7], wherein the intestinal barrier function is barrier function of a small intestine.

[9] The method according to any one of [1] to [8], wherein when the specimen is dry feces, the biomarker includes two, three, or four or more substances selected from Group A, and wherein when the specimen is wet feces, the biomarker includes two, three, or four or more substances selected from Group B.

[10] The method according to any one of [1] to [9], wherein when the specimen is dry feces, the biomarker includes one or more substances selected from the group consisting of formylanthranilic acid, diethanolamine, glycerol, and 5,6-dihydroxyindole, and wherein when the specimen is wet feces, the biomarker includes one or more substances selected from the group consisting of formylanthranilic acid, glycerol, formiminoglutamic acid, and 5,6-dihydroxyindole.

[11] The method according to [10], wherein the biomarker in the specimen is measured by capillary electrophoresis-mass spectrometry.

[12] The method according to any one of [1] to [9], wherein when the specimen is dry feces, the biomarker includes one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker includes one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

[13] The method according to [12], wherein the biomarker in the specimen is measured by liquid chromatography-mass spectrometry.

[14] A biomarker in a specimen derived from a subject for use in evaluating intestinal barrier function in the subject,

> wherein the specimen is dry feces or wet feces,
> wherein when the specimen is dry feces, the biomarker includes one or more substances selected from Group A, and
> wherein when the specimen is wet feces, the biomarker includes one or more substances selected from Group B.

[15] A biomarker in a specimen derived from a subject for use in diagnosing a disease or symptom caused by reduction in intestinal barrier function in the subject,

> wherein the specimen is dry feces or wet feces,
> wherein when the specimen is dry feces, the biomarker includes one or more substances selected from Group A, and
> wherein when the specimen is wet feces, the biomarker includes one or more substances selected from Group B.

[16] A biomarker in a specimen derived from a subject for use in evaluating the subject's risk of developing a disease or symptom caused by reduction in intestinal barrier function, or probability of the subject being affected by the disease or symptom,

> wherein the specimen is dry feces or wet feces,

wherein when the specimen is dry feces, the biomarker includes one or more substances selected from Group A, and

wherein when the specimen is wet feces, the biomarker includes one or more substances selected from Group B.

[17] A biomarker in a specimen derived from a subject for use in acquiring data for diagnosing a disease or symptom caused by reduction in intestinal barrier function in the subject,

wherein the specimen is dry feces or wet feces,

wherein when the specimen is dry feces, the biomarker includes one or more substances selected from Group A, and

wherein when the specimen is wet feces, the biomarker includes one or more substances selected from Group B.

[18] A biomarker in a specimen for use in screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject,

wherein the specimen is dry feces or wet feces,

wherein when the specimen is dry feces, the biomarker includes one or more substances selected from Group A, and

wherein when the specimen is wet feces, the biomarker includes one or more substances selected from Group B.

[19] A biomarker in a specimen for use in evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject,

wherein the specimen is dry feces or wet feces,

wherein when the specimen is dry feces, the biomarker includes one or more substances selected from Group A, and

wherein when the specimen is wet feces, the biomarker includes one or more substances selected from Group B.

[20] The biomarker according to any one of [14] to [19], wherein the intestinal barrier function is barrier function of a small intestine.

[21] The biomarker according to any one of [14] to [20], wherein when the specimen is dry feces, the biomarker includes two, three, or four or more substances selected from the Group A, and wherein when the specimen is wet feces, the biomarker includes two, three, or four or more substances selected from the Group B.

[22] The biomarker according to any one of [14] to [21], wherein when the specimen is dry feces, the biomarker includes one or more substances selected from the group consisting of formylanthranilic acid, diethanolamine, glycerol, and 5,6-dihydroxyindole, and wherein when the specimen is wet feces, the biomarker includes one or more substances selected from the group consisting of formylanthranilic acid, glycerol, formiminoglutamic acid, and 5,6-dihydroxyindole.

[23] The biomarker according to [22], wherein the biomarker in the specimen is measured by capillary electrophoresis-mass spectrometry.

[24] The biomarker according to any one of [14] to [21], wherein when the specimen is dry feces, the biomarker includes one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker includes one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

[25] The biomarker according to [24], wherein the biomarker in the specimen is measured by liquid chromatography-mass spectrometry.

[26] Use of a biomarker in a specimen derived from a subject for evaluating intestinal barrier function in the subject,

wherein the specimen is dry feces or wet feces,

wherein when the specimen is dry feces, the biomarker includes one or more substances selected from Group A, and

wherein when the specimen is wet feces, the biomarker includes one or more substances selected from Group B.

[27] Use of a biomarker in a specimen derived from a subject for diagnosing a disease or symptom caused by reduction in intestinal barrier function in the subject,

wherein the specimen is dry feces or wet feces,
wherein when the specimen is dry feces, the biomarker includes one or more substances selected from Group A, and
wherein when the specimen is wet feces, the biomarker includes one or more substances selected from Group B.

[28] Use of a biomarker in a specimen derived from a subject for evaluating the subject's risk of developing a disease or symptom caused by reduction in intestinal barrier function, or probability of the subject being affected by the disease or symptom,

wherein the specimen is dry feces or wet feces,
wherein when the specimen is dry feces, the biomarker includes one or more substances selected from Group A, and
wherein when the specimen is wet feces, the biomarker includes one or more substances selected from Group B.

[29] Use of a biomarker in a specimen derived from a subject for acquiring data for diagnosing a disease or symptom caused by reduction in intestinal barrier function in the subject,

wherein the specimen is dry feces or wet feces,
wherein when the specimen is dry feces, the biomarker includes one or more substances selected from Group A, and
wherein when the specimen is wet feces, the biomarker includes one or more substances selected from Group B.

[30] Use of a biomarker in a specimen for screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject,

wherein the specimen is dry feces or wet feces,
wherein when the specimen is dry feces, the biomarker includes one or more substances selected from Group A, and
wherein when the specimen is wet feces, the biomarker includes one or more substances selected from Group B.

[31] Use of a biomarker in a specimen for evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject,

wherein the specimen is dry feces or wet feces,
wherein when the specimen is dry feces, the biomarker includes one or more substances selected from Group A, and
wherein when the specimen is wet feces, the biomarker includes one or more substances selected from Group B.

[32] The use according to any one of [26] to [31], wherein the intestinal barrier function is barrier function of a small intestine.
[33] The use according to any one of [26] to [32], wherein when the specimen is dry feces, the biomarker includes two, three, or four or more substances selected from the Group A, and wherein when the specimen is wet feces, the biomarker includes two, three, or four or more substances selected from the Group B.
[34] The use according to any one of [26] to [33], wherein when the specimen is dry feces, the biomarker includes one or more substances selected from the group consisting of formylanthranilic acid, diethanolamine, glycerol, and 5,6-dihydroxyindole, and wherein when the specimen is wet feces, the biomarker includes one or more substances selected from the group consisting of formylanthranilic acid, glycerol, formiminoglutamic acid, and 5,6-dihydroxyindole.
[35] The use according to [34], wherein the biomarker in the specimen is measured by capillary electrophoresis-mass spectrometry.
[36] The use according to any one of [26] to [33], wherein when the specimen is dry feces, the biomarker includes one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker includes one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

[37] The use according to [36], wherein the biomarker in the specimen is measured by liquid chromatography-mass spectrometry.

[38] A kit for evaluating intestinal barrier function in a subject,

wherein the kit includes one or more reagents for measuring a biomarker in a specimen derived from the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[39] A kit for diagnosing a disease or symptom caused by reduction in intestinal barrier function in a subject,

wherein the kit includes one or more reagents for measuring a biomarker in a specimen derived from the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[40] A kit for evaluating a subject's risk of developing a disease or symptom caused by reduction in intestinal barrier function, or probability of the subject being affected by the disease or symptom,

wherein the kit includes one or more reagents for measuring a biomarker in a specimen derived from the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[41] A kit for acquiring data for diagnosing a disease or symptom caused by reduction in intestinal barrier function in a subject,

wherein the kit includes one or more reagents for measuring a biomarker in a specimen derived from the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[42] A kit for screening for a substance or composition having an improving effect on intestinal barrier function or a preventing or ameliorating effect on a disease or symptom caused by reduction in intestinal barrier function,

wherein the kit includes one or more reagents for measuring a biomarker in a specimen obtained from a subject having reduced intestinal barrier function after administration of a candidate substance or candidate composition to the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[43] A kit for evaluating a candidate substance or candidate composition for an improving effect on intestinal barrier function or a preventing or ameliorating effect on a disease or symptom caused by reduction in intestinal barrier function,

wherein the kit includes one or more reagents for measuring a biomarker in a specimen obtained from a subject having reduced intestinal barrier function after administration of the candidate substance or candidate composition to the subject,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[44] The kit according to any one of [38] to [43], wherein the intestinal barrier function is barrier function of a small intestine.

[45] The kit according to any one of [38] to [44], wherein when the specimen is dry feces, the biomarker includes two, three, or four or more substances selected from the Group A, and wherein when the specimen is wet feces, the biomarker includes two, three, or four or more substances selected from the Group B.

[46] The kit according to any one of [38] to [45], wherein when the specimen is dry feces, the biomarker includes one or more substances selected from the group consisting of formylanthranilic acid, diethanolamine, glycerol, and 5,6-dihydroxyindole, and wherein when the specimen is wet feces, the biomarker includes one or more substances selected from the group consisting of formylanthranilic acid, glycerol, formiminoglutamic acid, and 5,6-dihydroxyindole.

[47] The kit according to [46], wherein the biomarker in the specimen is measured by capillary electrophoresis-mass spectrometry.

[48] The kit according to any one of [38] to [45], wherein when the specimen is dry feces, the biomarker includes one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker includes one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

[49] The kit according to [48], wherein the biomarker in the specimen is measured by liquid chromatography-mass spectrometry.

[50] A method for producing a food product or pharmaceutical composition, the method including the step of blending a substance or composition obtained through screening by the method according to [6] with one or two or more components to constitute the food product or pharmaceutical composition.

[51] A computer program for allowing a computer to execute a method for evaluating intestinal barrier function in a subject, the method including the steps of:

(S101) acquiring a measurement value for a biomarker in a specimen derived from the subject;
(S102) comparing the measurement value acquired in step S101 with a reference value to acquire a comparison result; and
(S103) evaluating intestinal barrier function in the subject on the basis of the comparison result acquired in step S102,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[52] A computer program for allowing a computer to execute a method for diagnosing a disease or symptom caused by reduction in intestinal barrier function in a subject, the method including the steps of:

(S201) acquiring a measurement value for a biomarker in a specimen derived from the subject;
(S202) comparing the measurement value acquired in step S201 with a reference value to acquire a comparison result; and
(S203) diagnosing the disease or symptom on the basis of the comparison result acquired in step S202,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[53] A computer program for allowing a computer to execute a method for evaluating a subject's risk of developing a disease or symptom caused by reduction in intestinal barrier function, or probability of the subject being affected by the disease or symptom, the method including the steps of:

(S301) acquiring a measurement value for a biomarker in a specimen derived from the subject;
(S302) comparing the measurement value acquired in step S301 with a reference value to acquire a comparison result; and
(S303) evaluating the risk or the probability on the basis of the comparison result acquired in step S302, wherein the specimen is dry feces or wet feces,
wherein when the specimen is dry feces, the biomarker includes one or more substances selected from Group A, and
wherein when the specimen is wet feces, the biomarker includes one or more substances selected from Group B.

[54] A computer program for allowing a computer to execute a method for acquiring data for diagnosing a disease or symptom caused by reduction in intestinal barrier function in a subject, the method including the steps of:

(S401) acquiring a measurement value for a biomarker in a specimen derived from the subject; and
(S402) comparing the measurement value acquired in step S401 with a reference value to acquire a comparison result as the data,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[55] A computer program for allowing a computer to execute a method for screening for a substance or composition having an improving effect on intestinal barrier function or a preventing or ameliorating effect on a disease or symptom caused by reduction in intestinal barrier function, the method including the steps of:

(S501) acquiring a measurement value for a biomarker in a specimen obtained from a subject having reduced intestinal barrier function after administration of a candidate substance or candidate composition to the subject;
(S502) comparing the measurement value acquired in step S501 with a reference value to acquire a comparison result; and
(S503) evaluating the candidate substance or candidate composition for the improving effect on intestinal barrier function or the preventing or ameliorating effect on the disease or symptom on the basis of the comparison result acquired in step S502,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[56] A computer program for allowing a computer to execute a method for evaluating a candidate substance or candidate composition for an improving effect on intestinal barrier function or a preventing or ameliorating effect on a disease or symptom caused by reduction in intestinal barrier function, the method including the steps of:

(S601) acquiring a measurement value for a biomarker in a specimen obtained from a subject having reduced intestinal barrier function after administration of a candidate substance or candidate composition to the subject;
(S602) comparing the measurement value acquired in step S601 with a reference value to acquire a comparison result; and
(S603) evaluating the candidate substance or candidate composition for the improving effect on intestinal barrier function or the preventing or ameliorating effect on the disease or symptom on the basis of the comparison result acquired in step S602,
wherein the specimen is dry feces or wet feces,
wherein when the specimen is dry feces, the biomarker includes one or more substances selected from Group A, and
wherein when the specimen is wet feces, the biomarker includes one or more substances selected from Group B.

[57] The computer program according to any one of [51] to [56], wherein the intestinal barrier function is barrier function of a small intestine.
[58] The computer program according to any one of [51] to [57], wherein when the specimen is dry feces, the biomarker includes two, three, or four or more substances selected from the Group A, and wherein when the specimen is wet feces, the biomarker includes two, three, or four or more substances selected from the Group B.
[59] The computer program according to any one of [51] to [58], wherein when the specimen is dry feces, the biomarker includes one or more substances selected from the group consisting of formylanthranilic acid, diethanolamine, glycerol, and 5,6-dihydroxyindole, and wherein when the specimen is wet feces, the biomarker includes one or more substances selected from the group consisting of formylanthranilic acid, glycerol, formiminoglutamic acid, and 5,6-dihydroxyindole.

[60] The computer program according to [59], wherein the biomarker in the specimen is measured by capillary electrophoresis-mass spectrometry.

[61] The computer program according to any one of [51] to [58], wherein when the specimen is dry feces, the biomarker includes one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker includes one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

[62] The computer program according to [61], wherein the biomarker in the specimen is measured by liquid chromatography-mass spectrometry.

[63] A computer-readable recording medium storing the computer program according to any one of [51] to [62].

ADVANTAGEOUS EFFECTS OF INVENTION

The present invention provides a method for evaluating intestinal barrier function in a subject, a biomarker for the evaluation, use of a biomarker for the evaluation, a kit for the evaluation, and a computer program for allowing a computer to execute the method.

The present invention also provides a method for diagnosing a disease or symptom caused by reduction in intestinal barrier function in a subject, a biomarker for the diagnosis, use of a biomarker for the diagnosis, a kit for the diagnosis, and a computer program for allowing a computer to execute the method.

The present invention also provides a method for treating a disease or symptom caused by reduction in intestinal barrier function in a subject.

The present invention also provides a method for evaluating a subject's risk of developing a disease or symptom caused by reduction in intestinal barrier function, or probability of the subject being affected by the disease or symptom, a biomarker for the evaluation, use of a biomarker for the evaluation, a kit for the evaluation, and a computer program for allowing a computer to execute the method.

The present invention also provides a method for acquiring data for diagnosing a disease or symptom caused by reduction in intestinal barrier function in a subject, a biomarker for the acquisition, use of a biomarker for the acquisition, a kit for the acquisition, and a computer program for allowing a computer to execute the method.

The present invention also provides a method for screening for a substance or composition having an improving effect on intestinal barrier function or a preventing or ameliorating effect on a disease or symptom caused by reduction in intestinal barrier function, a biomarker for the screening, use of a biomarker for the screening, a kit for the screening, and a computer program for allowing a computer to execute the method.

The present invention also provides a method for evaluating a candidate substance or candidate composition for an improving effect on intestinal barrier function or a preventing or ameliorating effect on a disease or symptom caused by reduction in intestinal barrier function, a biomarker for the evaluation, use of a biomarker for the evaluation, a kit for the evaluation, and a computer program for allowing a computer to execute the method.

The present invention also provides a method for producing a food product or pharmaceutical composition.

BRIEF DESCRIPTION OF DRAWINGS

Figure 1 is a schematic diagram of an apparatus according to an embodiment of the present invention.
Figure 2 is a block diagram illustrating the hardware configuration of an apparatus according to an embodiment of the present invention.
Figure 3 is a flowchart illustrating a procedure with an apparatus according to an embodiment of the present invention.
Figure 4 is a flowchart illustrating a procedure with an apparatus according to an embodiment of the present invention.
Figure 5 is a flowchart illustrating a procedure with an apparatus according to an embodiment of the present invention.

DETAILED DESCRIPTION OF INVENTION

Hereinafter, the present invention will be described. Two or more of embodiments described herein can be combined, and such combinations are also included in the present invention. Herein, "E-X" indicates $\times 10^{-X}$, and "E+Y"

indicates $\times 10^Y$.

<<Aspect 1>>

Aspect 1 of the present invention relates to a method for evaluating intestinal barrier function in a subject.

In the method according to Aspect 1, a biomarker in a specimen derived from the subject is used as an indicator for evaluating intestinal barrier function in the subject.

The subject may be any animal that excretes feces without limitation. Such animals include vertebrates including mammals, reptiles, birds, amphibians, and fish, mammals and birds are preferred, and mammals are more preferred. The mammals include primates (e.g., humans, gorillas, chimpanzees, orangutans), rodents (e.g., mice, rats, hamsters, guinea pigs, rabbits), domestic animals (e.g., cattle, pigs, sheep, goats, horses), and pet animals (e.g., dogs, cats), and humans are preferred. The birds include poultry (e.g., chickens, wild ducks, ducks).

Feces are excretions from ani. Feces derived from the subject can be obtained from feces excreted by the subject according to a conventional method, and the feces obtained can be used as a specimen.

The moisture content of dry feces is preferably 20% by mass or less, more preferably 10% by mass or less, and more preferably 5% by mass or less, based on the mass of the dry feces. The lower limit value of the moisture content of dry feces is zero. Dry feces can be obtained by performing drying treatment for feces immediately after being excreted from the anus. The drying treatment can be performed, for example, by using a commercially available freeze-dryer. In the freeze-drying treatment, the treatment temperature is, for example, -80°C or more and 0°C or less, and the treatment time is, for example, 24 hours or more and 72 hours or less.

The moisture content of wet feces is preferably 40% by mass or more, more preferably 50% by mass or more, and more preferably 60% by mass or more, based on the mass of the wet feces. The upper limit is not limited, and, for example, 95% by mass or less, 90% by mass or less, 80% by mass or less, or 70% by mass or less. These upper limits may be each combined with any of the aforementioned lower limits. The wet feces may be feces immediately after being excreted from the anus, or feces obtained by thawing feces that have been cryopreserved immediately after being excreted from the anus. The temperature for the cryopreservation is preferably -100°C or more and -10°C or less, more preferably -80°C or more and -15°C or less, and even more preferably -80°C or more and - 60°C or less. The cryopreservation can be performed for feces immediately after being excreted from the anus with the feces contained in a container specialized for fecal sampling (e.g., Faeces tube, with blade, screw cap (manufactured by SARSTEDT AG & Co. KG)). The period of the cryopreservation is preferably 180 days or less from fecal sampling, more preferably 60 days or less from fecal sampling, and even more preferably 30 days or less from fecal sampling. In other words, wet feces within 180 days from fecal sampling is preferably subjected to measurement of a biomarker, wet feces within 60 days from fecal sampling is more preferably subjected to measurement of a biomarker, and wet feces within 30 days from fecal sampling is even more preferably subjected to measurement of a biomarker.

The biomarker is a substance or substance group that is present in living bodies and such that the increase or decrease of the *in vivo* concentration reflects the presence of a specific condition, symptom, disease, or the like, the degree of a symptom, the risk of development, the risk of being affected, a preventing effect, a therapeutic effect, an ameliorating effect, and so on. A substance group is a combination of one or more, preferably two or more substances. The biomarker is a substance or substance group to be measured in the present invention, and used as an indicator for evaluating intestinal barrier function in a subject.

The specimen derived from the subject is dry feces or wet feces. Use of dry feces or wet feces as a specimen enables simple evaluation of intestinal barrier function in the subject.

In the case where the specimen derived from the subject is dry feces, one or more substances selected from Group A are used as the biomarker for evaluating intestinal barrier function in the subject. That is, in the case where the specimen derived from the subject is dry feces, the biomarker in the specimen derived from the subject includes one or more substances selected from Group A.

The biomarker in the specimen derived from the subject may include one substance selected from Group A, or two or more substances selected from Group A. For achieving enhanced accuracy of evaluating intestinal barrier function in the subject, the biomarker in the specimen derived from the subject preferably includes two, three, or four or more substances selected from Group A.

In the case where the specimen derived from the subject is wet feces, one or more substances selected from Group B are used as the biomarker for evaluating intestinal barrier function in the subject. That is, in the case where the specimen derived from the subject is wet feces, the biomarker in the specimen derived from the subject includes one or more substances selected from Group B.

The biomarker in the specimen derived from the subject may include one substance selected from Group B, or two or more substances selected from Group B. For achieving enhanced accuracy of evaluating intestinal barrier function in the subject, the biomarker in the specimen derived from the subject preferably includes two, three, or four or more substances selected from Group B.

In the case where the specimen derived from the subject is dry feces, intestinal barrier function in the subject can be evaluated with high accuracy by using one or more substances selected from Group A as the biomarker. The substances of No. 1 to No. 15 and No. 63 to No. 86 in Group A give higher accuracy of evaluating intestinal barrier function than other substances in Group A, and the substances of No. 1 to No. 4 and No. 63 to No. 66 in Group A give particularly higher accuracy of evaluating intestinal barrier function than other substances in Group A.

In the case where the specimen derived from the subject is wet feces, intestinal barrier function in the subject can be evaluated with high accuracy by using one or more substances selected from Group B as the biomarker. The substances of No. 1 to No. 11 and No. 46 to No. 67 in Group B give higher accuracy of evaluating intestinal barrier function than other substances in Group B, and the substances of No. 1 to No. 4 and No. 46 to No. 49 in Group B give particularly higher accuracy of evaluating intestinal barrier function than other substances in Group B.

In an embodiment, the specimen derived from the subject is dry feces, and the biomarker in the specimen derived from the subject includes one or more substances selected from the substances of No. 1 to No. 4 in Group A (formylanthranilic acid, diethanolamine, glycerol, and 5,6-dihydroxyindole). Thereby, enhanced accuracy of evaluating intestinal barrier function in the subject is achieved. In addition to one or more substances selected from the four substances, the biomarker in the specimen derived from the subject may include one or more additional substances selected from Group A (e.g., one or more substances selected from the substances of No. 5 to No. 62 in Group A).

In another embodiment, the specimen derived from the subject is wet feces, and the biomarker in the specimen derived from the subject includes one or more substances selected from the substances of No. 1 to No. 4 in Group B (formylanthranilic acid, glycerol, formiminoglutamic acid, and 5,6-dihydroxyindole). Thereby, enhanced accuracy of evaluating intestinal barrier function in the subject is achieved. In addition to one or more substances selected from the four substances, the biomarker in the specimen derived from the subject may include one or more additional substances selected from Group B (e.g., one or more substances selected from the substances of No. 5 to No. 45 in Group B).

In still another embodiment, the specimen derived from the subject is dry feces, and the biomarker in the specimen derived from the subject includes one or more substances selected from the substances of No. 63 to No. 66 in Group A (3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate). Thereby, enhanced accuracy of evaluating intestinal barrier function in the subject is achieved. In addition to one or more substances selected from the four substances, the biomarker in the specimen derived from the subject may include one or more additional substances selected from Group A (e.g., one or more substances selected from the substances of No. 67 to No. 128 in Group A).

In still another embodiment, the specimen derived from the subject is wet feces, and the biomarker in the specimen derived from the subject includes one or more substances selected from the substances of No. 46 to No. 49 in Group B (6-methylquinoline, N1-[4-(1,3-oxazol-5-yl)phenyl]cyclopropane-1-carboxamide, 2-hydroxyquinoline, and 3-methyladipic acid). Thereby, enhanced accuracy of evaluating intestinal barrier function in the subject is achieved. In addition to one or more substances selected from the four substances, the biomarker in the specimen derived from the subject may include one or more additional substances selected from Group B (e.g., one or more substances selected from the substances of No. 50 to No. 99 in Group B).

In still another embodiment, the specimen derived from the subject is dry feces, and the biomarker in the specimen derived from the subject includes the substance of No. 1 in Group A (formylanthranilic acid). Thereby, enhanced accuracy of evaluating intestinal barrier function in the subject is achieved. In addition to the one substance, the biomarker in the specimen derived from the subject may include one or more additional substances selected from Group A (e.g., one or more substances selected from the substances of No. 2 to No. 62 in Group A).

In still another embodiment, the specimen derived from the subject is dry feces, and the biomarker in the specimen derived from the subject includes the substance of No. 2 in Group A (diethanolamine). Thereby, enhanced accuracy of evaluating intestinal barrier function in the subject is achieved. In addition to the one substance, the biomarker in the specimen derived from the subject may include one or more additional substances selected from Group A (e.g., one or more substances selected from the substances of No. 1 and No. 3 to No. 62 in Group A).

In still another embodiment, the specimen derived from the subject is dry feces, and the biomarker in the specimen derived from the subject includes the substance of No. 4 in Group A (5,6-dihydroxyindole). Thereby, enhanced accuracy of evaluating intestinal barrier function in the subject is achieved. In addition to the one substance, the biomarker in the specimen derived from the subject may include one or more additional substances selected from Group A (e.g., one or more substances selected from the substances of No. 1 to No. 3 and No. 5 to No. 62 in Group A).

In still another embodiment, the specimen derived from the subject is dry feces, and the biomarker in the specimen derived from the subject includes the substance of No. 6 in Group A (leucine). Thereby, enhanced accuracy of evaluating intestinal barrier function in the subject is achieved. In addition to the one substance, the biomarker in the specimen derived from the subject may include one or more additional substances selected from Group A (e.g., one or more substances selected from the substances of No. 1 to No. 5 and No. 7 to No. 62 in Group A).

In still another embodiment, the specimen derived from the subject is wet feces, and the biomarker in the specimen derived from the subject includes the substance of No. 1 in Group B (formylanthranilic acid). Thereby, enhanced accuracy of evaluating intestinal barrier function in the subject is achieved. In addition to the one substance, the biomarker in the specimen derived from the subject may include one or more additional substances selected from Group B (e.g., one or more substances selected from the substances of No. 2 to No. 45 in Group B).

In still another embodiment, the specimen derived from the subject is wet feces, and the biomarker in the specimen derived from the subject includes the substance of No. 4 in Group B (5,6-dihydroxyindole). Thereby, enhanced accuracy of evaluating intestinal barrier function in the subject is achieved. In addition to the one substance, the biomarker in the specimen derived from the subject may include one or more additional substances selected from Group B (e.g., one or more substances selected from the substances of No. 1 to No. 3 and No. 5 to No. 45 in Group B).

In still another embodiment, the specimen derived from the subject is wet feces, and the biomarker in the specimen derived from the subject includes the substance of No. 6 in Group B (4-guanidinobutyric acid). Thereby, enhanced accuracy of evaluating intestinal barrier function in the subject is achieved. In addition to the one substance, the biomarker in the specimen derived from the subject may include one or more additional substances selected from Group B (e.g., one or more substances selected from the substances of No. 1 to No. 5 and No. 7 to No. 45 in Group B).

In still another embodiment, the specimen derived from the subject is wet feces, and the biomarker in the specimen derived from the subject includes the substance of No. 7 in Group B (2'-deoxyadenosine and 5'-deoxyadenosine). Thereby, enhanced accuracy of evaluating intestinal barrier function in the subject is achieved. In addition to the one substance, the biomarker in the specimen derived from the subject may include one or more additional substances selected from Group B (e.g., one or more substances selected from the substances of No. 1 to No. 6 and No. 8 to No. 45 in Group B).

The method according to Aspect 1 includes the steps of:

(1a) measuring a biomarker in a specimen derived from the subject to acquire a measurement value for the biomarker; and

(1b) comparing the measurement value acquired in step 1a with a reference value to acquire a comparison result; and

(1c) evaluating intestinal barrier function in the subject on the basis of the comparison result acquired in step 1b.

<Step 1a>

Step 1a is a step of measuring a biomarker in a specimen derived from the subject to acquire a measurement value for the biomarker.

The specimen derived from the subject is dry feces or wet feces.

In the case where the specimen derived from the subject is dry feces, one or more substances selected from Group A is used as a biomarker for evaluating intestinal barrier function in the subject. That is, in the case where the specimen derived from the subject is dry feces, the biomarker to be measured in step 1a includes one or more substances selected from Group A.

In the case where the biomarker to be measured in step 1a includes one substance selected from Group A, the one substance is measured in step 1a. In the case where the biomarker to be measured in step 1a includes two or more substances selected from Group A, the two or more substances are measured in step 1a. For achieving enhanced accuracy of evaluating intestinal barrier function in the subject, it is preferable that the biomarker to be measured in step 1a includes two, three, or four or more substances selected from Group A (i.e., two, three, or four or more substances selected from Group A are measured in step 1a).

In the case where the specimen derived from the subject is wet feces, one or more substances selected from Group B are used as a biomarker for evaluating intestinal barrier function in the subject. That is, in the case where the specimen derived from the subject is wet feces, the biomarker to be measured in step 1a includes one or more substances selected from Group B.

In the case where the biomarker to be measured in step 1a includes one substance selected from Group B, the one substance is measured in step 1a. In the case where the biomarker to be measured in step 1a includes two or more substances selected from Group B, the two or more substances are measured in step 1a. For achieving enhanced accuracy of evaluating intestinal barrier function in the subject, it is preferable that the biomarker to be measured in step 1a includes two, three, or four or more substances selected from Group B (i.e., two, three, or four or more substances selected from Group B are measured in step 1a).

The phrase "measuring one substance" means measuring the concentration of the one substance, and the phrase "measuring two or more substances" means measuring the concentrations of the two or more substances, or determining a combined variable as a statistical analysis value from the concentrations of the two or more substances. Herein, a substance to be subjected to measurement (biomarker) may be referred to as a "target substance".

Each concentration may be a relative concentration or an absolute concentration, but is preferably an absolute concentration.

The relative concentration of a target substance includes a relative value that correlates with the absolute concentration of a target substance. The relative concentration of a target substance is, for example, a ratio of a measurement value for the target substance to a measurement value for an internal standard (e.g., the relative area value CE or relative area value LC described later). The relative concentration of a target substance may be a ratio given by one measurement sample, or the mean of ratios given by two or more measurement samples. Here, the two or more measurement samples are prepared from the same specimen.

The substance of No. 11 in Group A is composed of two substances (2'-deoxyadenosine and 5'-deoxyadenosine). For the substance of No. 11 in Group A, the two substances are collectively regarded as a single substance. Accordingly, the concentration of the substance of No. 11 in Group A refers to the total concentration of the two substances.

The substance of No. 58 in Group A is composed of two substances (3'-cytidylic acid and 2'-cytidylic acid). For the substance of No. 58 in Group A, the two substances are collectively regarded as a single substance. Accordingly, the concentration of the substance of No. 58 in Group A refers to the total concentration of 3'-cytidylic acid and 2'-cytidylic acid.

The substance of No. 7 in Group B is composed of two substances (2'-deoxyadenosine and 5'-deoxyadenosine). For the substance of No. 7 in Group B, the two substances are collectively regarded as a single substance. Accordingly, the concentration of the substance of No. 7 in Group B refers to the total concentration of the two substances.

The substance of No. 30 in Group B is composed of two substances (isobutyric acid and butyric acid). For the substance of No. 30 in Group B, the two substances are collectively regarded as a single substance. Accordingly, the concentration of the substance of No. 30 in Group B refers to the total concentration of the two substances.

The substance of No. 41 in Group B is composed of two substances (3'-cytidylic acid and 2'-cytidylic acid). For the substance of No. 41 in Group B, the two substances are collectively regarded as a single substance. Accordingly, the concentration of the substance of No. 41 in Group B refers to the total concentration of the two substances.

In the present invention, "DL" (see No. 118 in Group A and No. 94 in Group B) means a mixture of a D-form and an L-form.

Examples of the method for measuring a target substance include liquid chromatography-mass spectrometry (LC-MS), capillary electrophoresis-mass spectrometry (CE-MS), gas chromatography-mass spectrometry (GC-MS), mass spectrometry (MS), and high-performance liquid chromatography (HPLC), and CE-MS or LC-MS is preferred among these. CE-MS is suitable for measurement of water-soluble substances, and LC-MS is suitable for measurement of liposoluble substances. Particularly preferred among different types of CE-MS is capillary electrophoresis-Fourier transform mass spectrometry (CE-FTMS). Particularly preferred among different types of LC-MS is liquid chromatography-tandem mass spectrometry (LC-MS/MS).

Measurement samples to be used in the method for measuring a target substance can be prepared from the specimen according to a conventional method. Each measurement sample to be used in the method for measuring a target substance may contain an internal standard. The relative concentration of a target substance can be determined, for example, as a ratio of a measurement value for the target substance to a measurement value for an internal standard. The absolute concentration of a target substance can be determined, for example, from the relative concentration of the target substance with use of a pre-prepared calibration curve showing the relationship between absolute concentrations of the target substance and relative concentrations of the target substance.

In an embodiment, the method for measuring a target substance is CE-MS (preferably CE-FTMS). While measurement samples to be used for CE-MS can be prepared from the specimen according to a conventional method, it is preferred to prepare them from the specimen according to a method described in the Examples. While measurement by CE-MS can be performed under common conditions, it is preferred to perform the measurement under conditions described in the Examples. Each measurement sample to be used for CE-MS may contain an internal standard. Examples of the internal standard include a compound obtained by labeling a target substance with a stable isotope such as DL-phenyl-D5-alanine, a fatty acid methyl ester (e.g., methyl tetradecanoate), ribitol, and an internal standard (H3304-1002; HMT) from Human Metabolome Technologies, Inc. (HMT).

In the embodiment in which the method for measuring a target substance is CE-MS (preferably CE-FTMS), the relative concentration of a target substance can be determined, for example, as a ratio of the peak area acquired from the target substance in CE-MS to the peak area acquired from an internal standard in CE-MS (referred to as the "relative area value CE"). The relative concentration of a target substance may be a relative area value CE acquired from one measurement sample, or the mean of relative area values CE acquired from two or more measurement samples. The peak area for an internal standard can be determined as a peak area for a compound having a specific m/z value (the compound corresponds to the internal standard). The peak area for a target substance can be determined as a peak area for a compound having a specific m/z value (the compound corresponds to the target substance). The absolute concentration of a target substance can be determined, for example, from the relative concentration of the target substance with use of a pre-prepared calibration curve showing the relationship between absolute concentrations of the target substance and relative concentrations of the target substance.

In another embodiment, the method for measuring a target substance is LC-MS (preferably LC-MS/MS). While measurement samples to be used for LC-MS can be prepared from the specimen according to a conventional method, it is preferred to prepare them from the specimen according to a method described in the Examples. While measurement by LC-MS can be performed under common conditions, it is preferred to perform the measurement under conditions described in the Examples. Each measurement sample to be used for LC-MS may contain an internal standard. Examples of the internal standard include a compound obtained by labeling a target substance with a stable isotope such as DL-phenyl-D5-alanine, a fatty acid methyl ester (e.g., methyl tetradecanoate), ribitol, and an internal standard (H3304-1002; HMT) from Human Metabolome Technologies, Inc. (HMT).

In the embodiment in which the method for measuring a target substance is LC-MS (preferably LC-MS/MS), the relative concentration of a target substance can be determined, for example, as a ratio of the peak area acquired from the target substance in LC-MS to the peak area acquired from an internal standard in LC-MS (hereinafter, referred to as the "relative area value LC"). The relative concentration of a target substance may be a relative area value LC acquired from one measurement sample, or the mean of relative area values LC acquired from two or more measurement samples. The peak area for an internal standard can be determined as a peak area for a compound having a specific m/z value (the compound corresponds to the internal standard). The peak area for a target substance can be determined as a peak area for a compound having a specific m/z value (the compound corresponds to the target substance). The absolute concentration of a target substance can be determined, for example, from the relative concentration of the target substance with use of a pre-prepared calibration curve showing the relationship between absolute concentrations of the target substance and relative concentrations of the target substance.

Measurement of the substances of No. 1 to No. 62 in Group A is preferably performed by CE-MS (in particular, CE-FTMS), and measurement of the substances of No. 63 to No. 128 in Group A is preferably performed by LC-MS (in particular, LC-MS/MS). The m/z values of the substances in Group A are as shown in Table 18.

Measurement of the substances of No. 1 to No. 45 in Group B is preferably performed by CE-MS (in particular, CE-FTMS), and measurement of the substances of No. 46 to No. 99 in Group B is preferably performed by LC-MS (in particular, LC-MS/MS). The m/z values of the substances in Group B are as shown in Table 16.

In the case where two or more substances (the first substance, the second substance, ..., the n-th substance (wherein n is an integer of 2 or more)) in the specimen are measured and a combined variable as a statistical analysis value is determined from the concentrations of the two or more substances in the specimen, the combined variable is preferably determined from the following expression (1):

Combined variable = (Coefficient 1 × (Concentration of first substance)) + (Coefficient 2 × (Concentration of second substance)) + ... + (Coefficient n × (Concentration of n-th substance)) + Constant term ⋯ (1)

In the expression (1), each "concentration" may be a relative concentration or an absolute concentration, but is preferably an absolute concentration. In the case where the method for measuring substances is CE-MS (preferably CE-FTMS), the relative concentration of each substance is, for example, a relative area value CE. The relative concentration of each substance may be a relative area value CE acquired from one measurement sample, or the mean of relative area values CE acquired from two or more measurement samples. In the case where the method for measuring substances is LC-MS (preferably LC-MS/MS), the relative concentration of each substance is, for example, a relative area value LC. The relative concentration of each substance may be a relative area value LC acquired from one measurement sample, or the mean of relative area values LC acquired from two or more measurement samples.

n may be any integer of 2 or more without limitation, and is, for example, an integer of 2 or more and 128 or less (e.g., 3, 4, 6, 10, 50, 90, 99, 128).

The coefficients and constant term depend on the method for measuring substances, the type of concentration of substances (absolute concentration or relative concentration), and the number and types of substances to be combined. The coefficients and constant term can be determined through multinomial logistic regression analysis. The coefficients are partial regression coefficients determined through multinomial logistic regression analysis. Methods for determining the coefficients and constant term through multinomial logistic regression analysis are well known to those skilled in the art. For such methods, reference can be made to documents including Metab Brain Dis, DOI 10.1007/s11011-017-0029-x, Published online: 11 May 2017, PNAS, October 14, 2003, vol. 100, no. 21, pp. 12343-12348 (www.pnas.org/cgi/-doi/10.1073/pnas.2033602100, and Ann Transl Med, 2019, 7(16):388, p. 1-10 (http://dx.doi.org/10.21037/atm.2019.07.102). The coefficients to be used in determining the combined variable can be derived from measurement data in such a manner that the combined variable is close to 1 for a group with reduced intestinal barrier function, and close to 0 for a group with non-reduced intestinal barrier function (a group with normal intestinal barrier function).

In the case where the specimen obtained from the subject is dry feces, the biomarker to be measured in step 1a includes two or more substances selected from the substances of No. 1 to No. 4 in Group A (formylanthranilic acid, diethanolamine, glycerol, and 5,6-dihydroxyindole), and measurement of the relative area values (relative concentrations) of the two or more substances is performed by CE-FTMS under conditions described in the Examples, the combined variable can be determined from the concentrations of the two or more substances on the basis of the expression (1), for example, with use of coefficients and a constant term shown in Table 3. In Table 3, "CFD_A" refers to formylanthranilic acid, "CFD_B" refers to diethanolamine, "CFD_C" refers to glycerol, and "CFD_D" refers to 5,6-dihydroxyindole.

[Table 3]

Table 3 (Dry feces, No. 1 to No. 4 in Group A)

| Number of substances combined | First substance | Second substance | Third substance | Fourth substance | Coefficient 1 | Coefficient 2 | Coefficient 3 | Coefficient 4 | Constant term |
|---|---|---|---|---|---|---|---|---|---|
| 2 | CFD_A | CFD_B | - | - | -17298.7 | 3702.328 | - | - | -2.19746 |
| 2 | CFD_A | CFD_C | - | - | -14297.6 | 21.45617 | - | - | -2.47535 |
| 2 | CFD_A | CFD_D | - | - | -12048.9 | -3149.85 | - | - | 1.300389 |
| 2 | CFD_B | CFD_C | - | - | 2367.514 | 12.96843 | - | - | -4.56926 |
| 2 | CFD_B | CFD_D | - | - | 3332.51 | -5550.77 | - | - | -2.62519 |
| 2 | CFD_C | CFD_D | - | - | 25.47685 | -6938.24 | - | - | -3.48799 |
| 3 | CFD_A | CFD_B | CFD_C | - | -17952.4 | 3231.161 | 15.33274 | - | -4.27837 |
| 3 | CFD_A | CFD_B | CFD_D | - | -14901 | 3625.547 | -3682.68 | - | -1.91047 |
| 3 | CFD_A | CFD_C | CFD_D | - | -11867.7 | 29.77496 | -6297.06 | - | -3.27266 |
| 3 | CFD_B | CFD_C | CFD_D | - | 2539.144 | 20.39409 | -7416.16 | - | -5.10001 |
| 4 | CFD_A | CFD_B | CFD_C | CFD_D | -15259.7 | 3164.286 | 25.28346 | -7036.88 | -5.24682 |

In the case where the specimen obtained from the subject is dry feces, the biomarker to be measured in step 1a includes two or more substances selected from the substances of No. 63 to No. 66 in Group A (3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate), and measurement of the relative area values (relative concentrations) of the two or more substances is performed by LC-MS/MS under conditions described in the Examples, the combined variable can be determined from the concentrations of the two or more substances on the basis of the expression (1), for example, with use of coefficients and a constant term shown in Table 4. In Table 4, "LFD_A" refers to 3-methyladipic acid, "LFD_B" refers to 2-hydroxyquinoline, "LFD_C" refers to 7-(tert-butyl)-4-imino-1,2,3,4,5,6-hexahydropyrido[3,4-d] pyridazine-1,5-dione, and "LFD_D" refers to 2,4-dihydroxyheptadec-16-en-1-yl acetate.

[Table 4]

| Table 4 (Dry feces, No. 63 to No. 66 in Group A) | | | | | | | | | |
|---|---|---|---|---|---|---|---|---|---|
| Number of substances combined | First substance | Second substance | Third substance | Fourth substance | Coefficient 1 | Coefficient 2 | Coefficient 3 | Coefficient 4 | Constant term |
| 2 | LFD_A | LFD_B | - | - | 6.816E-09 | 5.138E-09 | - | - | -1.33186 |
| 2 | LFD_A | LFD_C | - | - | 1.158E-08 | -6.306E-09 | - | - | -0.85664 |
| 2 | LFD_A | LFD_D | - | - | 7.986E-09 | 5.644E-08 | - | - | -1.9842 |
| 2 | LFD_B | LFD_C | - | - | 4.742E-09 | -2.148E-09 | - | - | 0.184961 |
| 2 | LFD_B | LFD_D | - | - | 3.336E-09 | 4.479E-08 | - | - | -0.58412 |
| 2 | LFD_C | LFD_D | - | - | -1.988E-09 | 4.568E-08 | - | - | -0.16848 |
| 3 | LFD_A | LFD_B | LFD_C | - | 1.170E-08 | 8.392E-09 | -6.580E-09 | - | -1.07845 |
| 3 | LFD_A | LFD_B | LFD_D | - | 8.358E-09 | 5.395E-09 | 5.634E-08 | - | -2.20669 |
| 3 | LFD_A | LFD_C | LFD_D | - | 1.392E-08 | -6.947E-09 | 7.283E-08 | - | -1.9092 |
| 3 | LFD_B | LFD_C | LFD_D | - | 4.312E-09 | -2.103E-09 | 4.508E-08 | - | -0.26513 |
| 4 | LFD_A | LFD_B | LFD_C | LFD_D | 1.412E-08 | 9.098E-09 | -7.237E-09 | 7.650E-08 | -2.22191 |

In the case where the specimen obtained from the subject is wet feces, the biomarker to be measured in step 1a includes two or more substances selected from the substances of No. 1 to No. 4 in Group B (formylanthranilic acid, glycerol, formiminoglutamic acid, and 5,6-dihydroxyindole), and measurement of the relative area values (relative concentrations) of the two or more substances is performed by CE-FTMS under conditions described in the Examples, the combined variable can be determined from the concentrations of the two or more substances on the basis of the expression (1), for example, with use of coefficients and a constant term shown in Table 5. In Table 5, "CFW_A" refers to formylanthranilic acid, "CFW_B" refers to glycerol, "CFW_C" refers to formiminoglutamic acid, and "CFW_D" refers to 5,6-dihydroxyindole.

[Table 5]

Table 5 (Wet feces, No. 1 to No. 4 in Group B)

| Number of substances combined | First substance | Second substance | Third substance | Fourth substance | Coefficient 1 | Coefficient 2 | Coefficient 3 | Coefficient 4 | Constant term |
|---|---|---|---|---|---|---|---|---|---|
| 2 | CFW_A | CFW_B | - | - | -56373.7 | 119.734 | - | - | -3.7345 |
| 2 | CFW_A | CFW_C | - | - | -56821.9 | -2934.36 | - | - | 1.5299 |
| 2 | CFW_A | CFW_D | - | - | -52229.5 | -20227.9 | - | - | 1.6040 |
| 2 | CFW_B | CFW_C | - | - | 166.4293 | -6517.87 | - | - | -5.8306 |
| 2 | CFW_B | CFW_D | - | - | 171.4117 | -29442.1 | - | - | -6.1127 |
| 2 | CFW_C | CFW_D | - | - | -5698.56 | -28141.2 | - | - | 1.5158 |
| 3 | CFW_A | CFW_B | CFW_C | - | -51130.8 | 147.1595 | -5036 | - | -4.2455 |
| 3 | CFW_A | CFW_B | CFW_D | - | -48848.2 | 152.6549 | -26110.1 | - | -4.4914 |
| 3 | CFW_A | CFW_C | CFW_D | - | -47979.2 | -4139.54 | -23670.9 | - | 2.2165 |
| 3 | CFW_B | CFW_C | CFW_D | - | 225.7562 | -9196.28 | -40574.9 | - | -6.7672 |
| 4 | CFW_A | CFW_B | CFW_C | CFW_D | -39894.7 | 206.5271 | -7710.65 | -36186.4 | -5.4896 |

EP 4 679 087 A1

In the case where the specimen obtained from the subject is wet feces, the biomarker to be measured in step 1a includes two or more substances selected from the substances of No. 46 to No. 49 in Group B (6-methylquinoline, N1-[4-(1,3-oxazol-5-yl)phenyl]cyclopropane-1-carboxamide, 2-hydroxyquinoline, and 3-methyladipic acid), and measurement of the relative area values (relative concentrations) of the two or more substances is performed by LC-MS/MS under conditions described in the Examples, the combined variable can be determined from the concentrations of the two or more substances on the basis of the expression (1), for example, with use of coefficients and a constant term shown in Table 6. In Table 6, "LFW_A" refers to 6-methylquinoline, "LFW_B" refers to N1-[4-(1,3-oxazol-5-yl)phenyl]cyclopropane-1-carboxamide, "LFW_C" refers to 2-hydroxyquinoline, and "LFW_D" refers to 3-methyladipic acid.

[Table 6]

Table 6 (Wet feces, No. 46 to No. 49 in Group B)

| Number of Substances combined | First substance | Second substance | Third substance | Fourth substance | Coefficient 1 | Coefficient 2 | Coefficient 3 | Coefficient 4 | Constant term |
|---|---|---|---|---|---|---|---|---|---|
| 2 | LFW_A | LFW_B | - | - | -2.161E-07 | -1.229E-09 | - | - | 2.479913 |
| 2 | LFW_A | LFW_C | - | - | -2.336E-07 | 3.781E-08 | - | - | 2.418926 |
| 2 | LFW_A | LFW_D | - | - | -2.392E-07 | 2.425E-08 | - | - | 1.597554 |
| 2 | LFW_B | LFW_C | - | - | -1.186E-08 | 1.971E-08 | - | - | 0.177748 |
| 2 | LFW_B | LFW_D | - | - | -1.204E-08 | 2.312E-08 | - | - | -0.68039 |
| 2 | LFW_C | LFW_D | - | - | 2.995E-08 | 2.277E-08 | - | - | -1.21854 |
| 3 | LFW_A | LFW_B | LFW_C | - | -2.316E-07 | -6.853E-10 | 3.757E-08 | - | 2.416302 |
| 3 | LFW_A | LFW_B | LFW_D | - | -2.304E-07 | -2.974E-09 | 2.465E-08 | - | 1.571628 |
| 3 | LFW_A | LFW_C | LFW_D | - | -2.516E-07 | 5.096E-08 | 2.612E-08 | - | 1.380581 |
| 3 | LFW_B | LFW_C | LFW_D | - | -1.249E-08 | 2.870E-08 | 2.430E-08 | - | -0.8981 |
| 4 | LFW_A | LFW_B | LFW_C | LFW_D | -2.441E-07 | -3.114E-09 | 5.031E-08 | 2.659E-08 | 1.367542 |

<Step 1b>

Step 1b is a step of comparing the measurement value acquired in step 1a with a reference value to acquire a comparison result.

In the case where one substance in the specimen derived from the subject is measured in step 1a, a measurement value to be acquired in step 1a is, for example, the relative concentration or absolute concentration of the one substance, and preferably the absolute concentration of the one substance. In the case where the method for measuring the one substance is CE-MS (preferably CE-FTMS), the relative concentration of the one substance is, for example, a relative area value CE. The relative concentration of the one substance may be a relative area value CE acquired from one measurement sample, or the mean of relative area values CE acquired from two or more measurement samples. In the case where the method for measuring the one substance is LC-MS (preferably LC-MS/MS), the relative concentration of the one substance is, for example, a relative area value LC. The relative concentration of the one substance may be a relative area value LC acquired from one measurement sample, or the mean of relative area values LC acquired from two or more measurement samples.

In the case where two or more substances in the specimen derived from the subject are measured in step 1a, measurement values to be acquired in step 1a are, for example, the relative concentrations or absolute concentrations of the two or more substances, and preferably the absolute concentrations of the two or more substances. In the case where the method for measuring the substances is CE-MS (preferably CE-FTMS), the relative concentration of each substance is, for example, a relative area value CE. The relative concentration of each substance may be a relative area value CE acquired from one measurement sample, or the mean of relative area values CE acquired from two or more measurement samples. In the case where the method for measuring the substances is LC-MS (preferably LC-MS/MS), the relative concentration of each substance is, for example, a relative area value LC. The relative concentration of each substance may be a relative area value LC acquired from one measurement sample, or the mean of relative area values LC acquired from two or more measurement samples.

In the case where two or more substances in the specimen derived from the subject are measured in step 1a, a measurement value to be acquired in step 1a is, for example, the combined variable determined from the concentrations of the two or more substances, and preferably the combined variable determined from the concentrations of the two or more substances on the basis of the expression (1). The concentrations to be used in determining the combined variable may be relative concentrations or absolute concentrations, but are preferably absolute concentrations.

In the case where one substance in the specimen derived from the subject is measured in step 1a, a measurement value for the one substance is compared with a reference value for the one substance to acquire a comparison result in step 1b.

In the case where the measurement value for the one substance is a relative concentration, the reference value for the one substance is also a relative concentration. The latter term "relative concentration" is synonymous with the former term "relative concentration", and the latter relative concentration is determined in the same manner as for the former relative concentration. In the case where the measurement value for the one substance is a relative area value CE, the reference value for the one substance is also a relative area value CE. In the case where the measurement value for the one substance is a relative area value LC, the reference value for the one substance is also a relative area value LC.

In the case where the measurement value for the one substance is an absolute concentration, the reference value for the one substance is also an absolute concentration. The latter term "absolute concentration" is synonymous with the former term "absolute concentration", and the latter absolute concentration is determined in the same manner as for the former absolute concentration.

In the case where two or more substances in the specimen derived from the subject are measured in step 1a, measurement values for the two or more substances are compared with reference values for the two or more substances to acquire a comparison result in step 1b. For example, in the case where the substances of No. 1 and No. 2 in Group A are measured in step 1a, the measurement value for the substance of No. 1 is compared with the reference value for the substance of No. 1 and the measurement value for the substance of No. 2 is compared with the reference value for the substance of No. 2, thereby acquiring a comparison result in step 1b.

In the case where the measurement values for the two or more substances are relative concentrations, the reference values for the two or more substances are also relative concentrations. The latter term "relative concentration" is synonymous with the former term "relative concentration", and the latter relative concentrations are determined in the same manner as for the former relative concentrations. In the case where the measurement values for the substances are relative area values CE, the reference values for the substances are also relative area values CE. In the case where the measurement values for the substances are relative area values LC, the reference values for the substances are also relative area values LC.

In the case where the measurement values for the two or more substances are absolute concentrations, the reference values for the two or more substances are also absolute concentrations. The latter term "absolute concentration" is synonymous with the former term "absolute concentration", and the latter absolute concentrations are determined in the same manner as for the former absolute concentrations.

In the case where a combined variable determined from the concentrations of the two or more substances (preferably, a combined variable determined from the concentrations of the two or more substances on the basis of the expression (1)) is used as a measurement value for the two or more substances, the reference value for the two or more substances is also a combined variable determined from the concentrations of the two or more substances (preferably, a combined variable determined from the concentrations of the two or more substances on the basis of the expression (1)). The latter term "combined variable" is synonymous with the former term "combined variable", and the latter combined variable is determined in the same manner as for the former combined variable.

<Step 1c>

Step 1c is a step of evaluating intestinal barrier function in the subject on the basis of the comparison result acquired in step 1b.

In Aspect 1, the term "positive" means that the intestinal barrier function in the subject has been reduced, and the term "negative" means that the intestinal barrier function in the subject has not been reduced.

The intestinal barrier function to be evaluated may be the intestinal barrier function of the small intestine or the intestinal barrier function of the large intestine, but is preferably the intestinal barrier function of the small intestine.

In particular, the intestinal barrier function to be evaluated is the physical barrier function including tight junction structures.

For substances selected from Group A, the intestinal barrier function in the subject can be evaluated on the basis of whether the comparison result acquired in step 1b satisfies the criterion shown in Table 7. Specifically, for each substance being "high" with respect to the criterion shown in Table 7, if the measurement value acquired in step 1a is higher than the reference value (i.e., the comparison result acquired in step 1b satisfies the criterion shown in Table 7), the case can be evaluated as being positive; if the measurement value acquired in step 1a is equal to or lower than the reference value (i.e., the comparison result acquired in step 1b does not satisfy the criterion shown in Table 7), the case can be evaluated as being negative. For each substance being "low" with respect to the criterion shown in Table 7, if the measurement value acquired in step 1a is lower than the reference value (i.e., the comparison result acquired in step 1b satisfies the criterion shown in Table 7), the case can be evaluated as being positive; if the measurement value acquired in step 1a is equal to or higher than the reference value (i.e., the comparison result acquired in step 1b does not satisfy the criterion shown in Table 7), the case can be evaluated as being negative. The criterion shown in Table 7 can also be applied to aspects other than Aspect 1.

For substances selected from Group B, the intestinal barrier function in the subject can be evaluated on the basis of whether the comparison result acquired in step 1b satisfies the criterion shown in Table 8. Specifically, for each substance being "high" with respect to the criterion shown in Table 8, if the measurement value acquired in step 1a is higher than the reference value (i.e., the comparison result acquired in step 1b satisfies the criterion shown in Table 8), the case can be evaluated as being positive; if the measurement value acquired in step 1a is equal to or lower than the reference value (i.e., the comparison result acquired in step 1b does not satisfy the criterion shown in Table 8), the case can be evaluated as being negative. For each substance being "low" with respect to the criterion shown in Table 8, if the measurement value acquired in step 1a is lower than the reference value (i.e., the comparison result acquired in step 1b satisfies the criterion shown in Table 8), the case can be evaluated as being positive; if the measurement value acquired in step 1a is equal to or higher than the reference value (i.e., the comparison result acquired in step 1b does not satisfy the criterion shown in Table 8), the case can be evaluated as being negative. The criterion shown in Table 8 can also be applied to aspects other than Aspect 1.

In the case where one substance selected from Group A is measured in step 1a, if a comparison result for the one substance satisfies the criterion shown in Table 7, the case can be evaluated as being positive; if a comparison result for the one substance does not satisfy the criterion shown in Table 7, the case can be evaluated as being negative.

In the case where two or more substances selected from Group A are measured in step 1a, if a comparison result for at least one substance of the two or more substances satisfies the criterion shown in Table 7, the case can be evaluated as being positive; if none of comparison results for the two or more substances satisfies the criterion shown in Table 7, the case can be evaluated as being negative. The larger the number of substances with comparison results satisfying the criteria shown in Table 7, the higher the probability of being positive. Accordingly, two or more (e.g., two, three, or four or more) substances selected from Group A can be measured in step 1a to achieve enhanced accuracy of evaluating intestinal barrier function in the subject.

In the case where one substance selected from Group B is measured in step 1a, if a comparison result for the one substance satisfies the criterion shown in Table 8, the case can be evaluated as being positive; if a comparison result for the one substance does not satisfy the criterion shown in Table 8, the case can be evaluated as being negative.

In the case where two or more substances selected from Group B are measured in step 1a, if a comparison result for at least one substance of the two or more substances satisfies the criterion shown in Table 8, the case can be evaluated as being positive; if none of comparison results for the two or more substances satisfies the criterion shown in Table 8, the case can be evaluated as being negative. The larger the number of substances with comparison results satisfying the criteria shown in Table 8, the higher the probability of being positive. Accordingly, two or more (e.g., two, three, or four or more) substances selected from Group B can be measured in step 1a to achieve enhanced accuracy of evaluating intestinal barrier function in the subject.

[Table 7-1]

| Table 7 (Group A) | | |
|---|---|---|
| No. | Substance Name | Criterion |
| 1 | Formylanthranilic acid | High |
| 2 | Diethanolamine | Low |
| 3 | Glycerol | Low |
| 4 | 5,6-Dihydroxyindole | High |
| 5 | Formiminoglutamic acid | High |
| 6 | Leucine | Low |
| 7 | Myristoleic acid | Low |
| 8 | 3-Aminopropane-1,2-diol | Low |
| 9 | 4-Guanidinobutyric acid | Low |
| 10 | Phenlyalanine | Low |
| 11 | 2'-Deoxyadenosine and 5'-Deoxyadenosine | High |
| 12 | Lipoamide | Low |
| 13 | 3-Guanidinopropionic acid | Low |
| 14 | Acetanilide | High |
| 15 | Histidine | Low |
| 16 | γ-glutamyl-2-aminobutyric acid | High |
| 17 | Methionine sulfoxide | Low |
| 18 | Phenylhydrazine | Low |
| 19 | Tryptophan | Low |
| 20 | Tyrosine | Low |
| 21 | Hydroxyindole | High |
| 22 | Alanine | Low |
| 23 | p-Aminobenzoic acid | High |
| 24 | Penicillamine | Low |
| 25 | 2-Cyanopyridine | Low |
| 26 | Glutamine | Low |
| 27 | 2,4-Diaminobutyric acid | High |
| 28 | Trigonelline | Low |
| 29 | 2-Methylserine | Low |
| 30 | Glycylglycine | Low |
| 31 | N,N-Dimethylhistidine | Low |
| 32 | Valine | Low |
| 33 | N-Acetylphenylalanine | Low |
| 34 | Isoleucine | Low |

(continued)

| Table 7 (Group A) | | |
|---|---|---|
| No. | Substance Name | Criterion |
| 35 | Methionine | Low |
| 36 | γ-glutamyl-valyl-glycine | Low |
| 37 | N-Acetylaspartic acid | High |
| 38 | Threonic acid | Low |
| 39 | Alloisoleucine | Low |
| 40 | Arginine ethyl ester | Low |

[Table 7-2]

| Table 7 (Continued) | | |
|---|---|---|
| No. | Substance Name | Criterion |
| 41 | 2,6-Diaminopimelic acid | High |
| 42 | Glucose 6-phosphate | High |
| 43 | Adenosine | Low |
| 44 | Mucic acid | Low |
| 45 | Thymidine | Low |
| 46 | Glycocholic acid | Low |
| 47 | 2-Deoxyribose 1-phosphate | High |
| 48 | Isoleucyl-prolyl-proline | Low |
| 49 | Sedoheptulose 7-phosphate | High |
| 50 | Fumaric acid | Low |
| 51 | N-Acetylmethionine | Low |
| 52 | Fructose 6-phosphate | High |
| 53 | Glutamylglutamic acid | Low |
| 54 | Cysteine | Low |
| 55 | N-Acetylglucosamine 1-phosphate | High |
| 56 | Ribose 5-phosphate | High |
| 57 | Glucose 1-phosphate | High |
| 58 | 3'-Cytidylic acid and 2'-Cytidylic acid | High |
| 59 | Dihydroxyacetone phosphate | High |
| 60 | 4-Oxovaleric acid | Low |
| 61 | 2-Deoxyglucose 6-phosphate | High |
| 62 | Glucosamine 6-phosphate | High |
| 63 | 3-Methyladipic acid | Low |
| 64 | 2-Hydroxyquinoline | Low |
| 65 | 7-(tert-butyl)-4-imino-1,2,3,4,5,6-hexahydropyrido[3,4-d]pyridazine-1,5-dione) | Low |
| 66 | 2,4-dihydroxyheptadec-16-en-1-yl acetate | Low |
| 67 | α-Aspartylphenylalanine | Low |
| 68 | N1-[4-(1,3-Oxazol-5-yl)phenyl]cyclopropane-1-carboxamide | High |

(continued)

| Table 7 (Continued) | | |
|---|---|---|
| No. | Substance Name | Criterion |
| 69 | 1-(2,4-Dihydroxyquinolin-3-yl)ethan-1-one | Low |
| 70 | 4-(Diethylamino)salicylaldehyde | High |
| 71 | Atenolol | Low |
| 72 | 5-Hydroxyindole | High |
| 73 | 6-Methylquinoline | High |
| 74 | Guanine | Low |
| 75 | Carboxyibuprofen | Low |
| 76 | 3-Isopropylmalic acid | Low |
| 77 | Bis(methylbenzylidene)sorbitol | Low |
| 78 | 2-Furoic acid | Low |
| 79 | Orotic acid | High |

[Table 7-3]

| Table 7 (Continued) | | |
|---|---|---|
| No. | Substance Name | Criterion |
| 80 | 2-Acetamido-N-β-aspartyl-2-deoxyhexopyranosylamine | Low |
| 81 | Xanthosine | High |
| 82 | 1-propyl-1H-benzo[d]imidazole hydrobromide | High |
| 83 | 4-Phenolsulfonic acid | Low |
| 84 | Xanthohumol | Low |
| 85 | Laurolactam | Low |
| 86 | Adenine | Low |
| 87 | 2'-Deoxyguanosine | Low |
| 88 | 6-Hydroxypicolinic acid | Low |
| 89 | D-(-)-Quinic acid | Low |
| 90 | 4-{[(4,6-Dimethoxypyrimidin-2-yl)amino]methylidene}-2-phenyl-4,5-dihydro-1,3-oxazol-5-one | Low |
| 91 | 4-[(6E)-3-Hydroxy-8,10-dimethyl-2-(methylamino)-6-dodecen-1-yl]phenol | Low |
| 92 | Ethyl 2-cyano-3-(tetrahydro-3-thiophenylamino)acrylate | Low |
| 93 | 3-Succinoylpyridine | Low |
| 94 | Proline | Low |
| 95 | L-threo-3-Phenylserine | High |
| 96 | (2,4-Diamino-5-pyrimidinyl)(3,4,5-trimethoxyphenyl)methanone | Low |
| 97 | 3-Morpholino-4-tetrahydro-1H-pyrrol-1-ylcyclobut-3-ene-1,2-dione | Low |
| 98 | 16-Hydroxyhexadecanoic acid | Low |
| 99 | Syringic acid | Low |
| 100 | Tranexamic acid | Low |
| 101 | 4-(tert-butyl)phenyl 3,5-dimethylisoxazole-4-carboxylate | Low |
| 102 | 3-Methylhistidine | Low |

(continued)

| Table 7 (Continued) | | |
|---|---|---|
| No. | Substance Name | Criterion |
| 103 | 5-Methoxyindole | High |
| 104 | Perillic acid | Low |
| 105 | 6-Methyl[1,2,4]triazolo[4,3-b]pyridazin-8-ol | High |
| 106 | Piperonylonitrile | High |
| 107 | N1-(5-Chloro-2-hydroxyphenyl)-2-(morpholinomethyl)benzamide | Low |
| 108 | N-Methylhistamine | Low |
| 109 | Dibutylone | Low |
| 110 | (-)-Epigallocatechin | Low |
| 111 | Myriocin | Low |
| 112 | 4-Indolecarbaldehyde | High |
| 113 | Uracil | High |
| 114 | Dihydrothymine | Low |
| 115 | 3-Methylglutaric acid | Low |
| 116 | 1,5-Anhydro-D-glucitol | Low |

[Table 7-4]

| Table 7 (Continued) | | |
|---|---|---|
| No. | Substance Name | Criterion |
| 117 | Valethamate | Low |
| 118 | DL-Arginine | Low |
| 119 | (R)-Equol | High |
| 120 | Theobromine | Low |
| 121 | 1,5-Isoquinolinediol | Low |
| 122 | 12-Hydroxydodecanoic acid | Low |
| 123 | 1,3,5-trihydroxy-4-{[(2E)-3-(3-hydroxy-4-methoxyphenyl)prop-2-enoyl]oxy}cyclohexane-1-carboxylic acid | Low |
| 124 | 3-Hydroxypicolinic acid | Low |
| 125 | Theophylline | Low |
| 126 | 3,4-Dihydroxybenzaldehyde | Low |
| 127 | Acetylcholine | High |
| 128 | Tridecylic acid | High |

[Table 8-1]

| Table 8 (Group B) | | |
|---|---|---|
| NO. | Substance Name | Criterion |
| 1 | Formylanthranilic acid | High |
| 2 | Glycerol | Low |
| 3 | Formiminoglutamic acid | High |
| 4 | 5,6-Dihydroxyindole | High |

(continued)

| Table 8 (Group B) | | |
|---|---|---|
| NO. | Substance Name | Criterion |
| 5 | 3-Aminopropane-1,2-diol | Low |
| 6 | 4-Guanidinobutyric acid | Low |
| 7 | 2'-Deoxyadenosine and 5'-Deoxyadenosine | High |
| 8 | Acetanilide | High |
| 9 | Pyrophosphate | High |
| 10 | Myristoleic acid | Low |
| 11 | Diethanolamine | Low |
| 12 | Thiaproline | High |
| 13 | Hydroxyindole | High |
| 14 | 2,6-Diaminopimelic acid | High |
| 15 | p-Aminobenzoic acid | High |
| 16 | Lipoamide | Low |
| 17 | Phenylhydrazine | Low |
| 18 | 3-Guanidinopropionic acid | Low |
| 19 | N-Acetylaspartic acid | High |
| 20 | 2,4-Diaminobutyric acid | High |
| 21 | γ-glutamyl-2-aminobutyric acid | High |
| 22 | Cysteine | Low |
| 23 | Glycerol 3-phosphate | High |
| 24 | Glucose 6-phosphate | High |
| 25 | Mucic acid | Low |
| 26 | Trigonelline | Low |
| 27 | N-Carbamoylaspartic acid | High |
| 28 | Threonic acid | Low |
| 29 | Glycocholic acid | Low |
| 30 | Isobutyric acid and Butyric acid | High |
| 31 | 2-Deoxyribose 1-phosphate | High |
| 32 | Arginine ethyl ester | Low |
| 33 | N-Methylglutamic acid | High |
| 34 | Pterin | High |
| 35 | N-Acetylphenylalanine | Low |
| 36 | Ribulose 5-phosphate | High |
| 37 | Fructose 6-phosphate | High |
| 38 | Sedoheptulose 7-phosphate | High |
| 39 | N-Acetylglucosamine 1-phosphate | High |
| 40 | Ribose 5-phosphate | High |

[Table 8-2]

| Table 8 (Continued) | | |
| --- | --- | --- |
| NO. | Substance Name | Criterion |
| 41 | 3'-Cytidylic acid and 2'-Cytidylic acid | High |
| 42 | Dihydroxyacetone phosphate | High |
| 43 | 4-Oxovaleric acid | Low |
| 44 | Propranolol | High |
| 45 | 2-Deoxyglucose 6-phosphate | High |
| 46 | 6-Methylquinoline | High |
| 47 | N1-[4-(1,3-Oxazol-5-yl)phenyl]cyclopropane-1-carboxamide | High |
| 48 | 2-Hydroxyquinoline | Low |
| 49 | 3-Methyladipic acid | Low |
| 50 | L-threo-3-Phenylserine | High |
| 51 | 4-(Diethylamino)salicylaldehyde | High |
| 52 | 5-Methoxyindole | High |
| 53 | 7-(tert-butyl)-4-imino-1,2,3,4,5,6-hexahydropyrido[3,4-d] pyridazine-1,5-dione | Low |
| 54 | 4-Indolecarbaldehyde | High |
| 55 | Carboxyibuprofen | Low |
| 56 | Piperonylonitrile | High |
| 57 | 5-Hydroxyindole | High |
| 58 | Orotic acid | High |
| 59 | Glucose 1-phosphate | High |
| 60 | L-Alanyl-L-proline | High |
| 61 | 2-Acetamido-N-β-aspartyl-2-deoxyhexopyranosylamine | Low |
| 62 | α-Aspartylphenylalanine | Low |
| 63 | 6-Hydroxymelatonin | High |
| 64 | Xanthosine | High |
| 65 | Atenolol | Low |
| 66 | (R)-Equol | High |
| 67 | 2,4-dihydroxyheptadec-16-en-1-yl acetate | Low |
| 68 | Guanine | Low |
| 69 | 1-(4-methyl-2-pyridyl)pyrrolidine-2,5-dione | High |
| 70 | Laurolactam | Low |
| 71 | Olmesartan | High |
| 72 | Adenine | Low |
| 73 | Uracil | High |
| 74 | 6-Methyl[1,2,4]triazolo[4,3-b]pyridazin-8-ol | High |
| 75 | 1-propyl-1H-benzo[d]imidazole hydrobromide | High |
| 76 | Pseudouridine | High |
| 77 | D-(-)-Quinic acid | Low |
| 78 | Acetylcholine | High |

[Table 8-3]

| Table 8 (Continued) | | |
|---|---|---|
| NO. | Substance Name | Criterion |
| 79 | 3-Isopropylmalic acid | Low |
| 80 | 2-methoxy-5-(1H-1,2,4-triazol-5-yl)-4-(trifluoromethyl)pyridine | High |
| 81 | 3-Amino-2-naphthoic acid | High |
| 82 | Ornithine | High |
| 83 | 2'-Deoxyguanosine | Low |
| 84 | Bis(methylbenzylidene)sorbitol | Low |
| 85 | 2-Furoic acid | Low |
| 86 | 4-(tert-butyl)phenyl 3,5-dimethylisoxazole-4-carboxylate | Low |
| 87 | Pyridoxamine | High |
| 88 | Tridecylic acid | High |
| 89 | 3-Methylhistidine | Low |
| 90 | Hexanoic acid | High |
| 91 | Azobenzene | High |
| 92 | (-)-Epigallocatechin | Low |
| 93 | 3-(2-thienyl)-1,2,4-oxadiazole-5-carbohydrazide | High |
| 94 | DL-Lactic Acid | High |
| 95 | 6-Hydroxypicolinic acid | Low |
| 96 | Proline | Low |
| 97 | 1,3,5-trihydroxy-4-{[(2E)-3-(3-hydroxy-4-methoxyphenyl)prop-2-enoyl]oxy}cyclohexane-1-carboxylic acid | Low |
| 98 | Prolylglycine | High |
| 99 | 4-[(6E)-3-Hydroxy-8,10-dimethyl-2-(methylamino)-6-dodecen-1-yl]phenol | Low |

The reference value for each substance selected from Group A or Group B can be set according to a conventional method.

In an embodiment, for each substance selected from Group A or Group B, the average value of measurement values for the substance in specimens obtained from a negative group (a population with non-reduced intestinal barrier function) consisting of individuals of the same species as the subject (e.g., humans if the subject is a human) can be used as a reference value for the substance. Hereinafter, this embodiment (hereinafter, referred to as "Embodiment 1-1") will be described.

In forming the negative group, the determination of negative and positive individuals can be performed by a known testing method for intestinal barrier function (e.g., lactulose/mannitol test).

In the case where the specimen used in step 1a is dry feces, the specimens obtained from the negative group are also dry feces.

In the case where the specimen used in step 1a is wet feces, the specimens obtained from the negative group are also wet feces.

The term "measurement value" for each substance in the specimens obtained from the negative group is synonymous with that for the measurement value acquired in step 1a, and the measurement values are determined in the same manner as in step 1a.

In the case where the measurement value acquired in step 1a is the relative concentration of one substance selected from Group A or Group B, the reference value for the one substance to be used in step 1b is the average value of the relative concentrations of the one substance in the specimens obtained from the negative group. The latter term "relative concentration" is synonymous with the former term "relative concentration", and the latter relative concentrations are determined in the same manner as for the former relative concentration. In the case where the former relative concentration is a relative area value CE, the latter relative concentrations are also relative area values CE. In the case where the former relative concentration is a relative area value LC, the latter relative concentrations are also relative area values LC.

In the case where the measurement value acquired in step 1a is the absolute concentration of one substance selected from Group A or Group B, the reference value for the one substance to be used in step 1b is the average value of the absolute concentrations of the one substance in the specimens obtained from the negative group. The latter term "absolute concentration" is synonymous with the former term absolute concentration, and the latter absolute concentrations are determined in the same manner as for the former absolute concentration.

In the case where the measurement value acquired in step 1a is the relative concentrations of two or more substances selected from Group A or Group B, the reference value for each substance to be used in step 1b is the average value of the relative concentrations of the substance in the specimens obtained from the negative group. The latter term "relative concentration" is synonymous with the former term "relative concentration", and the latter relative concentrations are determined in the same manner as for the former relative concentrations. In the case where the former relative concentrations are relative area values CE, the latter relative concentrations are also relative area values CE. In the case where the former relative concentrations are relative area values LC, the latter relative concentrations are also relative area values LC.

In the case where the measurement value acquired in step 1a is the absolute concentrations of two or more substances selected from Group A or Group B, the reference value for each substance to be used in step 1b is the average value of the absolute concentrations of the substance in the specimens obtained from the negative group.

For substances selected from Group A, it is preferred to evaluate intestinal barrier function in the subject on the basis of whether the comparison result acquired in step 1b satisfies the criterion shown in Table 9; for substances selected from Group B, it is preferred to evaluate intestinal barrier function in the subject on the basis of whether the comparison result acquired in step 1b satisfies the criterion shown in Table 10.

Specifically, for a substance with a criterion of "high", a preferred criterion of x times or more, a more preferred criterion of y times or more, and an even more preferred criterion of z times or more in Table 9 or 10, if the measurement value acquired in step 1a is x times or more the average value, the case is preferably evaluated as being positive; if the measurement value acquired in step 1a is y times or more the average value, the case is more preferably evaluated as being positive; if the measurement value acquired in step 1a is z times or more the average value, the case is even more preferably evaluated as being positive. For a substance with a criterion of "low", a preferred criterion of x times or less, a more preferred criterion of y times or less, and an even more preferred criterion of z times or less in Table 9 or 10, if the measurement value acquired in step 1a is x times or less the average value, the case is preferably evaluated as being positive; if the measurement value acquired in step 1a is y times or less the average value, the case is more preferably evaluated as being positive; if the measurement value acquired in step 1a is z times or less the average value, the case is even more preferably evaluated as being positive.

Taking the substance of No. 1 in Group A as an example, if the measurement value acquired in step 1a is two times or more the average value, the case is preferably evaluated as being positive; if the measurement value acquired in step 1a is three times or more the average value, the case is more preferably evaluated as being positive; if the measurement value acquired in step 1a is five times or more the average value, the case is even more preferably evaluated as being positive.

Taking the substance of No. 2 in Group A as an example, if the measurement value acquired in step 1a is 0.8 times or less the average value, the case is preferably evaluated as being positive; if the measurement value acquired in step 1a is 0.7 times or less the average value, the case is more preferably evaluated as being positive; if the measurement value acquired in step 1a is 0.6 times or less the average value, the case is even more preferably evaluated as being positive.

[Table 9-1]

| Table 9 (Group A) | | | | | |
|---|---|---|---|---|---|
| NO. | Substance Name | Criterion | Preferred Criterion | More Preferred Criterion | Even More Preferred Criterion |
| 1 | Formylanthranilic acid | High | 2 times or more | 3 times or more | 5 times or more |
| 2 | Diethanolamine | Low | 0.8 times or less | 0.7 times or less | 0.5 times or less |
| 3 | Glycerol | Low | 0.9 times or less | 0.8 times or less | 0.6 times or less |
| 4 | 5,6-Dihydroxyindole | High | 2 times or more | 3 times or more | 5 times or more |

(continued)

| Table 9 (Group A) | | | | | |
|---|---|---|---|---|---|
| NO. | Substance Name | Criterion | Preferred Criterion | More Preferred Criterion | Even More Preferred Criterion |
| 5 | Formiminoglutamic acid | High | 1.2 times or more | 1.4 times or more | 2 times or more |
| 6 | Leucine | Low | 0.9 times or less | 0.7 times or less | 0.5 times or less |
| 7 | Myristoleic acid | Low | 0.7 times or less | 0.5 times or less | 0.3 times or less |
| 8 | 3-Aminopropane-1,2-diol | Low | 0.4 times or less | 0.3 times or less | 0.2 times or less |
| 9 | 4-Guanidinobutyric acid | Low | 0.6 times or less | 0.5 times or less | 0.3 times or less |
| 10 | Phenlyalanine | Low | 0.8 times or less | 0.7 times or less | 0.5 times or less |
| 11 | 2'-Deoxyadenosine and 5'-Deoxyadenosine | High | 1.5 times or more | 2 times or more | 3 times or more |
| 12 | Lipoamide | Low | 0.7 times or less | 0.5 times or less | 0.3 times or less |
| 13 | 3-Guanidinopropionic acid | Low | 0.4 times or less | 0.3 times or less | 0.2 times or less |
| 14 | Acetanilide | High | 1.2 times or more | 1.4 times or more | 2 times or more |
| 15 | Histidine | Low | 0.8 times or less | 0.7 times or less | 0.5 times or less |
| 16 | γ-glutamyl-2-aminobutyric acid | High | 1.2 times or more | 1.4 times or more | 2 times or more |
| 17 | Methionine sulfoxide | Low | 0.9 times or less | 0.7 times or less | 0.5 times or less |
| 18 | Phenylhydrazine | Low | 0.6 times or less | 0.5 times or less | 0.3 times or less |
| 19 | Tryptophan | Low | 0.9 times or less | 0.7 times or less | 0.5 times or less |
| 20 | Tyrosine | Low | 0.9 times or less | 0.7 times or less | 0.5 times or less |
| 21 | Hydroxyindole | High | 2 times or more | 3 times or more | 5 times or more |
| 22 | Alanine | Low | 0.9 times or less | 0.7 times or less | 0.5 times or less |
| 23 | p-Aminobenzoic acid | High | 1.5 times or more | 2 times or more | 3 times or more |

[Table 9-2]

| Table 9 (Continued) | | | | | |
|---|---|---|---|---|---|
| NO. | Substance Name | Criterion | Preferred Criterion | More Preferred Criterion | Even More Preferred Criterion |
| 24 | Penicillamine | Low | 0.9 times or less | 0.7 times or less | 0.5 times or less |
| 25 | 2-Cyanopyridine | Low | 0.7 times or less | 0.5 times or less | 0.3 times or less |
| 26 | Glutamine | Low | 0.9 times or less | 0.7 times or less | 0.5 times or less |
| 27 | 2,4-Diaminobutyric acid | High | 2 times or more | 3 times or more | 5 times or more |
| 28 | Trigonelline | Low | 0.7 times or less | 0.5 times or less | 0.3 times or less |
| 29 | 2-Methylserine | Low | 0.4 times or less | 0.3 times or less | 0.2 times or less |
| 30 | Glycylglycine | Low | 0.9 times or less | 0.7 times or less | 0.5 times or less |
| 31 | N,N-Dimethylhistidine | Low | 0.8 times or less | 0.6 times or less | 0.5 times or less |
| 32 | Valine | Low | 0.9 times or less | 0.7 times or less | 0.5 times or less |
| 33 | N-Acetylphenylalanine | Low | 0.8 times or less | 0.6 times or less | 0.4 times or less |
| 34 | Isoleucine | Low | 0.9 times or less | 0.7 times or less | 0.5 times or less |
| 35 | Methionine | Low | 0.9 times or less | 0.7 times or less | 0.5 times or less |
| 36 | $\gamma$-glutamyl-valyl-glycine | Low | 0.8 times or less | 0.6 times or less | 0.5 times or less |
| 37 | N-Acetylaspartic acid | High | 1.2 times or more | 1.4 times or more | 2 times or more |
| 38 | Threonic acid | Low | 0.3 times or less | 0.2 times or less | 0.1 times or less |
| 39 | Alloisoleucine | Low | 0.9 times or less | 0.7 times or less | 0.5 times or less |
| 40 | Arginine ethyl ester | Low | 0.9 times or less | 0.7 times or less | 0.5 times or less |
| 41 | 2,6-Diaminopimelic acid | High | 1.2 times or more | 1.4 times or more | 2 times or more |
| 42 | Glucose 6-phosphate | High | 2.0 times or more | 3.0 times or more | 4.0 times or more |
| 43 | Adenosine | Low | 0.7 times or less | 0.5 times or less | 0.3 times or less |
| 44 | Mucic acid | Low | 0.4 times or less | 0.2 times or less | 0.1 times or less |
| 45 | Thymidine | Low | 0.9 times or less | 0.7 times or less | 0.5 times or less |

[Table 9-3]

| Table 9 (Continued) | | | | | |
|---|---|---|---|---|---|
| NO. | Substance Name | Criterion | Preferred Criterion | More Preferred Criterion | Even More Preferred Criterion |
| 46 | Glycocholic acid | Low | 0.8 times or less | 0.6 times or less | 0.4 times or less |
| 47 | 2-Deoxyribose 1-phosphate | High | 1.5 times or more | 2 times or more | 3 times or more |
| 48 | Isoleucyl-prolyl-proline | Low | 0.9 times or less | 0.7 times or less | 0.4 times or less |
| 49 | Sedoheptulose 7-phosphate | High | 1.5 times or more | 3 times or more | 5 times or more |
| 50 | Fumaric acid | Low | 0.8 times or less | 0.6 times or less | 0.4 times or less |
| 51 | N-Acetylmethionine | Low | 0.7 times or less | 0.5 times or less | 0.3 times or less |

(continued)

Table 9 (Continued)

| NO. | Substance Name | Criterion | Preferred Criterion | More Preferred Criterion | Even More Preferred Criterion |
|---|---|---|---|---|---|
| 52 | Fructose 6-phosphate | High | 2 times or more | 3 times or more | 4 times or more |
| 53 | Glutamylglutamic acid | Low | 0.9 times or less | 0.7 times or less | 0.5 times or less |
| 54 | Cysteine | Low | 0.9 times or less | 0.7 times or less | 0.5 times or less |
| 55 | N-Acetylglucosamine 1-phosphate | High | 2 times or more | 3 times or more | 4 times or more |
| 56 | Ribose 5-phosphate | High | 1.1 times or more | 1.3 times or more | 1.5 times or more |
| 57 | Glucose 1-phosphate | High | 3 times or more | 5 times or more | 7 times or more |
| 58 | 3'-Cytidylic acid and 2'-Cytidylic acid | High | 8 times or more | 10 times or more | 15 times or more |
| 59 | Dihydroxyacetone phosphate | High | 1.5 times or more | 2 times or more | 3 times or more |
| 60 | 4-Oxovaleric acid | Low | 0.01 times or less | 0.003 times or less | 0.001 times or less |
| 61 | 2-Deoxyglucose 6-phosphate | High | 5 times or more | 7 times or more | 10 times or more |
| 62 | Glucosamine 6-phosphate | High | 2 times or more | 3 times or more | 4 times or more |
| 63 | 3-Methyladipic acid | Low | 0.4 times or less | 0.3 times or less | 0.2 times or less |
| 64 | 2-Hydroxyquinoline | Low | 0.9 times or less | 0.7 times or less | 0.5 times or less |
| 65 | 7-(tert-butyl)-4-imino-1,2,3,4,5,6-hexahydropyrido[3,4-d]pyridazine-1,5-dione) | Low | 0.8 times or less | 0.6 times or less | 0.4 times or less |
| 66 | 2,4-dihydroxyheptadec-16-en-1-yl acetate | Low | 0.7 times or less | 0.5 times or less | 0.3 times or less |

[Table 9-4]

Table 9 (Continued)

| NO. | Substance Name | Criterion | Preferred Criterion | More Preferred Criterion | Even More Preferred Criterion |
|---|---|---|---|---|---|
| 67 | α-Aspartylphenylalanine | Low | 0.8 times or less | 0.6 times or less | 0.4 times or less |
| 68 | N1-[4-(1,3-Oxazol-5-yl)phenyl]cyclopropane-1-carboxamide | High | 1.4 times or more | 1.6 times or more | 1.8 times or more |
| 69 | 1-(2,4-Dihydroxyquinolin-3-yl)ethan-1-one | Low | 0.1 times or less | 0.04 times or less | 0.01 times or less |

(continued)

| Table 9 (Continued) | | | | | |
|---|---|---|---|---|---|
| NO. | Substance Name | Criterion | Preferred Criterion | More Preferred Criterion | Even More Preferred Criterion |
| 70 | 4-(Diethylamino)salicylaldehyde | High | 1.5 times or more | 2 times or more | 3 times or more |
| 71 | Atenolol | Low | 0.7 times or less | 0.5 times or less | 0.3 times or less |
| 72 | 5-Hydroxyindole | High | 1.5 times or more | 2 times or more | 3 times or more |
| 73 | 6-Methylquinoline | High | 1.2 times or more | 1.4 times or more | 1.6 times or more |
| 74 | Guanine | Low | 0.6 times or less | 0.4 times or less | 0.2 times or less |
| 75 | Carboxyibuprofen | Low | 0.2 times or less | 0.1 times or less | 0.05 times or less |
| 76 | 3-Isopropylmalic acid | Low | 0.7 times or less | 0.5 times or less | 0.3 times or less |
| 77 | Bis(methylbenzylidene)sorbitol | Low | 0.9 times or less | 0.7 times or less | 0.5 times or less |
| 78 | 2-Furoic acid | Low | 0.8 times or less | 0.6 times or less | 0.4 times or less |
| 79 | Orotic acid | High | 1.4 times or more | 1.6 times or more | 1.8 times or more |
| 80 | 2-Acetamido-N-β-aspartyl-2-deoxyhexopyranosylamine | Low | 0.8 times or less | 0.6 times or less | 0.4 times or less |
| 81 | Xanthosine | High | 1.5 times or more | 2 times or more | 3 times or more |
| 82 | 1-propyl-1H-benzo[d]imidazole hydrobromide | High | 1.5 times or more | 3.0 times or more | 5.0 times or more |
| 83 | 4-Phenolsulfonic acid | Low | 0.1 times or less | 0.08 times or less | 0.05 times or less |
| 84 | Xanthohumol | Low | 0.8 times or less | 0.6 times or less | 0.4 times or less |
| 85 | Laurolactam | Low | 0.9 times or less | 0.8 times or less | 0.6 times or less |
| 86 | Adenine | Low | 0.4 times or less | 0.2 times or less | 0.1 times or less |

[Table 9-5]

| Table 9 (Continued) | | | | | |
|---|---|---|---|---|---|
| NO. | Substance Name | Criterion | Preferred Criterion | More Preferred Criterion | Even More Preferred Criterion |
| 87 | 2'-Deoxyguanosine | Low | 0.6 times or less | 0.4 times or less | 0.2 times or less |

(continued)

| Table 9 (Continued) | | | | | |
|---|---|---|---|---|---|
| NO. | Substance Name | Criterion | Preferred Criterion | More Preferred Criterion | Even More Preferred Criterion |
| 88 | 6-Hydroxypicolinic acid | Low | 0.9 times or less | 0.8 times or less | 0.6 times or less |
| 89 | D-(-)-Quinic acid | Low | 0.4 times or less | 0.3 times or less | 0.2 times or less |
| 90 | 4-{[(4,6-Dimethoxypyrimidin-2-yl)amino]methylidene}-2-phenyl-4,5-dihydro-1,3-oxazol-5-one | Low | 0.1 times or less | 0.04 times or less | 0.01 times or less |
| 91 | 4-[(6E)-3-Hydroxy-8,10-dimethyl-2-(methylamino)-6-dodecen-1-yl]phenol | Low | 0.8 times or less | 0.6 times or less | 0.4 times or less |
| 92 | Ethyl 2-cyano-3-(tetrahydro-3-thiophenylamino) acrylate | Low | 0.9 times or less | 0.7 times or less | 0.5 times or less |
| 93 | 3-Succinoylpyridine | Low | 0.9 times or less | 0.8 times or less | 0.6 times or less |
| 94 | Proline | Low | 0.5 times or less | 0.4 times or less | 0.3 times or less |
| 95 | L-threo-3-Phenylserine | High | 2 times or more | 3 times or more | 5 times or more |
| 96 | (2,4-Diamino-5-pyrimidinyl)(3,4,5-trimethoxyphenyl)methanone | Low | 0.7 times or less | 0.5 times or less | 0.3 times or less |
| 97 | 3-Morpholino-4-tetrahydro-1H-pyrrol-1-ylcyclobut-3-ene-1,2-dione | Low | 0.9 times or less | 0.7 times or less | 0.5 times or less |
| 98 | 16-Hydroxyhexadecanoic acid | Low | 0.7 times or less | 0.5 times or less | 0.3 times or less |
| 99 | Syringic acid | Low | 0.5 times or less | 0.4 times or less | 0.3 times or less |
| 100 | Tranexamic acid | Low | 0.01 times or less | 0.003 times or less | 0.001 times or less |
| 101 | 4-(tert-butyl)phenyl 3,5-dimethylisoxazole-4-carboxylate | Low | 0.8 times or less | 0.6 times or less | 0.4 times or less |
| 102 | 3-Methylhistidine | Low | 0.7 times or less | 0.5 times or less | 0.3 times or less |
| 103 | 5-Methoxyindole | High | 5 times or more | 14 times or more | 20 times or more |

[Table 9-6]

| Table 9 (Continued) | | | | | |
|---|---|---|---|---|---|
| NO. | Substance Name | Criterion | Preferred Criterion | More Preferred Criterion | Even More Preferred Criterion |
| 104 | Perillic acid | Low | 0.5 times or less | 0.4 times or less | 0.3 times or less |
| 105 | 6-Methyl[1,2,4]triazolo[4,3-b]pyridazin-8-ol | High | 1.1 times or more | 1.3 times or more | 1.6 times or more |

(continued)

| Table 9 (Continued) | | | | | |
|---|---|---|---|---|---|
| NO. | Substance Name | Criterion | Preferred Criterion | More Preferred Criterion | Even More Preferred Criterion |
| 106 | Piperonylonitrile | High | 1.5 times or more | 1.8 times or more | 2 times or more |
| 107 | N1-(5-Chloro-2-hydroxyphenyl)-2-(morpholinomethyl)benzamide | Low | 0.5 times or less | 0.4 times or less | 0.3 times or less |
| 108 | N-Methylhistamine | Low | 0.7 times or less | 0.5 times or less | 0.3 times or less |
| 109 | Dibutylone | Low | 0.5 times or less | 0.4 times or less | 0.3 times or less |
| 110 | (-)-Epigallocatechin | Low | 0.3 times or less | 0.2 times or less | 0.1 times or less |
| 111 | Myriocin | Low | 0.7 times or less | 0.5 times or less | 0.3 times or less |
| 112 | 4-Indolecarbaldehyde | High | 1.4 times or more | 1.7 times or more | 2 times or more |
| 113 | Uracil | High | 1.3 times or more | 1.5 times or more | 2 times or more |
| 114 | Dihydrothymine | Low | 0.8 times or less | 0.6 times or less | 0.4 times or less |
| 115 | 3-Methylglutaric acid | Low | 0.3 times or less | 0.2 times or less | 0.1 times or less |
| 116 | 1,5-Anhydro-D-glucitol | Low | 0.4 times or less | 0.3 times or less | 0.2 times or less |
| 117 | Valethamate | Low | 0.9 times or less | 0.8 times or less | 0.6 times or less |
| 118 | DL-Arginine | Low | 0.4 times or less | 0.3 times or less | 0.2 times or less |
| 119 | (R)-Equol | High | 2 times or more | 3 times or more | 5 times or more |
| 120 | Theobromine | Low | 0.8 times or less | 0.6 times or less | 0.4 times or less |
| 121 | 1,5-Isoquinolinediol | Low | 0.3 times or less | 0.2 times or less | 0.1 times or less |
| 122 | 12-Hydroxydodecanoic acid | Low | 0.9 times or less | 0.7 times or less | 0.5 times or less |

[Table 9-7]

| Table 9 (continued) | | | | | |
|---|---|---|---|---|---|
| NO. | Substance Name | Criterion | Preferred Criterion | More Preferred Criterion | Even More Preferred Criterion |
| 123 | 1,3,5-trihydroxy-4-{[(2E)-3-(3-hydroxy-4-methoxyphenyl)prop-2-enoyl]oxy}cyclohexane-1-carboxylic acid | Low | 0.9 times or less | 0.8 times or less | 0.6 times or less |

(continued)

| Table 9 (continued) | | | | | | |
|---|---|---|---|---|---|---|
| NO. | Substance Name | Criterion | Preferred Criterion | More Preferred Criterion | Even More Preferred Criterion |
| 124 | 3-Hydroxypicolinic acid | Low | 0.9 times or less | 0.8 times or less | 0.6 times or less |
| 125 | Theophylline | Low | 0.9 times or less | 0.7 times or less | 0.5 times or less |
| 126 | 3,4-Dihydroxybenzaldehyde | Low | 0.9 times or less | 0.7 times or less | 0.5 times or less |
| 127 | Acetylcholine | High | 2 times or more | 3 times or more | 5 times or more |
| 128 | Tridecylic acid | High | 1.3 times or more | 1.5 times or more | 2 times or more |

[Table 10-1]

| Table 10 (Group B) | | | | | |
|---|---|---|---|---|---|
| NO. | Substance Name | Criterion | Preferred Criterion | More Preferred Criterion | Even More Preferred Criterion |
| 1 | Formylanthranilic acid | High | 2 times or more | 3 times or more | 5 times or more |
| 2 | Glycerol | Low | 0.95 times or less | 0.9 times or less | 0.7 times or less |
| 3 | Formiminoglutamic acid | High | 1.3 times or more | 1.5 times or more | 2 times or more |
| 4 | 5,6-Dihydroxyindole | High | 2 times or more | 3 times or more | 5 times or more |
| 5 | 3-Aminopropane-1,2-diol | Low | 0.4 times or less | 0.3 times or less | 0.2 times or less |
| 6 | 4-Guanidinobutyric acid | Low | 0.8 times or less | 0.6 times or less | 0.4 times or less |
| 7 | 2'-Deoxyadenosine and 5'-Deoxyadenosine | High | 1.5 times or more | 2 times or more | 3 times or more |
| 8 | Acetanilide | High | 1.5 times or more | 1.8 times or more | 2 times or more |
| 9 | Pyrophosphate | High | 1.2 times or more | 1.4 times or more | 2 times or more |
| 10 | Myristoleic acid | Low | 0.5 times or less | 0.4 times or less | 0.3 times or less |
| 11 | Diethanolamine | Low | 0.9 times or less | 0.8 times or less | 0.6 times or less |
| 12 | Thiaproline | High | 1.4 times or more | 1.7 times or more | 2 times or more |
| 13 | Hydroxyindole | High | 1.7 times or more | 2.5 times or more | 4 times or more |
| 14 | 2,6-Diaminopimelic acid | High | 1.3 times or more | 1.5 times or more | 2 times or more |

(continued)

| Table 10 (Group B) | | | | | |
|---|---|---|---|---|---|
| NO. | Substance Name | Criterion | Preferred Criterion | More Preferred Criterion | Even More Preferred Criterion |
| 15 | p-Aminobenzoic acid | High | 1.5 times or more | 2 times or more | 3 times or more |
| 16 | Lipoamide | Low | 0.7 times or less | 0.5 times or less | 0.3 times or less |
| 17 | Phenylhydrazine | Low | 0.7 times or less | 0.5 times or less | 0.3 times or less |
| 18 | 3-Guanidinopropionic acid | Low | 0.7 times or less | 0.5 times or less | 0.3 times or less |
| 19 | N-Acetylaspartic acid | High | 1.4 times or more | 1.7 times or more | 2 times or more |
| 20 | 2,4-Diaminobutyric acid | High | 2 times or more | 3 times or more | 5 times or more |
| 21 | γ-glutamyl-2-aminobutyric acid | High | 1.1 times or more | 1.3 times or more | 1.6 times or more |
| 22 | Cysteine | Low | 0.9 times or less | 0.8 times or less | 0.6 times or less |
| 23 | Glycerol 3-phosphate | High | 1.5 times or more | 2 times or more | 3 times or more |

[Table 10-2]

| Table 10 (Continued) | | | | | |
|---|---|---|---|---|---|
| NO. | Substance Name | Criterion | Preferred Criterion | More Preferred Criterion | Even More Preferred Criterion |
| 24 | Glucose 6-phosphate | High | 2 times or more | 3 times or more | 5 times or more |
| 25 | Mucic acid | Low | 0.4 times or less | 0.3 times or less | 0.2 times or less |
| 26 | Trigonelline | Low | 0.8 times or less | 0.6 times or less | 0.4 times or less |
| 27 | N-Carbamoylaspartic acid | High | 1.5 times or more | 1.8 times or more | 2 times or more |
| 28 | Threonic acid | Low | 0.4 times or less | 0.3 times or less | 0.2 times or less |
| 29 | Glycocholic acid | Low | 0.8 times or less | 0.6 times or less | 0.4 times or less |
| 30 | Isobutyric acid and Butyric acid | High | **1.1** times or more | 1.3 times or more | 1.6 times or more |
| 31 | 2-Deoxyribose 1-phosphate | High | 1.5 times or more | 2 times or more | 3 times or more |
| 32 | Arginine ethyl ester | Low | 0.95 times or less | 0.9 times or less | 0.7 times or less |
| 33 | N-Methylglutamic acid | High | 1.05 times or more | 1.1 times or more | 1.2 times or more |

(continued)

| Table 10 (Continued) | | | | | |
|---|---|---|---|---|---|
| NO. | Substance Name | Criterion | Preferred Criterion | More Preferred Criterion | Even More Preferred Criterion |
| 34 | Pterin | High | 1.1 times or more | 1.3 times or more | 1.6 times or more |
| 35 | N-Acetylphenylalanine | Low | 0.8 times or less | 0.6 times or less | 0.4 times or less |
| 36 | Ribulose 5-phosphate | High | 1.5 times or more | 2 times or more | 3 times or more |
| 37 | Fructose 6-phosphate | High | 2 times or more | 3 times or more | 5 times or more |
| 38 | Sedoheptulose 7-phosphate | High | 1.5 times or more | 2 times or more | 3 times or more |
| 39 | N-Acetylglucosamine 1-phosphate | High | 2 times or more | 3 times or more | 5 times or more |
| 40 | Ribose 5-phosphate | High | 1.05 times or more | 1.1 times or more | 1.2 times or more |
| 41 | 3'-Cytidylic acid and 2'-Cytidylic acid | High | 8 times or more | 10 times or more | 15 times or more |
| 42 | Dihydroxyacetone phosphate | High | 1.5 times or more | 2 times or more | 3 times or more |
| 43 | 4-Oxovaleric acid | Low | 0.01 times or less | 0.003 times or less | 0.001 times or less |
| 44 | Propranolol | High | 4 times or more | 6 times or more | 8 times or more |
| 45 | 2-Deoxyglucose 6-phosphate | High | 4 times or more | 6 times or more | 8 times or more |

[Table 10-3]

| Table 10 (Continued) | | | | | |
|---|---|---|---|---|---|
| NO. | Substance Name | Criterion | Preferred Criterion | More Preferred Criterion | Even More Preferred Criterion |
| 46 | 6-Methylquinoline | High | 1.5 times or more | 1.8 times or more | 2 times or more |
| 47 | N1-[4-(1,3-Oxazol-5-yl)phenyl]cyclopropane-1-carboxamide | High | 1.5 times or more | 2 times or more | 3 times or more |
| 48 | 2-Hydroxyquinoline | Low | 0.9 times or less | 0.7 times or less | 0.5 times or less |
| 49 | 3-Methyladipic acid | Low | 0.5 times or less | 0.4 times or less | 0.3 times or less |
| 50 | L-threo-3-Phenylserine | High | 1.3 times or more | 1.5 times or more | 2 times or more |
| 51 | 4-(Diethylamino)salicylaldehyde | High | 1.5 times or more | 2 times or more | 3 times or more |
| 52 | 5-Methoxyindole | High | 5 times or more | 14 times or more | 20 times or more |
| 53 | 7-(tert-butyl)-4-imino-1,2,3,4,5,6-hexahydropyrido[3,4-d]pyridazine-1,5-dione | Low | 0.8 times or less | 0.6 times or less | 0.4 times or less |

(continued)

Table 10 (Continued)

| NO. | Substance Name | Criterion | Preferred Criterion | More Preferred Criterion | Even More Preferred Criterion |
|---|---|---|---|---|---|
| 54 | 4-Indolecarbaldehyde | High | 1.5 times or more | 2 times or more | 3 times or more |
| 55 | Carboxyibuprofen | Low | 0.3 times or less | 0.2 times or less | 0.1 times or less |
| 56 | Piperonylonitrile | High | 1.5 times or more | 2 times or more | 3 times or more |
| 57 | 5-Hydroxyindole | High | 1.7 times or more | 2.5 times or more | 4 times or more |
| 58 | Orotic acid | High | 1.4 times or more | 1.7 times or more | 2 times or more |
| 59 | Glucose 1-phosphate | High | 1.5 times or more | 2 times or more | 3 times or more |
| 60 | L-Alanyl-L-proline | High | 1.05 times or more | 1.1 times or more | 1.2 times or more |
| 61 | 2-Acetamido-N-β-aspartyl-2-deoxyhexopyranosylamine | Low | 0.9 times or less | 0.7 times or less | 0.5 times or less |
| 62 | α-Aspartylphenylalanine | Low | 0.8 times or less | 0.6 times or less | 0.4 times or less |
| 63 | 6-Hydroxymelatonin | High | 10 times or more | 30 times or more | 40 times or more |
| 64 | Xanthosine | High | 1.7 times or more | 2.5 times or more | 4 times or more |

[Table 10-4]

Table 10 (Continued)

| NO. | Substance Name | Criterion | Preferred Criterion | More Preferred Criterion | Even More Preferred Criterion |
|---|---|---|---|---|---|
| 65 | Atenolol | Low | 0.7 times or less | 0.5 times or less | 0.3 times or less |
| 66 | (R)-Equol | High | 2 times or more | 3 times or more | 5 times or more |
| 67 | 2,4-dihydroxyheptadec-16-en-1-yl acetate | Low | 0.7 times or less | 0.5 times or less | 0.3 times or less |
| 68 | Guanine | Low | 0.7 times or less | 0.5 times or less | 0.3 times or less |
| 69 | 1-(4-methyl-2-pyridyl)pyrrolidine-2,5-dione | High | 1.3 times or more | 1.5 times or more | 2 times or more |
| 70 | Laurolactam | Low | 0.95 times or less | 0.9 times or less | 0.7 times or less |
| 71 | Olmesartan | High | 10 times or more | 30 times or more | 40 times or more |

(continued)

| Table 10 (Continued) | | | | | |
|---|---|---|---|---|---|
| NO. | Substance Name | Criterion | Preferred Criterion | More Preferred Criterion | Even More Preferred Criterion |
| 72 | Adenine | Low | 0.4 times or less | 0.3 times or less | 0.2 times or less |
| 73 | Uracil | High | 1.5 times or more | 1.8 times or more | 2 times or more |
| 74 | 6-Methyl[1,2,4]triazolo[4,3-b]pyridazin-8-ol | High | 1.3 times or more | 1.5 times or more | 2 times or more |
| 75 | 1-propyl-1H-benzo[d]imidazole hydrobromide | High | 2 times or more | 3 times or more | 5 times or more |
| 76 | Pseudouridine | High | 1.2 times or more | 1.4 times or more | 2 times or more |
| 77 | D-(-)-Quinic acid | Low | 0.4 times or less | 0.3 times or less | 0.2 times or less |
| 78 | Acetylcholine | High | 2 times or more | 3 times or more | 5 times or more |
| 79 | 3-Isopropylmalic acid | Low | 0.8 times or less | 0.6 times or less | 0.4 times or less |
| 80 | 2-methoxy-5-(1H-1,2,4-triazol-5-yl)-4-(trifluoromethyl)pyridine | High | 1.5 times or more | 2 times or more | 3 times or more |
| 81 | 3-Amino-2-naphthoic acid | High | 1.1 times or more | 1.3 times or more | 1.6 times or more |
| 82 | Ornithine | High | 1.1 times or more | 1.3 times or more | 1.6 times or more |
| 83 | 2'-Deoxyguanosine | Low | 0.7 times or less | 0.5 times or less | 0.3 times or less |
| 84 | Bis(methylbenzylidene)sorbitol | Low | 0.95 times or less | 0.9 times or less | 0.7 times or less |

[Table 10-5

| Table 10 (Continued) | | | | | |
|---|---|---|---|---|---|
| NO. | Substance Name | Criterion | Preferred Criterion | More Preferred Criterion | Even More Preferred Criterion |
| 85 | 2-Furoic acid | Low | 0.9 times or less | 0.7 times or less | 0.5 times or less |
| 86 | 4-(tert-butyl)phenyl 3,5-dimethylisoxazole-4-carboxylate | Low | 0.8 times or less | 0.6 times or less | 0.4 times or less |
| 87 | Pyridoxamine | High | 1.5 times or more | 2 times or more | 3 times or more |
| 88 | Tridecylic acid | High | 1.4 times or more | 1.7 times or more | 2 times or more |
| 89 | 3-Methylhistidine | Low | 0.7 times or less | 0.5 times or less | 0.3 times or less |

(continued)

| Table 10 (Continued) | | | | | | |
|---|---|---|---|---|---|---|
| NO. | Substance Name | Criterion | Preferred Criterion | More Preferred Criterion | Even More Preferred Criterion |
| 90 | Hexanoic acid | High | 1.5 times or more | 2 times or more | 3 times or more |
| 91 | Azobenzene | High | 1.2 times or more | 1.4 times or more | 2 times or more |
| 92 | (-)-Epigallocatechin | Low | 0.4 times or less | 0.3 times or less | 0.2 times or less |
| 93 | 3-(2-thienyl)-1,2,4-oxadiazole-5-carbohydrazide | High | 1.7 times or more | 2.5 times or more | 4 times or more |
| 94 | DL-Lactic Acid | High | 1.2 times or more | 1.4 times or more | 2 times or more |
| 95 | 6-Hydroxypicolinic acid | Low | 0.9 times or less | 0.8 times or less | 0.6 times or less |
| 96 | Proline | Low | 0.7 times or less | 0.5 times or less | 0.3 times or less |
| 97 | 1,3,5-trihydroxy-4-{[(2E)-3-(3-hydroxy-4-methoxyphenyl)prop-2-enoyl]oxy}cyclohexane-1-carboxylic acid | Low | 0.9 times or less | 0.8 times or less | 0.6 times or less |
| 98 | Prolylglycine | High | 1.5 times or more | 1.8 times or more | 2 times or more |
| 99 | 4-[(6E)-3-Hydroxy-8,10-dimethyl-2-(methylamino)-6-dodecen-1-yl]phenol | Low | 0.8 times or less | 0.6 times or less | 0.4 times or less |

In another embodiment, for each substances selected from Group A or Group B, the median of measurement values for the substance in specimens obtained from a negative group (a population with non-reduced intestinal barrier function) consisting of individuals of the same species as the subject (e.g., humans if the subject is a human) can be used as a reference value for the substance. Hereinafter, this embodiment (hereinafter, referred to as "Embodiment 1-2") will be described.

Unless otherwise specified, the description of Embodiment 1-1 is also applied to Embodiment 1-2. In the application, the term "average value" is replaced with the term "median".

In still another embodiment, for each substance selected from Group A or Group B, a cutoff value obtained from an ROC (Receiver Operating Characteristic) curve for the substance can be used as a reference value for the substance. Hereinafter, this embodiment (hereinafter, referred to as "Embodiment 1-3") will be described.

The ROC curve for each substance can be prepared according to a conventional method on the basis of the concentrations of the substance in specimens obtained from a negative group (a population with non-reduced intestinal barrier function) and a positive group (a population with reduced intestinal barrier function) each consisting of individuals of the same species as the subject (e.g., humans if the subject is a human). In the ROC curve, the ordinate represents sensitivity, and the abscissa represents (1 - specificity). In forming the negative group and positive group, the determination of negative and positive individuals can be performed by a known testing method for intestinal barrier function (e.g., lactulose/mannitol test).

In the case where the specimen used in step 1a is dry feces, the specimens obtained from the negative group and positive group are also dry feces.

In the case where the specimen used in step 1a is wet feces, the specimens obtained from the negative group and positive group are also wet feces.

In the case where the measurement value acquired in step 1a is the relative concentration of one substance selected from Group A or Group B, the concentrations of the one substance to be used for preparation of the ROC curve for the one substance are also relative concentrations. The latter term "relative concentration" is synonymous with the former term "relative concentration", and the latter relative concentrations are determined in the same manner as for the former relative concentrations. In the case where the former relative concentration is a relative area value CE, the latter relative concentrations are also relative area values CE. In the case where the former relative concentration is a relative area value LC, the latter relative concentrations are also relative area values LC. In the case where the concentrations to be used for preparation of the ROC curve for the one substance are relative concentrations, the cutoff value to be obtained from the ROC curve for the one substance is also a relative concentration.

In the case where the measurement value acquired in step 1a is the absolute concentration of one substance selected from Group A or Group B, the concentrations of the one substance to be used for preparation of the ROC curve for the one substance are also absolute concentrations. The latter absolute concentrations are determined in the same manner as for the former absolute concentration. In the case where the concentrations to be used for preparation of the ROC curve for the one substance are absolute concentrations, the cutoff value to be obtained from the ROC curve for the one substance is also an absolute concentration.

In the case where the measurement value acquired in step 1a is the relative concentrations of two or more substances selected from Group A or Group B, the concentrations of each substance to be used for preparation of the ROC curve for the substance are also relative concentrations. The latter term "relative concentration" is synonymous with the former term "relative concentration", and the latter relative concentrations are determined in the same manner as for the former relative concentrations. In the case where the former relative concentrations are relative area values CE, the latter relative concentrations are also relative area values CE. In the case where the former relative concentrations are relative area values LC, the latter relative concentrations are also relative area values LC. In the case where the concentrations of each substance to be used for preparation of the ROC curve for the substance are relative concentrations, the cutoff value to be obtained from the ROC curve for the substance is also a relative concentration.

In the case where the measurement value acquired in step 1a is the absolute concentrations of two or more substances selected from Group A or Group B, the concentrations of each substance to be used for preparation of the ROC curve for the substance are also absolute concentrations. The latter absolute concentrations are determined in the same manner as for the former absolute concentrations. In the case where the concentrations of each substance to be used for preparation of the ROC curve are absolute concentrations, the cutoff value to be obtained from the ROC curve for the substance is also an absolute concentration.

A cutoff value is such a value that both high sensitivity and high specificity can be achieved when evaluation is carried out with reference to the value. A cutoff value can be appropriately set in consideration of the balance between sensitivity and specificity. For example, a value at which a high positive rate is exhibited in the positive group and a high negative rate is exhibited in the negative group can be set as a cutoff value. A method for setting a cutoff value is well known to those skilled in the art. Known examples therefor include a method in which a point on an ROC curve at which the distance from the upper left corner of the ROC curve is minimized is used as a cutoff value, and a method in which a point on an ROC curve at which the value of (sensitivity + specificity - 1) is maximized is used as a cutoff value. The point on an ROC curve at which the value of (sensitivity + specificity - 1) is maximized is called "Younden index".

Specific examples of cutoff values for substances selected from Group A are shown in Table 18, and specific examples of cutoff values for substances selected from Group B are shown in Table 16.

The cutoff value for each substance selected from Group A is preferably a value within the range of a value shown as P1 in Table A or more and a value shown as P3 in Table A or less. The cutoff value for each substance selected from Group A may be a value within the range of a value shown as P1 in Table A or more and a value shown as P2 in Table A or less, or a value within the range of a value shown as P2 in Table A or more and a value shown as P3 in Table A or less. The cutoff value for each substance selected from Group A may be, for example, a value shown as P1 in Table A, or a value shown as P2 in Table A, or a value shown as P3 in Table A.

The cutoff value for each substance selected from Group B is preferably a value within the range of a value shown as Q1 in Table B or more and a value shown as Q3 in Table B or less. The cutoff value for each substance selected from Group B may be a value within the range of a value shown as Q1 in Table B or more and a value shown as Q2 in Table B or less, or a value within the range of a value shown as Q2 in Table B or more and a value shown as Q3 in Table B or less. The cutoff value for each substance selected from Group B may be, for example, a value shown as Q1 in Table B, or a value shown as Q2 in Table B, or a value shown as Q3 in Table B.

[Table A-1]

| Table A (Group A) | | | | |
|---|---|---|---|---|
| No. | Substance Name | P1 | P2 | P3 |
| 1 | Formylanthranilic acid | 4.89E-05 | 6.99E-05 | 9.08E-05 |
| 2 | Diethanolamine | 6.80E-04 | 9.72E-04 | 1.26E-03 |
| 3 | Glycerol | 1.15E-01 | 1.65E-01 | 2.14E-01 |

(continued)

| Table A (Group A) | | | | |
| No. | Substance Name | P1 | P2 | P3 |
| 4 | 5,6-Dihydroxyindole | 3.28E-05 | 4.69E-05 | 6.09E-05 |
| 5 | Formiminoglutamic acid | 2.40E-04 | 3.42E-04 | 4.45E-04 |
| 6 | Leucine | 6.29E-01 | 8.99E-01 | 1.17E+00 |
| 7 | Myristoleic acid | 3.43E-05 | 4.91E-05 | 6.38E-05 |
| 8 | 3-Aminopropane-1,2-diol | 4.96E-04 | 7.08E-04 | 9.21E-04 |
| 9 | 4-Guanidinobutyric acid | 1.57E-03 | 2.25E-03 | 2.92E-03 |
| 10 | Phenlyalanine | 4.39E-01 | 6.27E-01 | 8.15E-01 |
| 11 | 2'-Deoxyadenosine and 5'-Deoxyadenosine | 2.49E-05 | 3.56E-05 | 4.63E-05 |
| 12 | Lipoamide | 5.16E-04 | 7.37E-04 | 9.58E-04 |
| 13 | 3-Guanidinopropionic acid | 2.88E-06 | 4.11E-06 | 5.34E-06 |
| 14 | Acetanilide | 6.14E-04 | 8.77E-04 | 1.14E-03 |
| 15 | Histidine | 1.26E-01 | 1.80E-01 | 2.34E-01 |
| 16 | $\gamma$-glutamyl-2-aminobutyric acid | 7.06E-05 | 1.01E-04 | 1.31E-04 |
| 17 | Methionine sulfoxide | 1.77E-02 | 2.53E-02 | 3.29E-02 |
| 18 | Phenylhydrazine | 3.68E-05 | 5.25E-05 | 6.83E-05 |
| 19 | Tryptophan | 6.46E-02 | 9.22E-02 | 1.20E-01 |
| 20 | Tyrosine | 2.88E-01 | 4.12E-01 | 5.35E-01 |
| 21 | Hydroxyindole | 2.32E-03 | 3.31E-03 | 4.30E-03 |
| 22 | Alanine | 1.73E-01 | 2.48E-01 | 3.22E-01 |
| 23 | p-Aminobenzoic acid | 5.49E-04 | 7.84E-04 | 1.02E-03 |

[Table A-2]

| Table A (Continued) | | | | |
| No. | Substance Name | P1 | P2 | P3 |
| 24 | Penicillamine | 1.59E-04 | 2.27E-04 | 2.95E-04 |
| 25 | 2-Cyanopyridine | 9.05E-04 | 1.29E-03 | 1.68E-03 |
| 26 | Glutamine | 1.05E-01 | 1.50E-01 | 1.95E-01 |
| 27 | 2,4-Diaminobutyric acid | 1.42E-04 | 2.03E-04 | 2.64E-04 |
| 28 | Trigonelline | 3.03E-03 | 4.33E-03 | 5.63E-03 |
| 29 | 2-Methylserine | 4.99E-04 | 7.13E-04 | 9.27E-04 |
| 30 | Glycylglycine | 1.94E-03 | 2.77E-03 | 3.60E-03 |
| 31 | N,N-Dimethylhistidine | 4.81E-04 | 6.87E-04 | 8.93E-04 |
| 32 | Valine | 2.89E-01 | 4.14E-01 | 5.38E-01 |
| 33 | N-Acetylphenylalanine | 8.97E-05 | 1.28E-04 | 1.67E-04 |
| 34 | Isoleucine | 3.60E-01 | 5.15E-01 | 6.69E-01 |
| 35 | Methionine | 1.85E-01 | 2.64E-01 | 3.43E-01 |
| 36 | $\gamma$-glutamyl-valyl-glycine | 1.83E-04 | 2.61E-04 | 3.39E-04 |
| 37 | N-Acetylaspartic acid | 2.89E-03 | 4.13E-03 | 5.37E-03 |

(continued)

| Table A (Continued) | | | | |
|---|---|---|---|---|
| No. | Substance Name | P1 | P2 | P3 |
| 38 | Threonic acid | 2.26E-04 | 3.22E-04 | 4.19E-04 |
| 39 | Alloisoleucine | 3.12E-03 | 4.46E-03 | 5.80E-03 |
| 40 | Arginine ethyl ester | 7.96E-06 | 1.14E-05 | 1.48E-05 |
| 41 | 2,6-Diaminopimelic acid | 1.76E-03 | 2.52E-03 | 3.27E-03 |
| 42 | Glucose 6-phosphate | 1.27E-03 | 1.82E-03 | 2.36E-03 |
| 43 | Adenosine | 4.58E-04 | 6.55E-04 | 8.51E-04 |
| 44 | Mucic acid | 1.81E-04 | 2.59E-04 | 3.37E-04 |
| 45 | Thymidine | 8.23E-03 | 1.18E-02 | 1.53E-02 |
| 46 | Glycocholic acid | 1.01E-05 | 1.45E-05 | 1.88E-05 |

[Table A-3]

| Table A (Continued) | | | | |
|---|---|---|---|---|
| No. | Substance Name | P1 | P2 | P3 |
| 47 | 2-Deoxyribose 1-phosphate | 1.78E-05 | 2.55E-05 | 3.31E-05 |
| 48 | Isoleucyl-prolyl-proline | 6.15E-04 | 8.79E-04 | 1.14E-03 |
| 49 | Sedoheptulose 7-phosphate | 6.41E-05 | 9.16E-05 | 1.19E-04 |
| 50 | Fumaric acid | 2.49E-03 | 3.56E-03 | 4.62E-03 |
| 51 | N-Acetylmethionine | 2.29E-04 | 3.28E-04 | 4.26E-04 |
| 52 | Fructose 6-phosphate | 3.71E-05 | 5.31E-05 | 6.90E-05 |
| 53 | Glutamylglutamic acid | 5.31E-03 | 7.59E-03 | 9.86E-03 |
| 54 | Cysteine | 6.48E-06 | 9.26E-06 | 1.20E-05 |
| 55 | N-Acetylglucosamine 1-phosphate | 1.53E-05 | 2.19E-05 | 2.85E-05 |
| 56 | Ribose 5-phosphate | 2.05E-05 | 2.93E-05 | 3.80E-05 |
| 57 | Glucose 1-phosphate | 1.64E-04 | 2.34E-04 | 3.04E-04 |
| 58 | 3'-Cytidylic acid and 2'-Cytidylic acid | 2.05E-05 | 2.93E-05 | 3.81E-05 |
| 59 | Dihydroxyacetone phosphate | 9.45E-05 | 1.35E-04 | 1.76E-04 |
| 60 | 4-Oxovaleric acid | 7.00E-08 | 1.00E-07 | 1.30E-07 |
| 61 | 2-Deoxyglucose 6-phosphate | 2.19E-05 | 3.12E-05 | 4.06E-05 |
| 62 | Glucosamine 6-phosphate | 2.60E-05 | 3.71E-05 | 4.82E-05 |
| 63 | 3-Methyladipic acid | 7.73E+07 | 1.10E+08 | 1.44E+08 |
| 64 | 2-Hydroxyquinoline | 6.68E+06 | 9.54E+06 | 1.24E+07 |
| 65 | 7-(tert-butyl)-4-imino-1,2,3,4,5,6-hexahydropyrido[3,4-d]pyridazine-1,5-dione) | 2.13E+08 | 3.05E+08 | 3.96E+08 |
| 66 | 2,4-dihydroxyheptadec-16-en-1-yl acetate | 3.61E+06 | 5.16E+06 | 6.70E+06 |
| 67 | α-Aspartylphenylalanine | 5.16E+06 | 7.37E+06 | 9.58E+06 |
| 68 | N1-[4-(1,3-Oxazol-5-yl)phenyl]cyclopropane-1-carboxamide | 4.57E+07 | 6.52E+07 | 8.48E+07 |

[Table A-4]

| Table A (Continued) | | | | |
|---|---|---|---|---|
| No. | Substance Name | P1 | P2 | P3 |
| 69 | 1-(2,4-Dihydroxyquinolin-3-yl)ethan-1-one | 1.68E+06 | 2.40E+06 | 3.12E+06 |
| 70 | 4-(Diethylamino)salicylaldehyde | 1.82E+07 | 2.60E+07 | 3.38E+07 |
| 71 | Atenolol | 4.76E+06 | 6.80E+06 | 8.83E+06 |
| 72 | 5-Hydroxyindole | 2.98E+07 | 4.26E+07 | 5.54E+07 |
| 73 | 6-Methylquinoline | 3.07E+07 | 4.39E+07 | 5.71E+07 |
| 74 | Guanine | 1.07E+08 | 1.53E+08 | 1.98E+08 |
| 75 | Carboxyibuprofen | 3.64E+05 | 5.21E+05 | 6.77E+05 |
| 76 | 3-Isopropylmalic acid | 1.54E+07 | 2.21E+07 | 2.87E+07 |
| 77 | Bis(methylbenzylidene)sorbitol | 1.77E+09 | 2.53E+09 | 3.29E+09 |
| 78 | 2-Furoic acid | 1.18E+06 | 1.69E+06 | 2.20E+06 |
| 79 | Orotic acid | 2.65E+07 | 3.78E+07 | 4.92E+07 |
| 80 | 2-Acetamido-N-(3-aspartyl-2-deoxyhexopyranosylamine | 7.59E+06 | 1.08E+07 | 1.41E+07 |
| 81 | Xanthosine | 2.72E+05 | 3.88E+05 | 5.05E+05 |
| 82 | 1-propyl-1H-benzo[d]imidazole hydrobromide | 3.07E+06 | 4.38E+06 | 5.70E+06 |
| 83 | 4-Phenolsulfonic acid | 4.29E+06 | 6.12E+06 | 7.96E+06 |
| 84 | Xanthohumol | 2.73E+06 | 3.91E+06 | 5.08E+06 |
| 85 | Laurolactam | 1.65E+08 | 2.35E+08 | 3.06E+08 |
| 86 | Adenine | 2.06E+07 | 2.94E+07 | 3.82E+07 |
| 87 | 2'-Deoxyguanosine | 6.14E+07 | 8.78E+07 | 1.14E+08 |
| 88 | 6-Hydroxypicolinic acid | 3.31E+06 | 4.73E+06 | 6.15E+06 |
| 89 | D-(-)-Quinic acid | 1.19E+07 | 1.70E+07 | 2.20E+07 |
| 90 | 4-{[(4,6-Dimethoxypyrimidin-2-yl)amino]methylidene}-2-phenyl-4,5-dihydro-1,3-oxazol-5-one | 4.49E+05 | 6.41E+05 | 8.33E+05 |

[Table A-5]

| Table A (Continued) | | | | |
|---|---|---|---|---|
| No. | Substance Name | P1 | P2 | P3 |
| 91 | 4-[(6E)-3-Hydroxy-8,10-dimethyl-2-(methylamino)-6-dodecen-1-yl]phenol | 5.70E+05 | 8.14E+05 | 1.06E+06 |
| 92 | Ethyl 2-cyano-3-(tetrahydro-3-thiophenylamino)acrylate | 5.30E+08 | 7.57E+08 | 9.85E+08 |
| 93 | 3-Succinoylpyridine | 3.26E+06 | 4.65E+06 | 6.05E+06 |
| 94 | Proline | 9.19E+07 | 1.31E+08 | 1.71E+08 |
| 95 | L-threo-3-Phenylserine | 9.56E+07 | 1.37E+08 | 1.77E+08 |
| 96 | (2,4-Diamino-5-pyrimidinyl)(3,4,5-trimethoxyphenyl)methanone | 1.17E+06 | 1.68E+06 | 2.18E+06 |
| 97 | 3-Morpholino-4-tetrahydro-1H-pyrrol-1-ylcyclobut-3-ene-1,2-dione | 1.34E+07 | 1.92E+07 | 2.49E+07 |
| 98 | 16-Hydroxyhexadecanoic acid | 2.10E+09 | 3.00E+09 | 3.90E+09 |
| 99 | Syringic acid | 5.66E+06 | 8.08E+06 | 1.05E+07 |
| 100 | Tranexamic acid | 4.71E+06 | 6.72E+06 | 8.74E+06 |

(continued)

| Table A (Continued) | | | | |
|---|---|---|---|---|
| No. | Substance Name | P1 | P2 | P3 |
| 101 | 4-(tert-butyl)phenyl 3,5-dimethylisoxazole-4-carboxylate | 1.18E+08 | 1.69E+08 | 2.19E+08 |
| 102 | 3-Methylhistidine | 9.88E+07 | 1.41E+08 | 1.83E+08 |
| 103 | 5-Methoxyindole | 7.54E+05 | 1.08E+06 | 1.40E+06 |
| 104 | Perillic acid | 6.99E+05 | 9.98E+05 | 1.30E+06 |
| 105 | 6-Methyl[1,2,4]triazolo[4,3-b]pyridazin-8-ol | 8.04E+07 | 1.15E+08 | 1.49E+08 |
| 106 | Piperonylonitrile | 2.33E+06 | 3.34E+06 | 4.34E+06 |
| 107 | N1-(5-Chloro-2-hydroxyphenyl)-2-(morpholinomethyl)benzamide | 1.67E+05 | 2.39E+05 | 3.11E+05 |
| 108 | N-Methylhistamine | 1.18E+06 | 1.68E+06 | 2.18E+06 |
| 109 | Dibutylone | 1.10E+07 | 1.57E+07 | 2.04E+07 |
| 110 | (-)-Epigallocatechin | 1.35E+06 | 1.93E+06 | 2.51E+06 |
| 111 | Myriocin | 3.23E+05 | 4.62E+05 | 6.00E+05 |
| 112 | 4-Indolecarbaldehyde | 1.14E+08 | 1.63E+08 | 2.12E+08 |

[Table A-6]

| Table A (Continued) | | | | |
|---|---|---|---|---|
| No. | Substance Name | P1 | P2 | P3 |
| 113 | Uracil | 4.47E+07 | 6.38E+07 | 8.30E+07 |
| 114 | Dihydrothymine | 1.90E+06 | 2.72E+06 | 3.53E+06 |
| 115 | 3-Methylglutaric acid | 3.06E+08 | 4.37E+08 | 5.67E+08 |
| 116 | 1,5-Anhydro-D-glucitol | 4.38E+07 | 6.25E+07 | 8.13E+07 |
| 117 | Valethamate | 3.58E+05 | 5.12E+05 | 6.65E+05 |
| 118 | DL-Arginine | 1.06E+08 | 1.51E+08 | 1.97E+08 |
| 119 | (R)-Equol | 5.54E+05 | 7.92E+05 | 1.03E+06 |
| 120 | Theobromine | 3.64E+07 | 5.20E+07 | 6.76E+07 |
| 121 | 1,5-Isoquinolinediol | 2.40E+06 | 3.42E+06 | 4.45E+06 |
| 122 | 12-Hydroxydodecanoic acid | 5.56E+07 | 7.95E+07 | 1.03E+08 |
| 123 | 1,3,5-trihydroxy-4-{[(2E)-3-(3-hydroxy-4-methoxyphenyl)prop-2-enoyl]oxy}cyclohexane-1-carboxylic acid | 5.93E+06 | 8.47E+06 | 1.10E+07 |
| 124 | 3-Hydroxypicolinic acid | 1.75E+07 | 2.49E+07 | 3.24E+07 |
| 125 | Theophylline | 7.50E+07 | 1.07E+08 | 1.39E+08 |
| 126 | 3,4-Dihydroxybenzaldehyde | 1.43E+06 | 2.04E+06 | 2.66E+06 |
| 127 | Acetylcholine | 8.29E+08 | 1.18E+09 | 1.54E+09 |
| 128 | Tridecylic acid | 3.19E+08 | 4.56E+08 | 5.93E+08 |

[Table B-1]

| Table B (Group B) | | | | |
|---|---|---|---|---|
| No. | Substance Name | Q1 | Q2 | Q3 |
| 1 | Formylanthranilic acid | 1.21E-05 | 1.74E-05 | 2.26E-05 |
| 2 | Glycerol | 2.82E-02 | 4.03E-02 | 5.24E-02 |

(continued)

| Table B (Group B) | | | | |
|---|---|---|---|---|
| No. | Substance Name | Q1 | Q2 | Q3 |
| 3 | Formiminoglutamic acid | 5.79E-05 | 8.27E-05 | 1.08E-04 |
| 4 | 5,6-Dihydroxyindole | 1.17E-05 | 1.67E-05 | 2.17E-05 |
| 5 | 3-Aminopropane-1,2-diol | 7.00E-08 | 1.00E-07 | 1.30E-07 |
| 6 | 4-Guanidinobutyric acid | 3.51E-04 | 5.01E-04 | 6.51E-04 |
| 7 | 2'-Deoxyadenosine and 5'-Deoxyadenosine | 5.35E-06 | 7.65E-06 | 9.94E-06 |
| 8 | Acetanilide | 1.31E-04 | 1.88E-04 | 2.44E-04 |
| 9 | Pyrophosphate | 2.64E-03 | 3.78E-03 | 4.91E-03 |
| 10 | Myristoleic acid | 1.84E-05 | 2.63E-05 | 3.42E-05 |
| 11 | Diethanolamine | 1.69E-04 | 2.41E-04 | 3.14E-04 |
| 12 | Thiaproline | 4.30E-05 | 6.14E-05 | 7.98E-05 |
| 13 | Hydroxyindole | 3.25E-04 | 4.65E-04 | 6.04E-04 |
| 14 | 2,6-Diaminopimelic acid | 3.99E-04 | 5.71E-04 | 7.42E-04 |
| 15 | p-Aminobenzoic acid | 1.57E-04 | 2.25E-04 | 2.92E-04 |
| 16 | Lipoamide | 1.29E-04 | 1.84E-04 | 2.39E-04 |
| 17 | Phenylhydrazine | 5.98E-06 | 8.54E-06 | 1.11E-05 |
| 18 | 3-Guanidinopropionic acid | 7.90E-07 | 1. 13E-06 | 1.47E-06 |
| 19 | N-Acetylaspartic acid | 5.76E-04 | 8.23E-04 | 1.07E-03 |
| 20 | 2,4-Diaminobutyric acid | 2.67E-05 | 3.82E-05 | 4.96E-05 |
| 21 | γ-glutamyl-2-aminobutyric acid | 1.41E-05 | 2.02E-05 | 2.62E-05 |
| 22 | Cysteine | 2.71E-06 | 3.87E-06 | 5.04E-06 |
| 23 | Glycerol 3-phosphate | 2.89E-05 | 4.13E-05 | 5.37E-05 |
| 24 | Glucose 6-phosphate | 1.79E-04 | 2.55E-04 | 3.32E-04 |

[Table B-2]

| Table B (Continued) | | | | |
|---|---|---|---|---|
| No. | Substance Name | Q1 | Q2 | Q3 |
| 25 | Mucic acid | 4.09E-05 | 5.85E-05 | 7.60E-05 |
| 26 | Trigonelline | 5.45E-04 | 7.79E-04 | 1.01E-03 |
| 27 | N-Carbamoylaspartic acid | 5.56E-05 | 7.95E-05 | 1.03E-04 |
| 28 | Threonic acid | 6.43E-05 | 9.19E-05 | 1.19E-04 |
| 29 | Glycocholic acid | 2.78E-06 | 3.98E-06 | 5.17E-06 |
| 30 | Isobutyric acid and Butyric acid | 3.86E-02 | 5.52E-02 | 7.17E-02 |
| 31 | 2-Deoxyribose 1-phosphate | 5.41E-06 | 7.73E-06 | 1.00E-05 |
| 32 | Arginine ethyl ester | 1.46E-06 | 2.09E-06 | 2.71E-06 |
| 33 | N-Methylglutamic acid | 2.03E-04 | 2.91E-04 | 3.78E-04 |
| 34 | Pterin | 5.72E-05 | 8.17E-05 | 1.06E-04 |
| 35 | N-Acetylphenylalanine | 2.54E-05 | 3.63E-05 | 4.72E-05 |
| 36 | Ribulose 5-phosphate | 1.61E-05 | 2.30E-05 | 2.99E-05 |

(continued)

| Table B (Continued) | | | | |
|---|---|---|---|---|
| No. | Substance Name | Q1 | Q2 | Q3 |
| 37 | Fructose 6-phosphate | 9.44E-06 | 1.35E-05 | 1.75E-05 |
| 38 | Sedoheptulose 7-phosphate | 2.59E-05 | 3.70E-05 | 4.81E-05 |
| 39 | N-Acetylglucosamine 1-phosphate | 4.21E-06 | 6.01E-06 | 7.81E-06 |
| 40 | Ribose 5-phosphate | 8.48E-06 | 1.21E-05 | 1.58E-05 |
| 41 | 3'-Cytidylic acid and 2'-Cytidylic acid | 9.70E-06 | 1.39E-05 | 1.80E-05 |
| 42 | Dihydroxyacetone phosphate | 1.92E-05 | 2.75E-05 | 3.57E-05 |
| 43 | 4-Oxovaleric acid | 7.00E-08 | 1.00E-07 | 1.30E-07 |
| 44 | Propranolol | 1.26E-06 | 1.80E-06 | 2.34E-06 |
| 45 | 2-Deoxyglucose 6-phosphate | 8.76E-06 | 1.25E-05 | 1.63E-05 |
| 46 | 6-Methylquinoline | 7.08E+06 | 1.01E+07 | 1.31E+07 |
| 47 | N1-[4-(1,3-Oxazol-5-yl)phenyl]cyclopropane-1-carboxamide | 1.03E+07 | 1.47E+07 | 1.91E+07 |

[Table B-3]

| Table B (Continued) | | | | |
|---|---|---|---|---|
| No. | Substance Name | Q1 | Q2 | Q3 |
| 48 | 2-Hydroxyquinoline | 1.45E+06 | 2.08E+06 | 2.70E+06 |
| 49 | 3-Methyladipic acid | 1.95E+07 | 2.79E+07 | 3.63E+07 |
| 50 | L-threo-3-Phenylserine | 1.84E+07 | 2.63E+07 | 3.42E+07 |
| 51 | 4-(Diethylamino)salicylaldehyde | 4.58E+06 | 6.54E+06 | 8.51E+06 |
| 52 | 5-Methoxyindole | 2.07E+05 | 2.96E+05 | 3.84E+05 |
| 53 | 7-(tert-butyl)-4-imino-1,2,3,4,5,6-hexahydropyrido[3,4-d] pyridazine-1,5-dione | 4.29E+07 | 6.14E+07 | 7.98E+07 |
| 54 | 4-Indolecarbaldehyde | 2.28E+07 | 3.26E+07 | 4.23E+07 |
| 55 | Carboxyibuprofen | 8.67E+04 | 1.24E+05 | 1.61E+05 |
| 56 | Piperonylonitrile | 5.89E+05 | 8.41E+05 | 1.09E+06 |
| 57 | 5-Hydroxyindole | 6.76E+06 | 9.66E+06 | 1.26E+07 |
| 58 | Orotic acid | 8.88E+06 | 1.27E+07 | 1.65E+07 |
| 59 | Glucose 1-phosphate | 4.75E+06 | 6.79E+06 | 8.83E+06 |
| 60 | L-Alanyl-L-proline | 1.78E+07 | 2.54E+07 | 3.31E+07 |
| 61 | 2-Acetamido-N-β-aspartyl-2-deoxyhexopyranosylamine | 1.98E+06 | 2.82E+06 | 3.67E+06 |
| 62 | α-Aspartylphenylalanine | 1.13E+06 | 1.61E+06 | 2.10E+06 |
| 63 | 6-Hydroxymelatonin | 8.93E+04 | 1.28E+05 | 1.66E+05 |
| 64 | Xanthosine | 8.09E+04 | 1.16E+05 | 1.50E+05 |
| 65 | Atenolol | 9.96E+05 | 1.42E+06 | 1.85E+06 |
| 66 | (R)-Equol | 1.45E+05 | 2.07E+05 | 2.69E+05 |
| 67 | 2,4-dihydroxyheptadec-16-en-1-yl acetate | 1.04E+06 | 1.49E+06 | 1.93E+06 |
| 68 | Guanine | 1.62E+07 | 2.31E+07 | 3.01E+07 |
| 69 | 1-(4-methyl-2-pyridyl)pyrrolidine-2,5-dione | 9.42E+05 | 1.35E+06 | 1.75E+06 |

(continued)

| Table B (Continued) | | | | |
|---|---|---|---|---|
| No. | Substance Name | Q1 | Q2 | Q3 |
| 70 | Laurolactam | 3.89E+07 | 5.55E+07 | 7.22E+07 |

[Table B-4]

| Table B (Continued) | | | | |
|---|---|---|---|---|
| No. | Substance Name | Q1 | Q2 | Q3 |
| 71 | Olmesartan | 3.76E+05 | 5.38E+05 | 6.99E+05 |
| 72 | Adenine | 6.20E+06 | 8.85E+06 | 1.15E+07 |
| 73 | Uracil | 1.04E+07 | 1.48E+07 | 1.92E+07 |
| 74 | 6-Methyl[1,2,4]triazolo[4,3-b]pyridazin-8-ol | 2.05E+07 | 2.93E+07 | 3.81E+07 |
| 75 | 1-propyl-1H-benzo[d]imidazole hydrobromide | 6.34E+05 | 9.06E+05 | 1.18E+06 |
| 76 | Pseudouridine | 4.75E+06 | 6.79E+06 | 8.83E+06 |
| 77 | D-(-)-Quinic acid | 7.99E+06 | 1.14E+07 | 1.48E+07 |
| 78 | Acetylcholine | 2.11E+08 | 3.01E+08 | 3.92E+08 |
| 79 | 3-Isopropylmalic acid | 2.83E+06 | 4.04E+06 | 5.26E+06 |
| 80 | 2-methoxy-5-(1H-1,2,4-triazol-5-yl)-4-(trifluoromethyl)pyridine | 7.41E+05 | 1.06E+06 | 1.38E+06 |
| 81 | 3-Amino-2-naphthoic acid | 6.22E+06 | 8.88E+06 | 1.15E+07 |
| 82 | Ornithine | 1.52E+07 | 2.18E+07 | 2.83E+07 |
| 83 | 2'-Deoxyguanosine | 1.18E+07 | 1.69E+07 | 2.20E+07 |
| 84 | Bis(methylbenzylidene)sorbitol | 4.59E+08 | 6.56E+08 | 8.53E+08 |
| 85 | 2-Furoic acid | 3.43E+05 | 4.90E+05 | 6.36E+05 |
| 86 | 4-(tert-butyl)phenyl 3,5-dimethylisoxazole-4-carboxylate | 4.16E+07 | 5.94E+07 | 7.72E+07 |
| 87 | Pyridoxamine | 2.17E+06 | 3.10E+06 | 4.04E+06 |
| 88 | Tridecylic acid | 8.99E+07 | 1.28E+08 | 1.67E+08 |
| 89 | 3-Methylhistidine | 1.22E+07 | 1.74E+07 | 2.26E+07 |
| 90 | Hexanoic acid | 1.33E+07 | 1.90E+07 | 2.47E+07 |
| 91 | Azobenzene | 2.07E+07 | 2.96E+07 | 3.84E+07 |
| 92 | (-)-Epigallocatechin | 2.77E+05 | 3.96E+05 | 5.15E+05 |
| 93 | 3-(2-thienyl)-1,2,4-oxadiazole-5-carbohydrazide | 2.53E+05 | 3.61E+05 | 4.69E+05 |

[Table B-5]

| Table B (Continued) | | | | |
|---|---|---|---|---|
| No. | Substance Name | Q1 | Q2 | Q3 |
| 94 | DL-Lactic Acid | 4.54E+06 | 6.48E+06 | 8.42E+06 |
| 95 | 6-Hydroxypicolinic acid | 8.23E+05 | 1.18E+06 | 1.53E+06 |
| 96 | Proline | 2.01E+07 | 2.87E+07 | 3.74E+07 |
| 97 | 1,3,5-trihydroxy-4-{[(2E)-3-(3-hydroxy-4-methoxyphenyl)prop-2-enoyl]oxy}cyclohexane-1-carboxylic acid | 1.25E+06 | 1.79E+06 | 2.33E+06 |
| 98 | Prolylglycine | 6.72E+06 | 9.60E+06 | 1.25E+07 |

(continued)

| Table B (Continued) | | | | |
|---|---|---|---|---|
| No. | Substance Name | Q1 | Q2 | Q3 |
| 99 | 4-[(6E)-3-Hydroxy-8,10-dimethyl-2-(methylamino)-6-dodecen-1-yl]phenol | 1.34E+05 | 1.92E+05 | 2.49E+05 |

In still another embodiment, for two or more substances selected from Group A or Group B, a cutoff value for the two or more substances can be used as a reference value for the two or more substances, wherein the cutoff value is obtained in such a manner that the concentrations of the two or more substances are measured for specimens obtained from a negative group (a population with non-reduced intestinal barrier function) and a positive group (a population with reduced intestinal barrier function) each consisting of individuals of the same species as the subject (e.g., humans if the subject is a human), a combined variable is determined from the concentrations of the two or more substances on the basis of the expression (1), an ROC curve is prepared on the basis of the combined variable determined, and the cutoff value is determined from the ROC curve prepared. Hereinafter, this embodiment (hereinafter, referred to as "Embodiment 1-4") will be described.

Embodiment 1-4 is applied to the case where the measurement value acquired in step 1a is a combined variable determined from the concentrations of two or more substances selected from Group A or Group B on the basis of the expression (1).

In the case where the measurement value acquired in step 1a is a combined variable determined from the relative concentrations of two or more substances selected from Group A or Group B on the basis of the expression (1), the concentrations to be used for determination of a cutoff value are also relative concentrations. The latter term "relative concentration" is synonymous with the former term "relative concentration", and the latter relative concentrations are determined in the same manner as for the former relative concentrations. In the case where the former relative concentrations are relative area values CE, the latter relative concentrations are also relative area values CE. In the case where the former relative concentrations are relative area values LC, the latter relative concentrations are also relative area values LC.

In the case where the measurement value acquired in step 1a is a combined variable determined from the absolute concentrations of two or more substances selected from Group A or Group B on the basis of the expression (1), the concentrations to be used for determination of a cutoff value are also absolute concentrations. The latter term absolute concentration is synonymous with the former term "absolute concentration", and the latter absolute concentrations are determined in the same manner as for the former absolute concentrations.

In forming the negative group and positive group, the determination of negative and positive individuals can be performed by a known testing method for intestinal barrier function (e.g., lactulose/mannitol test). An ROC curve can be prepared according to a conventional method on the basis of a combined variable obtained from the negative group and positive group. In the ROC curve, the ordinate represents sensitivity, and the abscissa represents (1 - specificity). In forming the negative group and positive group, the determination of negative and positive individuals can be performed by a known testing method for intestinal barrier function (e.g., lactulose/mannitol test). For cutoff values, the description thereof given for Embodiment 1-3 is applied.

In the case where the specimen is dry feces, the biomarker to be measured in step 1a includes two or more substances selected from the substances of No. 1 to No. 4 in Group A (formylanthranilic acid, diethanolamine, glycerol, and 5,6-dihydroxyindole), and measurement of the relative area values (relative concentrations) of the two or more substances is performed by CE-FTMS under conditions described in the Examples, specific examples of the coefficients and constant term are as shown in Table 3, and specific examples of the cutoff values are as shown in Table 11. In Table 11, "CFD_A", "CFD_B", "CFD_C", and "CFD_D" are synonymous with those in Table 3.

[Table 11]

| Table 11 (Dry feces, No. 1 to No. 4 in Group A) | | | | | | |
|---|---|---|---|---|---|---|
| No. | Number of substances combined | First substance | Second substance | Third substance | Fourth substance | Cutoff value |
| 1 | 2 | CFD_A | CFD_B | - | - | -0.3002 |
| 2 | 2 | CFD_A | CFD_C | - | - | 0.0797 |
| 3 | 2 | CFD_A | CFD_D | - | - | 0.2544 |

(continued)

| Table 11 (Dry feces, No. 1 to No. 4 in Group A) | | | | | | |
|---|---|---|---|---|---|---|
| No. | Number of substances combined | First substance | Second substance | Third substance | Fourth substance | Cutoff value |
| 4 | 2 | CFD_B | CFD_C | - | - | -0.1633 |
| 5 | 2 | CFD_B | CFD_D | - | - | -0.0437 |
| 6 | 2 | CFD_C | CFD_D | - | - | -0.3603 |
| 7 | 3 | CFD_A | CFD_B | CFD_C | - | 0.1550 |
| 8 | 3 | CFD_A | CFD_B | CFD_D | - | 0.2333 |
| 9 | 3 | CFD_A | CFD_C | CFD_D | - | -0.0331 |
| 10 | 3 | CFD_B | CFD_C | CFD_D | - | 0.2835 |
| 11 | 4 | CFD_A | CFD_B | CFD_C | CFD_D | 0.1220 |

Each of the cutoff values of No. 1 to No. 11 in Table 11 is preferably a value within the range of a value shown as R1 in Table C or more and a value shown as R3 in Table C or less. Each of the cutoff values of No. 1 to No. 11 in Table 11 may be a value within the range of a value shown as R1 in Table C or more and a value shown as R2 in Table C or less, or a value within the range of a value shown as R2 in Table C or more and a value shown as R3 in Table C or less. Each of the cutoff values of No. 1 to No. 11 in Table 11 may be a value shown as R1 in Table C, or a value shown as R2 in Table C, or a value shown as R3 in Table C.

[Table C]

Table C

| No. | R1 | R2 | R3 |
|---|---|---|---|
| 1 | -0.3903 | -0.3002 | -0.2101 |
| 2 | 0.0558 | 0.0797 | 0.1036 |
| 3 | 0.1781 | 0.2544 | 0.3307 |
| 4 | -0.2123 | -0.1633 | -0.1143 |
| 5 | -0.0568 | -0.0437 | -0.0306 |
| 6 | -0.4684 | -0.3603 | -0.2522 |
| 7 | 0.1085 | 0.1550 | 0.2015 |
| 8 | 0.1633 | 0.2333 | 0.3033 |
| 9 | -0.0430 | -0.0331 | -0.0232 |
| 10 | 0.1985 | 0.2835 | 0.3686 |
| 11 | 0.0854 | 0.1220 | 0.1586 |

In the case where the specimen is dry feces, the biomarker to be measured in step 1a includes two or more substances selected from the substances of No. 63 to No. 66 in Group A (3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate), and measurement of the relative area values (relative concentrations) of the two or more substances is performed by LC-MS/MS under conditions described in the Examples, specific examples of the coefficients and constant term are as shown in Table 4, and specific examples of the cutoff values are as shown in Table 12. In Table 12, "LFD_A", "LFD_B", "LFD_C", and "LFD_D" are synonymous with those in Table 4.

[Table 12]

| No. | Number of substances combined | First substance | Second substance | Third substance | Fourth substance | Cutoff value |
|---|---|---|---|---|---|---|
| Table 12 (Dry feces, No. 63 to No. 66 in Group A) | | | | | | |
| 1 | 2 | LFD_A | LFD_B | - | - | -0.5638 |
| 2 | 2 | LFD_A | LFD_C | - | - | -0.2997 |
| 3 | 2 | LFD_A | LFD_D | - | - | -0.6363 |
| 4 | 2 | LFD_B | LFD_C | - | - | 0.1345 |
| 5 | 2 | LFD_B | LFD_D | - | - | -0.1973 |
| 6 | 2 | LFD_C | LFD_D | - | - | -0.0680 |
| 7 | 3 | LFD_A | LFD_B | LFD_C | - | -0.3814 |
| 8 | 3 | LFD_A | LFD_B | LFD_D | - | -0.7610 |
| 9 | 3 | LFD_A | LFD_C | LFD_D | - | -0.5532 |
| 10 | 3 | LFD_B | LFD_C | LFD_D | - | -0.1248 |
| 11 | 4 | LFD_A | LFD_B | LFD_C | LFD_D | -0.5374 |

Each of the cutoff values of No. 1 to No. 11 in Table 12 is preferably a value within the range of a value shown as S1 in Table D or more and a value shown as S3 in Table D or less. Each of the cutoff values of No. 1 to No. 11 in Table 12 may be a value within the range of a value shown as S1 in Table D or more and a value shown as S2 in Table D or less, or a value within the range of a value shown as S2 in Table D or more and a value shown as S3 in Table D or less. Each of the cutoff values of No. 1 to No. 11 in Table 12 may be a value shown as S1 in Table D, or a value shown as S2 in Table D, or a value shown as S3 in Table D.

[Table D]

Table D

| No. | S1 | S2 | S3 |
|---|---|---|---|
| 1 | -0.7329 | -0.5638 | -0.3947 |
| 2 | -0.3896 | -0.2997 | -0.2098 |
| 3 | -0.8272 | -0.6363 | -0.4454 |
| 4 | 0.0942 | 0.1345 | 0.1749 |
| 5 | -0.2565 | -0.1973 | -0.1381 |
| 6 | -0.0884 | -0.0680 | -0.0476 |
| 7 | -0.4958 | -0.3814 | -0.2670 |
| 8 | -0.9893 | -0.7610 | -0.5327 |
| 9 | -0.7192 | -0.5532 | -0.3872 |
| 10 | -0.1622 | -0.1248 | -0.0874 |
| 11 | -0.6986 | -0.5374 | -0.3762 |

In the case where the specimen is wet feces, the biomarker to be measured in step 1a includes two or more substances selected from the substances of No. 1 to No. 4 in Group B (formylanthranilic acid, glycerol, formiminoglutamic acid, and 5,6-dihydroxyindole), and measurement of the relative area values (relative concentrations) of the two or more substances is performed by CE-FTMS under conditions described in the Examples, specific examples of the coefficients and constant term are as shown in Table 5, and specific examples of the cutoff values are as shown in Table 13. In Table 13, "CFW_A", "CFW_B", "CFW_C", and "CFW_D" are synonymous with those in Table 5.

[Table 13]

| Table 13 (Wet feces, No. 1 to No. 4 in Group B) | | | | | | |
|---|---|---|---|---|---|---|
| No. | Number of substances combined | First substance | Second substance | Third substance | Fourth substance | Cutoff value |
| 1 | 2 | CFW_A | CFW_B | - | - | 0.0409 |
| 2 | 2 | CFW_A | CFW_C | - | - | 0.2945 |
| 3 | 2 | CFW_A | CFW_D | - | - | 0.6941 |
| 4 | 2 | CFW_B | CFW_C | - | - | 0.2897 |
| 5 | 2 | CFW_B | CFW_D | - | - | 0.2488 |
| 6 | 2 | CFW_C | CFW_D | - | - | 0.2742 |
| 7 | 3 | CFW_A | CFW_B | CFW_C | - | 0.2086 |
| 8 | 3 | CFW_A | CFW_B | CFW_D | - | 0.2846 |
| 9 | 3 | CFW_A | CFW_C | CFW_D | - | 0.2070 |
| 10 | 3 | CFW_B | CFW_C | CFW_D | - | 0.1500 |
| 11 | 4 | CFW_A | CFW_B | CFW_C | CFW_D | 0.0357 |

Each of the cutoff values of No. 1 to No. 11 in Table 13 is preferably a value within the range of a value shown as T1 in Table E or more and a value shown as T3 in Table E or less. Each of the cutoff values of No. 1 to No. 11 in Table 13 may be a value within the range of a value shown as T1 in Table E or more and a value shown as T2 in Table E or less, or a value within the range of a value shown as T2 in Table E or more and a value shown as T3 in Table E or less. Each of the cutoff values of No. 1 to No. 11 in Table 13 may be a value shown as T1 in Table E, or a value shown as T2 in Table E, or a value shown as T3 in Table E.

[Table E]

Table E

| No. | T1 | T2 | T3 |
|---|---|---|---|
| 1 | 0.0286 | 0.0409 | 0.0532 |
| 2 | 0.2062 | 0.2945 | 0.3829 |
| 3 | 0.4859 | 0.6941 | 0.9023 |
| 4 | 0.2028 | 0.2897 | 0.3766 |
| 5 | 0.1742 | 0.2488 | 0.3234 |
| 6 | 0.1919 | 0.2742 | 0.3565 |
| 7 | 0.1460 | 0.2086 | 0.2712 |
| 8 | 0.1992 | 0.2846 | 0.3700 |
| 9 | 0.1449 | 0.2070 | 0.2691 |
| 10 | 0.1050 | 0.1500 | 0.1950 |
| 11 | 0.0250 | 0.0357 | 0.0464 |

In the case where the specimen is wet feces, the biomarker to be measured in step 1a includes two or more substances selected from the substances of No. 46 to No. 49 in Group B (6-methylquinoline, N1-[4-(1,3-oxazol-5-yl) phenyl]cyclopropane-1-carboxamide, 2-hydroxyquinoline, and 3-methyladipic acid), and measurement of the relative area values (relative concentrations) of the two or more substances is performed by LC-MS/MS under conditions described in the Examples, specific examples of the coefficients and constant term are as shown in Table 6, and specific examples of the cutoff values are as shown in Table 14. In Table 14, "LFW_A", "LFW_B", "LFW_C", and "LFW_D" are synonymous with those in Table 6.

[Table 14]

| Table 14 ( Wet feces, No 46 to No. 49 in Group B) | | | | | | |
|---|---|---|---|---|---|---|
| No. | Number of substances combined | First substance | Second substance | Third substance | Fourth substance | Cutoff value |
| 1 | 2 | LFW_A | LFW_B | - | - | 0.2831 |
| 2 | 2 | LFW_A | LFW_C | - | - | 0.1288 |
| 3 | 2 | LFW_A | LFW_D | - | - | 0.4390 |
| 4 | 2 | LFW_B | LFW_C | - | - | 0.0309 |
| 5 | 2 | LFW_B | LFW_D | - | - | -0.6398 |
| 6 | 2 | LFW_C | LFW_D | - | - | -0.4317 |
| 7 | 3 | LFW_A | LFW_B | LFW_C | - | 0.1412 |
| 8 | 3 | LFW_A | LFW_B | LFW_D | - | -0.0486 |
| 9 | 3 | LFW_A | LFW_C | LFW_D | - | 0.4135 |
| 10 | 3 | LFW_B | LFW_C | LFW_D | - | -0.3123 |
| 11 | 4 | LFW_A | LFW_B | LFW_C | LFW_D | 0.1091 |

Each of the cutoff values of No. 1 to No. 11 in Table 14 is preferably a value within the range of a value shown as U1 in Table F or more and a value shown as U3 in Table F or less. Each of the cutoff values of No. 1 to No. 11 in Table 14 may be a value within the range of a value shown as U1 in Table F or more and a value shown as U2 in Table F or less, or a value within the range of a value shown as U2 in Table F or more and a value shown as U3 in Table F or less. Each of the cutoff values of No. 1 to No. 11 in Table 14 may be a value shown as U1 in Table F, or a value shown as U2 in Table F, or a value shown as U3 in Table F.

[Table F]

Table F

| No. | U1 | U2 | U3 |
|---|---|---|---|
| 1 | 0.1982 | 0.2831 | 0.3680 |
| 2 | 0.0902 | 0.1288 | 0.1674 |
| 3 | 0.3073 | 0.4390 | 0.5707 |
| 4 | 0.0216 | 0.0309 | 0.0402 |
| 5 | -0.8317 | -0.6398 | -0.4479 |
| 6 | -0.5612 | -0.4317 | -0.3022 |
| 7 | 0.0988 | 0.1412 | 0.1836 |
| 8 | -0.0632 | -0.0486 | -0.0340 |
| 9 | 0.2895 | 0.4135 | 0.5376 |
| 10 | -0.4060 | -0.3123 | -0.2186 |
| 11 | 0.0764 | 0.1091 | 0.1418 |

<<Aspect 2>>

Aspect 2 of the present invention relates to a method for diagnosing a disease or symptom caused by reduction in intestinal barrier function in a subject. Hereinafter, "a disease or symptom caused by reduction in intestinal barrier function" may be referred to as "the disease or symptom".

The method according to Aspect 2 of the present invention includes the steps of:

(2a) measuring a biomarker in a specimen derived from the subject to acquire a measurement value for the biomarker;

(2b) comparing the measurement value acquired in step 2a with a reference value to acquire a comparison result; and

(2c) diagnosing the disease or symptom caused by reduction in intestinal barrier function in the subject on the basis of the comparison result acquired in step 2b,

wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is also applied to Aspect 2. In the application, "step 1a" is replaced with "step 2a", "step 1b" is replaced with "step 2b", "step 1c" is replaced with "step 2c", the phrase "evaluating intestinal barrier function in the subject" is replaced with the phrase "diagnosing the disease or symptom in the subject", the phrase "accuracy of evaluating intestinal barrier function in the subject" is replaced with the phrase "accuracy of diagnosing the disease or symptom in the subject", the phrase "evaluating as being positive" is replaced with the phrase "diagnosing as being positive", and the phrase "evaluating as being negative" is replaced with the phrase "diagnosing as being negative".

In Aspect 2, the term "positive" means that the subject is affected by the disease or symptom, and the term "negative" means that the subject is not affected by the disease or symptom.

The disease or symptom to be diagnosed may be a disease or symptom caused by reduction in the intestinal barrier function of the small intestine, or a disease or symptom caused by reduction in the intestinal barrier function of the large intestine, but is preferably a disease or symptom caused by reduction in the intestinal barrier function of the small intestine.

Examples of the disease or symptom caused by reduction in intestinal barrier function include enteritis, allergic diseases, psychiatric diseases, non-alcoholic fatty liver diseases, type II diabetes mellitus, metabolic syndrome, and adiposity.

Examples of the enteritis include irritable bowel syndrome, ulcerative colitis, and Crohn's disease. Examples of the allergic diseases include food allergies, atopic dermatitis, and asthma. Examples of the psychiatric diseases include anxiety disorder and depression. Examples of the non-alcoholic fatty liver diseases include non-alcoholic fatty liver and non-alcoholic steatohepatitis.

Diagnosis is a practice typically performed by a physician. A physician diagnoses whether the subject is affected by the disease or symptom caused by reduction in intestinal barrier function on the basis of the comparison result acquired in step 2b; if the subject is affected by the disease or symptom caused by reduction in intestinal barrier function, the physician may determine whether prevention or treatment of the disease or symptom is needed for the subject, or select a method for preventing or treating the disease or symptom for the subject, or perform prevention or treatment of the disease or symptom for the subject. The term "prevention" includes prevention, suppression, and retardation of the onset of the disease or symptom. The term "treatment" includes suppression of the progress or exacerbation of the disease or symptom, retardation of the progress or exacerbation of the disease or symptom, and relief, mitigation, amelioration, and cure of the disease or symptom.

The method according to Aspect 2 of the present invention may be a method for assisting diagnosis of a disease or symptom caused by reduction in intestinal barrier function in a subject. The practice of assisting diagnosis of the disease or symptom is a practice of providing the comparison result acquired in step 2b as data useful for diagnosis of the disease or symptom, and this may be a medical practice or a non-medical practice, but is typically a non-medical practice. Diagnosis by a physician can be performed on the basis of the data provided through the practice of assisting diagnosis of the disease or symptom plus additional one or two or more pieces of information. Diagnosis by a physician can involve the experience, techniques, and the like of the physician.

< <Aspect 3> >

Aspect 3 of the present invention relates to a method for treating a disease or symptom caused by reduction in intestinal barrier function in a subject. Hereinafter, "a disease or symptom caused by reduction in intestinal barrier function" may be referred to as "the disease or symptom".

The method according to Aspect 3 of the present invention includes the steps of:

(3a) measuring a biomarker in a specimen derived from the subject to acquire a measurement value for the biomarker;

(3b) comparing the measurement value acquired in step 3a with a reference value to acquire a comparison result;

(3c) diagnosing the disease or symptom caused by reduction in intestinal barrier function in the subject on the basis of the comparison result acquired in step 3b; and

(3d) administering a substance or composition for treating the disease or symptom caused by reduction in intestinal barrier function to the subject if the subject is diagnosed to be affected by the disease or symptom caused by reduction in intestinal barrier function,

wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 2 is applied to Aspect 3. In the application, "step 2a" is replaced with "step 3a", "step 2b" is replaced with "step 3b", and "step 2c" is replaced with "step 3c".

For the substance or composition to be administered to the subject diagnosed to be affected by the disease or symptom caused by reduction in intestinal barrier function for treating the disease or symptom, appropriate one can be selected to fit with the type of the disease or symptom caused by reduction in intestinal barrier function by which the subject is affected. The term "treatment" includes suppression of the progression or exacerbation of a disease or symptom, retardation of the progression or exacerbation of the disease or symptom, and relief, mitigation, amelioration, and cure of the disease or symptom.

Substances or compositions for treating irritable bowel syndrome include high-molecular-weight polymers and serotonin receptor antagonists. Substances or compositions for treating ulcerative colitis or Crohn's disease include 5-aminosalicylate preparations and corticosteroids. Substances or compositions for treating allergic diseases include antihistamines. Substances or compositions for treating anxiety disorder or depression include selective serotonin reuptake inhibitors and noradrenergic antidepressants. Substances or compositions for treating non-alcoholic fatty liver diseases include antioxidants, therapeutics for diabetes mellitus, and therapeutics for dyslipidemia.

The substance or composition for treating the disease or symptom may be administered in the form of a food composition containing the substance or composition. Examples of the food composition include yogurt, probiotics, prebiotics, and supplements. The food composition may be, for example, a food for specified health uses, a food with functional claims, or a food for medical use.

Examples of the route of administration of the substance or composition for treating the disease or symptom include oral and parenteral (e.g., intranasal, ophthalmic, ear-drop, transdermal, tracheobronchial, intrarectal, urinary, subcutaneous, intramuscular, intravenous) administrations. The substance or composition for treating the disease or symptom may be formulated into a dosage form suitable for the route of administration. Examples of the dosage form include a tablet and an injection. In formulating, a proper excipient for oral administration (e.g., a diluent, a disintegrant, a lubricant, a binder) or an excipient suitable for parenteral administration (e.g., a diluting agent, a solvent) can be used. The dose and frequency of administration of the substance or composition for treating the disease or symptom can be appropriately adjusted in consideration of the dosage form and the age, body weight, and so on of the subject. The frequency of administration per day may be once, or two or more times. The period of administration may be 1 day or 2 days or more, (e.g., 1 week or more, 2 weeks or more, 3 weeks or more, or 4 weeks or more).

< <Aspect 4> >

Aspect 4 of the present invention relates to a method for evaluating a subject's risk of developing a disease or symptom caused by reduction in intestinal barrier function, or probability of the subject being affected by the disease or symptom in a subject. Hereinafter, "a subject's risk of developing a disease or symptom caused by reduction in intestinal barrier function" may be referred to as "the risk of the development", and "probability of the subject being affected by a disease or symptom caused by reduction in intestinal barrier function" may be referred to as "the probability of being affected".

The method according to Aspect 4 includes the steps of:

(4a) measuring a biomarker in a specimen derived from the subject to acquire a measurement value for the biomarker; and

(4b) comparing the measurement value acquired in step 4a with a reference value to acquire a comparison result; and

(4c) evaluating the risk of the development or the probability of being affected on the basis of the comparison result acquired in step 4b,

wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is applied to Aspect 4. In the application, "step 1a" is replaced with "step 4a", "step 1b" is replaced with "step 4b", "step 1c" is replaced with "step 4c", the phrase "evaluating intestinal barrier function in the subject" is replaced with the phrase "evaluating the risk of the development or the probability of being affected", and the phrase "accuracy of evaluating intestinal barrier function in the subject" is replaced with the phrase "accuracy of evaluating the risk of the development or the probability of being affected".

In Aspect 4, the term "positive" means that the risk of the development or the probability of being affected is present or high, and the term "negative" means that the risk of the development or the probability of being affected is absent or low.

The risk of the development to be evaluated may be a subject's risk of developing a disease or symptom caused by reduction in the intestinal barrier function of the small intestine, or a subject's risk of developing a disease or symptom caused by reduction in the intestinal barrier function of the large intestine, but is preferably a subject's risk of developing a disease or symptom caused by reduction in the intestinal barrier function of the small intestine.

The probability of being affected to be evaluated may be probability of the subject being affected by a disease or symptom caused by reduction in the intestinal barrier function of the small intestine, or probability of the subject being affected by a disease or symptom caused by reduction in the intestinal barrier function of the large intestine, but is preferably probability of the subject being affected by a disease or symptom caused by reduction in the intestinal barrier function of the small intestine.

For the disease or symptom caused by reduction in intestinal barrier function, the description thereof given for Aspect 2 is applied.

<<Aspect 5> >

Aspect 5 of the present invention relates to a method for acquiring data for diagnosing a disease or symptom caused by reduction in intestinal barrier function in a subject. Hereinafter, "a disease or symptom caused by reduction in intestinal barrier function" may be referred to as "the disease or symptom".

The method according to Aspect 5 includes the steps of:

(5a) measuring a biomarker in a specimen derived from the subject to acquire a measurement value for the biomarker; and

(5b) comparing the measurement value acquired in step 5a with a reference value to acquire a comparison result as the data for diagnosing the disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is applied to Aspect 5. In the application, "step 1a" is replaced with "step 5a", "step 1b" is replaced with "step 5b", the phrase "evaluating intestinal barrier function in the subject" is replaced with the phrase "acquiring data for diagnosing the disease or symptom in the subject", and the phrase "accuracy of evaluating intestinal barrier function in the subject" is replaced with the phrase "accuracy of diagnosing the disease or symptom in the subject".

In Aspect 5, the term "positive" means that the subject is affected by the disease or symptom, and the term "negative" means that the subject is not affected by the disease or symptom.

The disease or symptom to be evaluated may be a disease or symptom caused by reduction in the intestinal barrier function of the small intestine, or a disease or symptom caused by reduction in the intestinal barrier function of the large intestine, but is preferably a disease or symptom caused by reduction in the intestinal barrier function of the small intestine.

For the disease or symptom caused by reduction in intestinal barrier function, the description thereof given for Aspect 2 is applied.

For substances selected from Group A, a comparison result satisfying the criterion shown in Table 7 can be acquired as data for diagnosing as being positive, and a comparison result not satisfying the criterion shown in Table 7 can be acquired as data for diagnosing as being negative. Specifically, for a substance being "high" with respect to the criterion shown in Table 7, a comparison result that the measurement value acquired in step 5a is higher than the reference value can be acquired as data for diagnosing as being positive, and a comparison result that the measurement value acquired in step 5a is equal to or lower than the reference value can be acquired as data for diagnosing as being negative. For a substance being "low" with respect to the criterion shown in Table 7, a comparison result that the measurement value acquired in step 5a is lower than the reference value can be acquired as data for diagnosing as being positive, and a comparison result that the measurement value acquired in step 5a is equal to or higher than the reference value can be acquired as data for diagnosing as being negative.

For substances selected from Group B, a comparison result satisfying the criterion shown in Table 8 can be acquired as data for diagnosing as being positive, and a comparison result not satisfying the criterion shown in Table 8 can be acquired as data for diagnosing as being negative. Specifically, for a substance being "high" with respect to the criterion shown in Table 8, a comparison result that the measurement value acquired in step 5a is higher than the reference value can be acquired as data for diagnosing as being positive, and a comparison result that the measurement value acquired in step 5a is equal to or lower than the reference value can be acquired as data for diagnosing as being negative. For a substance being "low" with respect to the criterion shown in Table 8, a comparison result that the measurement value acquired in step 5a is lower than the reference value can be acquired as data for diagnosing as being positive, and a comparison result that the measurement value acquired in step 5a is equal to or higher than the reference value can be acquired as data for diagnosing as being negative.

In the case where one substance selected from Group A is measured in step 5a, if a comparison result for the one substance satisfies the criterion shown in Table 7, the comparison result can be acquired as data for diagnosing as being negative; if a comparison result for the one substance does not satisfy the criterion shown in Table 7, the comparison result can be acquired as data for diagnosing as being negative.

In the case where two or more substances selected from Group A are measured in step 5a, if a comparison result for at least one substance of the two or more substances satisfies the criterion shown in Table 7, the comparison result can be acquired as data for diagnosing as being positive; if none of comparison results for the two or more substances satisfies the criterion shown in Table 7, the comparison results can be acquired as data for diagnosing as being negative. The larger the number of substances with comparison results satisfying the criteria shown in Table 7, the higher the probability of being positive. Accordingly, two or more (e.g., two, three, or four or more) substances selected from Group A can be measured in step 5a to achieve enhanced accuracy of diagnosing a disease or symptom in a subject.

In the case where one substance selected from Group B is measured in step 5a, if a comparison result for the one substance satisfies the criterion shown in Table 8, the comparison result can be acquired as data for diagnosing as being positive; if a comparison result for the one substance does not satisfy the criterion shown in Table 8, the comparison result can be acquired as data for diagnosing as being negative.

In the case where two or more substances selected from Group B are measured in step 5a, if a comparison result for at least one substance of the two or more substances satisfies the criterion shown in Table 8, the comparison result can be acquired as data for diagnosing as being positive; if none of comparison results for the two or more substances satisfies the criterion shown in Table 8, the comparison results can be acquired as data for diagnosing as being negative. The larger the number of substances with comparison results satisfying the criteria shown in Table 8, the higher the probability of being positive. Accordingly, two or more (e.g., two, three, or four or more) substances selected from Group B can be measured in step 5a to achieve enhanced accuracy of diagnosing a disease or symptom in a subject.

In the case where Embodiment 1-1 of Aspect 1 is applied to Aspect 5, for each substance selected from Group A, a comparison result based on the criterion shown in Table 9 is preferably acquired as data for diagnosis; for each substance selected from Group B, a comparison result based on the criterion shown in Table 10 is preferably acquired as data for diagnosis.

Specifically, for a substance with a criterion of "high", a preferred criterion of x times or more, a more preferred criterion of y times or more, and an even more preferred criterion of z times or more in Table 9 or 10, a comparison result that the measurement value acquired in step 5a is x times or more the average value is preferably acquired as data for diagnosing as being positive, a comparison result that the measurement value acquired in step 5a is y times or more the average value is more preferably acquired as data for diagnosing as being positive, and a comparison result that the measurement value acquired in step 5a is z times or more the average value is even more preferably acquired as data for diagnosing as being positive. For a substance with a criterion of "low", a preferred criterion of x times or less, a more preferred criterion of y times or less, and an even more preferred criterion of z times or less in Table 9 or 10, a comparison result that the measurement value acquired in step 5a is x times or less the average value is preferably acquired as data for diagnosing as being positive, a comparison result that the measurement value acquired in step 5a is y times or less the average value is more preferably acquired as data for diagnosing as being positive, and a comparison result that the measurement value acquired in step 5a is z times or less the average value is even more preferably acquired as data for diagnosing as being positive.

Taking the substance of No. 1 in Group A as an example, a comparison result that the measurement value acquired in step 5a is two times or more the average value is preferably acquired as data for diagnosing as being positive, a comparison result that the measurement value acquired in step 5a is three times or more the average value is more preferably acquired as data for diagnosing as being positive, and a comparison result that the measurement value acquired in step 5a is five times or more the average value is even more preferably acquired as data for diagnosing as being positive.

Taking the substance of No. 2 in Group A as an example, a comparison result that the measurement value acquired in step 5a is 0.8 times or less the average value is preferably acquired as data for diagnosing as being positive, a comparison result that the measurement value acquired in step 5a is 0.7 times or less the average value is more preferably acquired as data for diagnosing as being positive, and a comparison result that the measurement value acquired in step 5a is 0.5 times or less the average value is even more preferably acquired as data for diagnosing as being positive.

The data acquired in step 5b can be used for diagnosing the disease or symptom caused by reduction in intestinal barrier function in the subject, or for assisting diagnosis of the disease or symptom caused by reduction in intestinal barrier function in the subject.

Diagnosis is a practice typically performed by a physician. A physician diagnoses whether the subject is affected by the disease or symptom caused by reduction in intestinal barrier function on the basis of the data acquired in step 5b; if the subject is affected by the disease or symptom caused by reduction in intestinal barrier function, the physician may determine whether prevention or treatment of the disease or symptom is needed for the subject, or select a method for preventing or treating the disease or symptom for the subject, or perform prevention or treatment of the disease or symptom for the subject. The term "prevention" includes prevention, suppression, and retardation of the onset of the disease or symptom. The term "treatment" includes suppression of the progress or exacerbation of the disease or symptom, retardation of the progress or exacerbation of the disease or symptom, and relief, mitigation, amelioration, and cure of the disease or symptom.

The practice of assisting diagnosis of the disease or symptom is a practice of providing the data acquired in step 5b as data for diagnosis of the disease or symptom, and this may be a medical practice or a non-medical practice, but is typically a non-medical practice. Diagnosis by a physician can be performed on the basis of the data provided through the practice of assisting diagnosis of the disease or symptom plus additional one or two or more data.

< <Aspect 6> >

Aspect 6 of the present invention relates to a method for screening for a substance or composition having an improving effect on intestinal barrier function or a preventing or ameliorating effect on a disease or symptom caused by reduction in intestinal barrier function. Hereinafter, the phrase "a preventing or ameliorating effect on a disease or symptom caused by reduction in intestinal barrier function" may be referred to as "the preventing or ameliorating effect on the disease or symptom".

The method according to Aspect 6 includes the steps of:

(6a) administering a candidate substance or candidate composition to a subject having reduced intestinal barrier function;
(6b) measuring a biomarker in a specimen obtained from the subject after administration of the candidate substance or candidate composition to acquire a measurement value for the biomarker; and
(6c) comparing the measurement value acquired in step 6b with a reference value to acquire a comparison result; and
(6d) evaluating the candidate substance or candidate composition for the improving effect on intestinal barrier function or the preventing or ameliorating effect on the disease or symptom on the basis of the comparison result acquired in step 6c,
wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is applied to Aspect 6. In the application, "step 1a" is replaced with "step 6b", "step 1b" is replaced with "step 6c", "step 1c" is replaced with "step 6d", the phrase "evaluating intestinal barrier function in the subject" is replaced with the phrase "evaluating the candidate substance or candidate composition for the improving effect on intestinal barrier function or the preventing or ameliorating effect on the disease or symptom", and the phrase "accuracy of evaluating intestinal barrier function in the subject" is replaced with the phrase "accuracy of evaluating the candidate substance or candidate composition for the improving effect on intestinal barrier function or the preventing or ameliorating effect on the disease or symptom".

In Aspect 6, the term "positive" means that the candidate substance or candidate composition has the improving effect on intestinal barrier function or the preventing or ameliorating effect on the disease or symptom, and the term "negative" means that the candidate substance or candidate composition does not have the improving effect on intestinal barrier function or the preventing or ameliorating effect on the disease or symptom. The term "prevention" includes prevention, suppression, and retardation of the onset of the disease or symptom. The term "amelioration" includes suppression of the progression or exacerbation of the disease or symptom, retardation of the progression or exacerbation of the disease or symptom, and relief, mitigation, amelioration, and cure of the disease or symptom.

The improving effect on intestinal barrier function to be evaluated may be an improving effect on the intestinal barrier function of the small intestine, or an improving effect on the intestinal barrier function of the large intestine, but is preferably an improving effect on the intestinal barrier function of the small intestine.

The preventing or ameliorating effect on the disease or symptom to be evaluated may be a preventing or ameliorating effect on a disease or symptom caused by reduction in the intestinal barrier function of the small intestine, or a preventing or ameliorating effect on a disease or symptom caused by reduction in the intestinal barrier function of the large intestine, but is preferably a preventing or ameliorating effect on a disease or symptom caused by reduction in the intestinal barrier function of the small intestine.

For the disease or symptom caused by reduction in intestinal barrier function, the description thereof given for Aspect 2 is applied.

The method according to Aspect 6 allows screening to obtain a candidate substance or candidate composition evaluated to have the improving effect on intestinal barrier function or the preventing or ameliorating effect on the disease or symptom as a substance or composition having the improving effect on intestinal barrier function or the preventing or ameliorating effect on the disease or symptom.

Step 6a is a step of administering a candidate substance or candidate composition to a subject having reduced intestinal barrier function.

The reduced intestinal barrier function in the subject can be confirmed by a known testing method for intestinal barrier function (e.g., lactulose/mannitol test), or by the method according to Aspect 1.

The method according to Aspect 6 may include a step of evaluating intestinal barrier function in a subject to confirm reduced intestinal barrier function in the subject by the method according to Aspect 1 before step 6a. In the case where the method according to Aspect 6 includes this step, the biomarker to be measured in step 1a in Aspect 1 may be the same as or different from the biomarker to be measured in step 6b in terms of type, but they are preferably the same for enhanced accuracy of evaluating the candidate substance or candidate composition for the improving effect on intestinal barrier function or the preventing or ameliorating effect on the disease or symptom.

The method according to Aspect 6 may include a step of selecting a subject having reduced intestinal barrier function by the method according to Aspect 1 before step 6a. In the case where the method according to Aspect 6 includes this step, the biomarker to be measured in step 1a in Aspect 1 may be the same as or different from the biomarker to be measured in step 6b in terms of type, but they are preferably the same for enhanced accuracy of evaluating the candidate substance or candidate composition for the improving effect on intestinal barrier function or the preventing or ameliorating effect on the disease or symptom.

The subject to which the candidate substance or candidate composition is to be administered is not limited as long as the intestinal barrier function has been reduced, and may be a human having reduced intestinal barrier function or a model animal (except humans) having reduced intestinal barrier function, but is preferably a model animal (except humans) having reduced intestinal barrier function. Such model animals include vertebrates including mammals, reptiles, birds, amphibians, and fish, mammals and birds are preferred, and mammals are more preferred. The mammals include primates (e.g., gorillas, chimpanzees, orangutans), rodents (e.g., mice, rats, hamsters, guinea pigs, rabbits), domestic animals (e.g., cattle, pigs, sheep, goats, horses), and pet animals (e.g., dogs, cats). The birds include poultry (e.g., chickens, wild ducks, ducks).

The candidate substance or candidate composition to be administered to the subject having reduced intestinal barrier function is not limited, and can be selected from, for example, high-molecular-weight compounds, low-molecular-weight compounds, cell cultures, cell extracts, antibodies, proteins, peptides, nucleic acids, carbohydrates, inorganic salts, metal complexes, microorganisms (bacteria (probiotics), yeasts, etc.), dietary fibers, resistant carbohydrates, resistant proteins, fermented products (including fermented foods), prebiotics, and any combination of two or more of these.

Examples of the route of administration of the candidate substance or candidate composition to the subject include oral and parenteral (e.g., intranasal, ophthalmic, ear-drop, transdermal, tracheobronchial, intrarectal, urinary, subcutaneous, intramuscular, intravenous) administrations. The candidate substance or candidate composition may be formulated into a dosage form suitable for the route of administration. Examples of the dosage form include a tablet and an injection. In formulating, a proper excipient for oral administration (e.g., a diluent, a disintegrant, a lubricant, a binder) or an excipient suitable for parenteral administration (e.g., a diluting agent, a solvent) can be used. The dose and frequency of administration of the candidate substance or candidate composition can be appropriately adjusted in consideration of the dosage form and the age, body weight, and so on of the subject. The frequency of administration per day may be once, or two or more times. The period of administration may be 1 day or 2 days or more (e.g., 1 week or more, 2 weeks or more, 3 weeks or more, or 4 weeks or more).

Step 6b is a step of measuring a biomarker in a specimen obtained from the subject after administration of the candidate substance or candidate composition to acquire a measurement value for the biomarker.

Step 6b can be performed in the same manner as step 1a in Aspect 1, except that a specimen obtained from a subject having reduced intestinal barrier function after administration of the candidate substance or candidate composition to the subject is used.

The specimen to be used in step 6b is obtained from a subject having reduced intestinal barrier function after administration of the candidate substance or candidate composition to the subject. The specimen may be obtained from the subject at multiple time points after administration of the candidate substance or candidate composition. The time point to obtain the specimen can be appropriately adjusted in consideration of the dosage form and the age, body weight, and so on of the subject. In an embodiment, the time point to obtain the specimen is, for example, any time point between 0.5 and 24 hours after administration, or any time point between 0.5 and 12 hours after administration, or any time point between 1 and 10 hours after administration. Here, the meaning of "after administration" may be "after one administration" or "after two or more continuous administrations". The meaning of "after two or more continuous administrations" may be, for example, "after the final administration of continuous administrations for 4 weeks or more", or "after the final administration of continuous administrations for 1 day to 4 weeks", or "after the final administration of continuous administrations for 3 days to 3 weeks", or "after the final administration of continuous administrations for 1 week to 2 weeks".

Step 6c is a step of comparing the measurement value acquired in step 6b with a reference value to acquire a comparison result.

Step 6c can be performed in the same manner as step 1b in Aspect 1, except that the measurement value acquired in step 6b is used.

Step 6d is a step of evaluating the candidate substance or candidate composition for the improving effect on intestinal barrier function or the preventing or ameliorating effect on the disease or symptom on the basis of the comparison result acquired in step 6c.

Step 6d can be performed in the same manner as step 1c in Aspect 1, except that the candidate substance or candidate composition is evaluated for the improving effect on intestinal barrier function or the preventing or ameliorating effect on the disease or symptom.

For substances selected from Group A, the candidate substance or candidate composition can be evaluated for the improving effect on intestinal barrier function or the preventing or ameliorating effect on the disease or symptom on the basis of whether the comparison result acquired in step 6c satisfies the criterion shown in Table 7. Specifically, for a substance being "high" with respect to the criterion shown in Table 7, if the measurement value acquired in step 6b is higher than the reference value (i.e., the comparison result acquired in step 6c satisfies the criterion shown in Table 7), the case can be evaluated as being negative; if the measurement value acquired in step 6b is equal to or lower than the reference value (i.e., the comparison result acquired in step 6c does not satisfy the criterion shown in Table 7), the case can be evaluated as being positive. For a substance being "low" with respect to the criterion shown in Table 7, if the measurement value acquired in step 6b is lower than the reference value (i.e., the comparison result acquired in step 6c satisfies the criterion shown in Table 7), the case can be evaluated as being negative; if the measurement value acquired in step 6b is equal to or higher than the reference value (i.e., the comparison result acquired in step 6c does not satisfy the criterion shown in Table 7), the case can be evaluated as being positive.

For substances selected from Group B, the candidate substance or candidate composition can be evaluated for the improving effect on intestinal barrier function or the preventing or ameliorating effect on the disease or symptom on the basis of whether the comparison result acquired in step 6c satisfies the criterion shown in Table 8. Specifically, for a substance being "high" with respect to the criterion shown in Table 8, if the measurement value acquired in step 6b is higher than the reference value (i.e., the comparison result acquired in step 6c satisfies the criterion shown in Table 8), the case can be evaluated as being negative; if the measurement value acquired in step 6b is equal to or lower than the reference value (i.e., the comparison result acquired in step 6c does not satisfy the criterion shown in Table 8), the case can be evaluated as being positive. For a substance being "low" with respect to the criterion shown in Table 8, if the measurement value acquired in step 6b is lower than the reference value (i.e., the comparison result acquired in step 6c satisfies the criterion shown in Table 8), the case can be evaluated as being negative; if the measurement value acquired in step 6b is equal to or higher than the reference value (i.e., the comparison result acquired in step 6c does not satisfy the criterion shown in Table 8), the case can be evaluated as being positive.

In the case where one substance selected from Group A is measured in step 6b, if a comparison result for the one substance satisfy the criterion shown in Table 7, the case can be evaluated as being negative; if a comparison result for the one substance does not satisfy the criterion shown in Table 7, the case can be evaluated as being positive.

In the case where two or more substances selected from Group A are measured in step 6b, if all comparison results for the two or more substances satisfy the criteria shown in Table 7, the case can be evaluated as being negative; if a comparison result for at least one substance of the two or more substances does not satisfy the criterion shown in Table 7, the case can be evaluated as being positive. The larger the number of substances with comparison results not satisfying the criteria shown in Table 7, the higher the probability of being positive. Accordingly, two or more (e.g., two, three, or four or more) substances selected from Group A can be measured in step 6b to achieve enhanced accuracy of evaluating a candidate substance or candidate composition for the improving effect on intestinal barrier function or the preventing or ameliorating effect on the disease or symptom.

In the case where one substance selected from Group B is measured in step 6b, if a comparison result for the one substance satisfy the criterion shown in Table 8, the case can be evaluated as being negative; if a comparison result for the one substance does not satisfy the criterion shown in Table 8, the case can be evaluated as being positive.

In the case where two or more substances selected from Group B are measured in step 6b, if all comparison results for the two or more substances satisfy the criteria shown in Table 8, the case can be evaluated as being negative; if a comparison result for at least one substance of the two or more substances does not satisfy the criterion shown in Table 8, the case can be evaluated as being positive. The larger the number of substances with comparison results not satisfying the criteria shown in Table 8, the higher the probability of being positive. Accordingly, two or more (e.g., two, three, or four or more) substances selected from Group B can be measured in step 6b to achieve enhanced accuracy of evaluating a candidate substance or candidate composition for the improving effect on intestinal barrier function or the preventing or ameliorating effect on the disease or symptom.

< <Aspect 7> >

Aspect 7 of the present invention relates to a method for evaluating a candidate substance or candidate composition for an improving effect on intestinal barrier function or a preventing or ameliorating effect on a disease or symptom caused by reduction in intestinal barrier function. Hereinafter, the phrase "a preventing or ameliorating effect on a disease or symptom caused by reduction in intestinal barrier function" may be referred to as "the preventing or ameliorating effect on the disease or symptom".

The method according to Aspect 7 includes the steps of:

(7a) administering a candidate substance or candidate composition to a subject having reduced intestinal barrier function;
(7b) measuring a biomarker in a specimen obtained from the subject after administration of the candidate substance or candidate composition to acquire a measurement value for the biomarker; and
(7c) comparing the measurement value acquired in step 7b with a reference value to acquire a comparison result; and
(7d) evaluating the candidate substance or candidate composition for the improving effect on intestinal barrier function or the preventing or ameliorating effect on the disease or symptom on the basis of the comparison result acquired in step 7c,
wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 6 is applied to Aspect 7. In the application, "step 6a" is replaced with "step 7a", "step 6b" is replaced with "step 7b", "step 6c" is replaced with "step 7c", and "step 6d" is replaced with "step 7d".

< <Aspect 8 > >

Aspect 8 of the present invention relates to a biomarker in a specimen derived from a subject for use in evaluating intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 1 is applied to Aspect 8.

< <Aspect 9> >

Aspect 9 of the present invention relates to a biomarker in a specimen derived from a subject for use in diagnosing a disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 2 is applied to Aspect 9.

< <Aspect 10 > >

Aspect 10 of the present invention relates to a biomarker in a specimen derived from a subject for use in evaluating a subject's risk of developing a disease or symptom caused by reduction in intestinal barrier function, or probability of the subject being affected by the disease or symptom, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 4 is applied to Aspect 10.

< <Aspect 11 > >

Aspect 11 of the present invention relates to a biomarker in a specimen derived from a subject for use in acquiring data for diagnosing a disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 5 is applied to Aspect 11.

<<Aspect 12>>

Aspect 12 of the present invention relates to a biomarker in a specimen for use in screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 6 is applied to Aspect 12.

<<Aspect 13>>

Aspect 13 of the present invention relates to a biomarker in a specimen for use in evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 7 is applied to Aspect 13.

< <Aspect 14>>

Aspect 14 of the present invention relates to use of a biomarker in a specimen derived from a subject for evaluating intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 1 is applied to Aspect 14.

<<Aspect 15>>

Aspect 15 of the present invention relates to use of a biomarker in a specimen derived from a subject for diagnosing a disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 2 is applied to Aspect 15.

< <Aspect 16>>

Aspect 16 of the present invention relates to use of a biomarker in a specimen derived from a subject for evaluating a subject's risk of developing a disease or symptom caused by reduction in intestinal barrier function, or probability of the subject being affected by the disease or symptom, wherein the specimen is dry feces or wet feces, wherein when the specimen is dry feces, the biomarker includes one or more substances selected from Group A, and wherein when the specimen is wet feces, the biomarker includes one or more substances selected from Group B.
Unless otherwise specified, the description of Aspect 4 is applied to Aspect 16.

< <Aspect 17>>

Aspect 17 of the present invention relates to use of a biomarker in a specimen derived from a subject for acquiring data for diagnosing a disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 5 is applied to Aspect 17.

< <Aspect 18 > >

Aspect 18 of the present invention relates to use of a biomarker in a specimen for screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 6 is applied to Aspect 18.

< <Aspect 19>>

Aspect 19 of the present invention relates to use of a biomarker in a specimen for evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 7 is applied to Aspect 19.

< <Aspect 20>>

Aspect 20 of the present invention relates to a kit for evaluating intestinal barrier function in a subject, wherein the kit includes one or more reagents for measuring a biomarker in a specimen derived from the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is applied to Aspect 20.

The kit may include one reagent, or two or more reagents.

A reagent of any type that fits with the type of method for measuring a target substance can be appropriately selected. Examples of the method for measuring a target substance include liquid chromatography-mass spectrometry (LC-MS), capillary electrophoresis-mass spectrometry (CE-MS), gas chromatography-mass spectrometry (GC-MS), mass spectrometry (MS), and high-performance liquid chromatography (HPLC), and CE-MS or LC-MS is preferred among these. CE-MS is suitable for measurement of water-soluble substances, and LC-MS is suitable for measurement of liposoluble substances. Particularly preferred among different types of CE-MS is capillary electrophoresis-Fourier transform mass spectrometry (CE-FTMS). Particularly preferred among different types of LC-MS is liquid chromatography-tandem mass spectrometry (LC-MS/MS).

In an embodiment, the kit includes one or more reagents for measuring the concentration of a target substance by CE-MS. The concentration may be a relative concentration or an absolute concentration, but is preferably an absolute concentration. The relative concentration is, for example, a relative area value CE. Examples of the reagent for measuring the concentration of a target substance by CE-MS include authentic samples of one or more substances selected from Group A or Group B.

In another embodiment, the kit include one or more reagents for measuring the concentration of a target substance by LC-MS. The concentration may be a relative concentration or an absolute concentration, but is preferably an absolute concentration. The relative concentration is, for example, a relative area value LC. Examples of the reagent for measuring the concentration of a target substance by LC-MS include authentic samples of one or more substances selected from Group A or Group B.

< <Aspect 21 > >

Aspect 21 of the present invention relates to a kit for diagnosing a disease or symptom caused by reduction in intestinal barrier function in a subject, wherein the kit includes one or more reagents for measuring a biomarker in a specimen derived from the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 2 is applied to Aspect 21.

For the reagents, the description thereof given for Aspect 20 is applied.

< <Aspect 22>>

Aspect 22 of the present invention relates to a kit for evaluating a subject's risk of developing a disease or symptom caused by reduction in intestinal barrier function, or probability of the subject being affected by the disease or symptom, wherein the kit includes one or more reagents for measuring a biomarker in a specimen derived from the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 4 is applied to Aspect 22.

For the reagents, the description thereof given for Aspect 20 is applied.

< <Aspect 23> >

Aspect 23 of the present invention relates to a kit for acquiring data for diagnosing a disease or symptom caused by reduction in intestinal barrier function in a subject, wherein the kit includes one or more reagents for measuring a biomarker in a specimen derived from the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 5 is applied to Aspect 23.

For the reagents, the description thereof given for Aspect 20 is applied.

< <Aspect 24>>

Aspect 24 of the present invention relates to a kit for screening for a substance or composition having an improving effect on intestinal barrier function or a preventing or ameliorating effect on a disease or symptom caused by reduction in intestinal barrier function, wherein the kit includes one or more reagents for measuring a biomarker in a specimen obtained from a subject having reduced intestinal barrier function after administration of a candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 6 is applied to Aspect 24.

For the reagents, the description thereof given for Aspect 20 is applied.

< <Aspect 25>>

Aspect 25 of the present invention relates to a kit for evaluating a candidate substance or candidate composition for an improving effect on intestinal barrier function or a preventing or ameliorating effect on a disease or symptom caused by reduction in intestinal barrier function, wherein the kit includes one or more reagents for measuring a biomarker in a specimen obtained from a subject having reduced intestinal barrier function after administration of the candidate substance or candidate composition to the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 7 is applied to Aspect 25.

For the reagents, the description thereof given for Aspect 20 is applied.

< <Aspect 26>>

Aspect 26 of the present invention relates to a method for producing a food product or pharmaceutical composition, the method including the step of blending a substance or composition obtained through screening by the method according to Aspect 6 with one or two or more components to constitute the food product or pharmaceutical composition.

The one or two or more components to be blended with the substance or composition obtained through screening by the method according to Aspect 6 may be any ones acceptable as components for a food product or pharmaceutical composition without limitation, and appropriate ones can be selected to fit with the type of food product or pharmaceutical composition to be produced.

The method according to Aspect 26 may include a step of screening for a substance or composition by the method according to Aspect 6.

< <Aspect 27> >

Aspect 27 of the present invention relates to a computer program for allowing a computer to execute a method for evaluating intestinal barrier function in a subject.

The method that a computer is allowed to execute by the computer program according to Aspect 27 includes the steps of:

(S101) acquiring a measurement value for a biomarker in a specimen derived from the subject;
(S102) comparing the measurement value acquired in step S101 with a reference value to acquire a comparison result; and
(S103) evaluating intestinal barrier function in the subject on the basis of the comparison result acquired in step S102, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 1 is applied to Aspect 27.

The computer program according to Aspect 27 allows a computer to execute a method for evaluating intestinal barrier function in a subject when the computer program is executed by the computer. The computer program according to Aspect 27 includes an instruction to allow a computer to execute a method for evaluating intestinal barrier function in a subject.

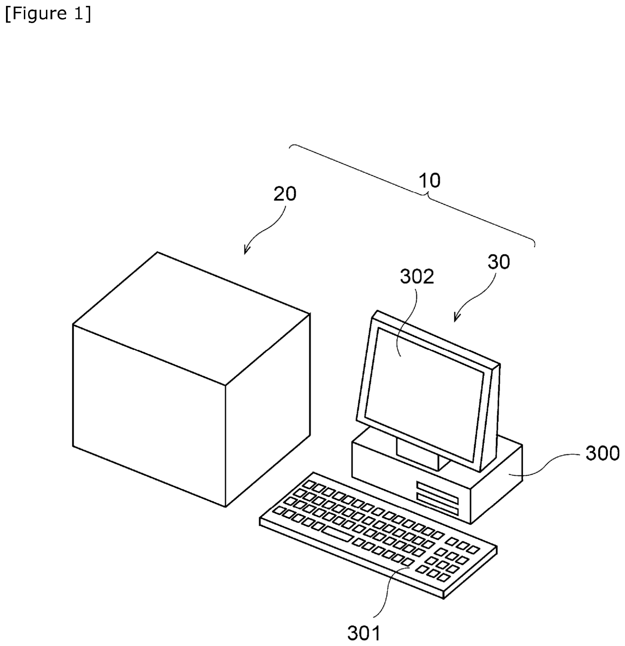

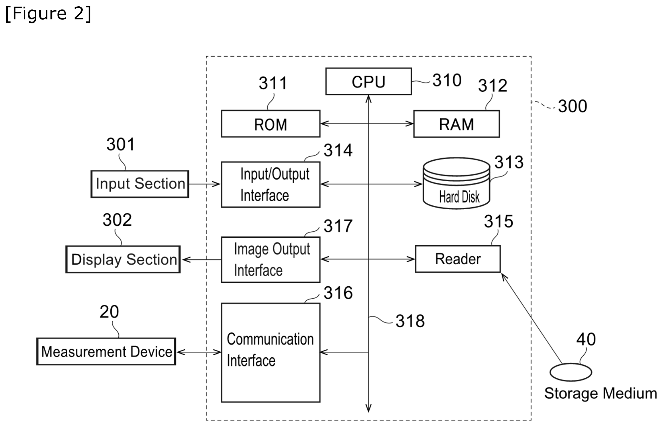

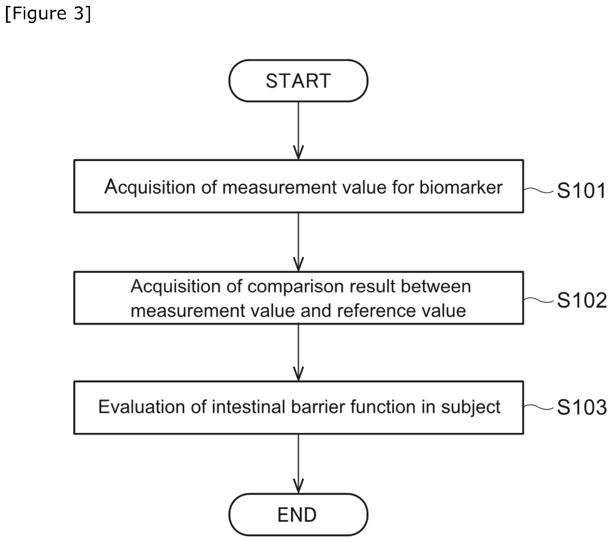

Hereinafter, an embodiment of Aspect 27 will be described with reference to drawings. Figure 1 is a schematic diagram of an apparatus to be used in the present embodiment, Figure 2 is a block diagram illustrating the hardware configuration of the apparatus of the present embodiment, and Figures 3 to 5 are each a flowchart illustrating a procedure with the apparatus of the present embodiment.

As illustrated in Figure 1, an apparatus 10 includes a computer system 30. As illustrated in Figure 1, the apparatus 10 may include a measurement device 20 connected to the computer system 30. The computer system 30 may be a system provided separately from the measurement device 20, or a system including the measurement device 20 therein. The apparatus 10 may be an apparatus in which the measurement device 20 and the computer system 30 are integrally configured.

The measurement device 20 executes measurement of a biomarker in a specimen derived from the subject to acquire a measurement value for the biomarker (e.g., an absolute concentration, a relative concentration, or a combined variable) or a measurement value necessary for calculating a measurement value for the biomarker (e.g., a measurement value necessary for calculating a relative concentration or a combined variable), and sends the acquired value to the computer system 30. For the specimen derived from the subject, the measurement of the biomarker, and the measurement value for the biomarker, the description thereof given for Aspect 1 is applied.

For example, the measurement device 20 is an automated measurement device that executes measurement of the biomarker by CE-MS (in particular, CE-FTMS) or LC-MS (in particular, LC-MS/MS) to acquire a measurement value for the biomarker (e.g., a relative area value CE, a relative area value LC, or a combined variable) or a measurement value necessary for calculating a measurement value for the biomarker (e.g., a measurement value necessary for calculating a relative area value CE, a relative area value LC, or a combined variable).

The measurement device 20 is needed to be able to execute measurement of the biomarker in a measurement sample (e.g., a measurement sample to be used for CE-MS (in particular, CE-FTMS) or LC-MS (in particular, LC-MS/MS)) and the subsequent process. Processes prior to measurement of the biomarker in a measurement sample such as collection of feces derived from the subject (i.e., collection of feces to be used as a specimen from feces excreted by the subject), preparation of a measurement sample from feces derived from the subject, and setting of a measurement sample to the measurement device 20 may be executed by a human or by the measurement device 20. In an embodiment, processes prior to measurement of the biomarker in a measurement sample are executed by a human.

As illustrated in Figure 2, the computer system 30 includes: a computer main body 300; an input section 301; and a display section 302 that displays specimen information, evaluation results, and so on.

Based on the measurement value for the biomarker, a processor of the computer system 30 executes the computer program, installed in a hard disk 313, for evaluating intestinal barrier function in the subject.

As illustrated in Figure 2, the computer main body 300 includes: a CPU (Central Processing Unit) 310; a ROM (Read Only Memory) 311; a RAM (Random Access Memory) 312; a hard disk 313; an input/output interface 314; a reader 315; a communication interface 316; and an image output interface 317. The CPU 310, ROM 311, RAM 312, hard disk 313, input/output interface 314, reader 315, communication interface 316, and image output interface 317 are connected together via buses 318 in a manner that allows data communication. The measurement device 20 is communicatively connected to the computer system 30 via the communication interface 316.

The CPU 310 is capable of executing a program stored in the ROM 311 or hard disk 313 or a program loaded in the RAM 312. Through execution of a program by the CPU 310, the apparatus 10 functions as an apparatus for evaluating intestinal barrier function in the subject.

The ROM 311 is configured with a mask ROM, a PROM, an EPROM, an EEPROM, or the like. A computer program to be executed by the CPU 310 and data to be used for execution of the computer program are stored in the ROM 311.

The RAM 312 is configured with an SRAM, a DRAM, or the like. The RAM 312 is used for reading programs stored in the ROM 311 and hard disk 313. In addition, the RAM 312 is used as a working area for the CPU 310 when a program is executed.

In the hard disk 313, an operating system to be executed by the CPU 310, computer programs including application programs, and data to be used for execution of the computer programs have been installed.

The reader 315 is configured with a flexible disk drive, a CD-ROM drive, a DVD-ROM drive, a USB port, an SD card reader, a CF card reader, a memory stick reader, a solid state drive, or the like. The reader 315 is capable of reading programs or data stored in a transportable storage medium 400.

The input/output interface 314 is configured with a serial interface such as USB, IEEE 1394, and RS-232C, a parallel interface such as SCSI, IDE, and IEEE 1284, and an analog interface consisting of a D/A converter, an A/D converter, or the like. To the input/output interface 314, the input section 301 including a keyboard and a mouse is connected. An operator can input various instructions to the computer main body 300 via the input section 301.

The communication interface 316 is, for example, an Ethernet (R) interface. The computer main body 300 is capable of executing sending of print data, for example, to a printer, a computer of the subject or a service provider, or a smartphone terminal of the subject through the communication interface 316.

The image output interface 317 is connected to the display section 302 configured with an LCD, a CRT, or the like. With this configuration, the display section 302 is capable of outputting video signals corresponding to image data given by the CPU 310. The display section 302 displays an image (picture) according to inputted video signals. The display section 302 may display raw data themselves, or an illustration, a drawing, or the like made by converting raw data for easy understanding by the subject.

Hereinafter, a procedure with the apparatus 10 will be described with reference to Figures 3 to 5.

As illustrated in Figure 3, in step S101, the CPU 310 acquires a measurement value for a biomarker in a specimen derived from the subject. For the specimen derived from the subject and the measurement value for the biomarker, the description thereof given for Aspect 1 is applied.

In an embodiment, the measurement device 20 executes measurement of a biomarker in a specimen derived from the subject, acquires a measurement value for the biomarker (e.g., an absolute concentration, a relative concentration, or a combined variable), and sends the acquired value to the computer system 30. For the specimen derived from the subject, the measurement of the biomarker, and the measurement value for the biomarker, the description thereof given for Aspect 1 is applied. The CPU 310 stores the measurement value for the biomarker, the measurement value sent from the measurement device 20, in the hard disk 313. In step S301, the CPU 310 acquires the measurement value for the biomarker, the measurement value stored in the hard disk 313.

In another embodiment, the measurement device 20 executes measurement of a biomarker in a specimen derived from the subject, acquires a measurement value necessary for calculating a measurement value for the biomarker (e.g., a measurement value necessary for calculating a relative concentration or a combined variable), and sends the acquired value to the computer system 30. For the specimen derived from the subject, the measurement of the biomarker, and the measurement value for the biomarker, the description thereof given for Aspect 1 is applied. Based on the measurement value sent from the measurement device 20, the CPU 310 calculates a measurement value for the biomarker (e.g., a relative concentration or a combined variable), and stores the measurement value for the biomarker in the hard disk 313. In step S301, the CPU 310 acquires the measurement value for the biomarker, the measurement value stored in the hard disk 313.

In still another embodiment, an operator inputs a measurement value for a biomarker (e.g., an absolute concentration, a relative concentration, or a combined variable), the measurement value acquired in advance through measurement of the biomarker in a specimen derived from the subject, via the input section 301. For the specimen derived from the subject, the measurement of the biomarker, and the measurement value for the biomarker, the description thereof given for Aspect 1 is applied. The CPU 310 stores the inputted measurement value for the biomarker in the hard disk 313. In step S301, the CPU 310 acquires the measurement value for the biomarker, the measurement value stored in the hard disk 313.

In still another embodiment, an operator inputs a measurement value necessary for calculating a measurement value for a biomarker (e.g., a measurement value necessary for calculating a relative concentration or a combined variable), the measurement value acquired in advance through measurement of the biomarker in a specimen derived from the subject, via the input section 301. For the specimen derived from the subject, the measurement of the biomarker, and the measurement value for the biomarker, the description thereof given for Aspect 1 is applied. Based on the inputted measurement value, the CPU 310 calculates a measurement value for the biomarker (e.g., a relative concentration or a combined variable), and stores the measurement value for the biomarker in the hard disk 313. In step S301, the CPU 310 acquires the measurement value for the biomarker, the measurement value stored in the hard disk 313.

As illustrated in Figure 3, in step S102, the CPU 310 compares the measurement value for the biomarker with a reference value stored in the hard disk 313 to acquire a comparison result. For the reference value, the comparison, and the comparison result, the description thereof given for Aspect 1 is applied. The CPU 310 stores the acquired comparison result in the hard disk 313. The CPU 310 may output the acquired comparison result to allow the display section 302 to display the comparison result, or allow a printer to print the comparison result, or transfer the comparison result to another computer or an application (such as a smartphone). In outputting the comparison result, the CPU 310 may allow the display section 302 to display the measurement value for the biomarker, the reference value, and others as reference information, or allow a printer to print them, or transfer them to another computer or an application (such as a smartphone). Together with the comparison result, the CPU 310 may allow the display section 302 to display advice on life, foods recommended to ingest (including functional foods), and others for improved intestinal barrier function, or allow a printer to print them, or transfer them to another computer or an application (such as a smartphone). Thereby, an index for evaluating intestinal barrier function in the subject can be provided, for example, to a physician, a nurse, a caregiver, a tester, a testing business operator, a service provider, or the subject himself/herself.

As illustrated in Figure 3, in step S103, the CPU 310 evaluates intestinal barrier function in the subject on the basis of the comparison result acquired in step S102. For the evaluation, the description thereof given for Aspect 1 is applied. The CPU 310 stores the evaluation result in the hard disk 313. The CPU 310 may output the evaluation result to allow the display section 302 to display the evaluation result, or allow a printer to print the evaluation result, or transfer the evaluation result to another computer or an application (such as a smartphone). In outputting the evaluation result, the CPU 310 may allow the display section 302 to display the measurement value for the biomarker, the reference value, and others as reference information, or allow a printer to print them, or transfer them to another computer or an application (such as a smartphone). Together with the evaluation result, the CPU 310 may allow the display section 302 to display advice on life, foods recommended to ingest (including functional foods), and others for improved intestinal barrier function, or allow a printer to print them, or transfer them to another computer or an application (such as a smartphone). Thereby, an index for evaluating intestinal barrier function in the subject can be provided, for example, to a physician, a nurse, a caregiver, a tester, a testing business operator, a service provider, or the subject himself/herself.

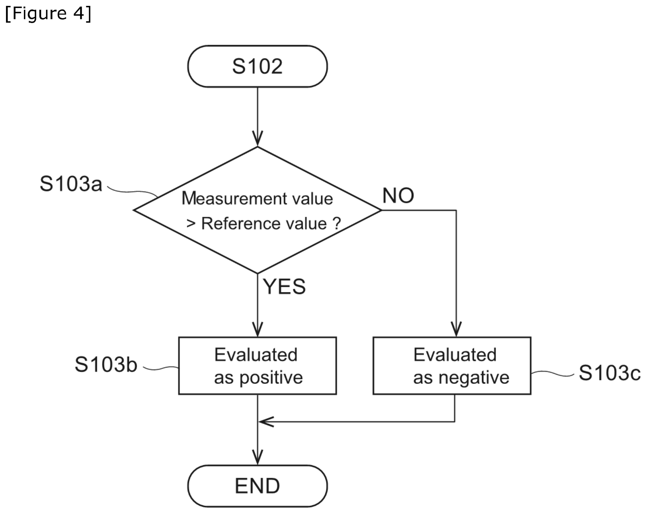

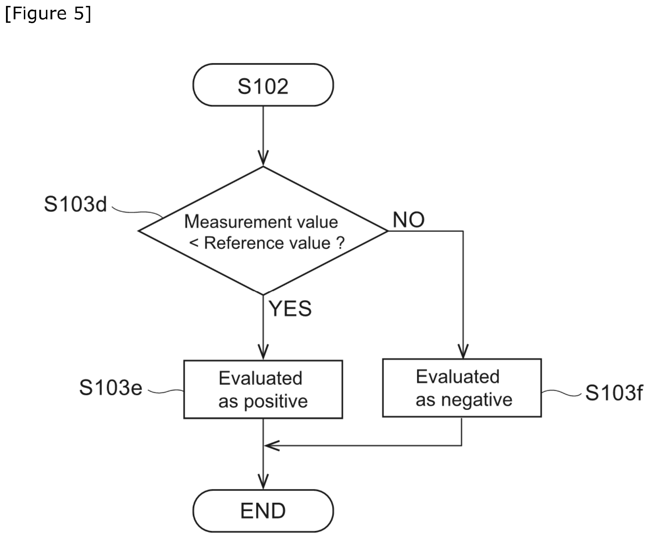

For substances selected from Group A, the CPU 310 evaluates intestinal barrier function in the subject on the basis of whether the comparison result acquired in step S102 satisfies the criterion shown in Table 7. Specifically, for a substance being "high" with respect to the criterion shown in Table 7, as shown in Figure 4, the CPU 310 determines whether the measurement value acquired in step 101 is higher than the reference value (i.e., whether the comparison result satisfies the criterion shown in Table 7) (step S103a); if the measurement value acquired in step 101 is higher than the reference value (i.e., the comparison result satisfies the criterion shown in Table 7), the case is evaluated as being positive (step S103b); if the measurement value acquired in step 101 is equal to or lower than the reference value (i.e., the comparison result does not satisfy the criterion shown in Table 7), the case is evaluated as being negative (step S103c). For a substance being "low" with respect to the criterion shown in Table 7, as shown in Figure 5, the CPU 310 determines whether the measurement value acquired in step 101 is lower than the reference value (i.e., whether the comparison result satisfies the criterion shown in Table 7) (step S103d); if the measurement value acquired in step 101 is lower than the reference value (i.e., the comparison result satisfies the criterion shown in Table 7), the case is evaluated as being positive (step S103e); if the measurement value acquired in step 101 is equal to or higher than the reference value (i.e., the comparison result does not satisfy the criterion shown in Table 7), the case is evaluated as being negative (step S103f).

For substances selected from Group B, the CPU 310 evaluates intestinal barrier function in the subject on the basis of whether the comparison result acquired in step S102 satisfies the criterion shown in Table 8. Specifically, for a substance being "high" with respect to the criterion shown in Table 8, as shown in Figure 4, the CPU 310 determines whether the measurement value acquired in step 101 is higher than the reference value (i.e., whether the comparison result satisfies the criterion shown in Table 8) (step S103a); if the measurement value acquired in step 101 is higher than the reference value (i.e., the comparison result satisfies the criterion shown in Table 8), the case is evaluated as being positive (step S103b); if the measurement value acquired in step 101 is equal to or lower than the reference value (i.e., the comparison result does not satisfy the criterion shown in Table 8), the case is evaluated as being negative (step S103c). For a substance being "low" with respect to the criterion shown in Table 8, as shown in Figure 5, the CPU 310 determines whether the measurement value acquired in step 101 is lower than the reference value (i.e., whether the comparison result satisfies the criterion shown in Table 8) (step S103d); if the measurement value acquired in step 101 is lower than the reference value (i.e., the comparison result satisfies the criterion shown in Table 8), the case is evaluated as being positive (step S103e); if the measurement value acquired in step 101 is equal to or higher than the reference value (i.e., the comparison result does not satisfy the criterion shown in Table 8), the case is evaluated as being negative (step S103f).

<<Aspect 28>>

Aspect 28 of the present invention relates to a computer program for allowing a computer to execute a method for diagnosing a disease or symptom caused by reduction in intestinal barrier function in a subject. Hereinafter, "a disease or symptom caused by reduction in intestinal barrier function" may be referred to as "the disease or symptom".
The method that a computer is allowed to execute by the computer program according to Aspect 28 includes the steps of:

> (S201) acquiring a measurement value for a biomarker in a specimen derived from the subject;
> (S202) comparing the measurement value acquired in step S201 with a reference value to acquire a comparison result; and
> (S203) diagnosing the disease or symptom caused by reduction in intestinal barrier function in the subject on the basis of the comparison result acquired in step S202,
> wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 2 is applied to Aspect 28. Unless otherwise specified, the description of Aspect 27 is applied to Aspect 28. In the application, "step S101" is replaced with "step S201", "step S102" is replaced with "step S202", "step S103" is replaced with "step S203", the phrase "evaluating intestinal barrier function in the subject" is replaced with the phrase "diagnosing the disease or symptom in the subject", the phrase "accuracy of evaluating intestinal barrier function in the subject" is replaced with the phrase "accuracy of diagnosing the disease or symptom in the subject", the phrase "evaluating as being positive" is replaced with the phrase "diagnosing as being positive", and the phrase "evaluating as being negative" is replaced with the phrase "diagnosing as being negative".

< <Aspect 29> >

Aspect 29 of the present invention relates to a computer program for allowing a computer to execute a method for evaluating a subject's risk of developing a disease or symptom caused by reduction in intestinal barrier function, or probability of the subject being affected by the disease or symptom. Hereinafter, "a subject's risk of developing a disease or symptom caused by reduction in intestinal barrier function" may be refers to as "the risk of the development", and "probability of the subject being affected by a disease or symptom caused by reduction in intestinal barrier function" may be referred to as "the probability of being affected".
The method that a computer is allowed to execute by the computer program according to Aspect 29 includes the steps of:

> (S301) acquiring a measurement value for a biomarker in a specimen derived from the subject;
> (S302) comparing the measurement value acquired in step S301 with a reference value to acquire a comparison result; and
> (S303) evaluating the risk or the probability on the basis of the comparison result acquired in step S302,
> wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 4 is applied to Aspect 29. Unless otherwise specified, the description of Aspect 27 is applied to Aspect 29. In the application, "step S101" is replaced with "step S301", "step S102" is replaced with "step S302", "step S103" is replaced with "step S303", the phrase "evaluating intestinal barrier function in the subject" is replaced with the phrase "evaluating the risk of the development or the probability of being affected", and the phrase "accuracy of evaluating intestinal barrier function in the subject" is replaced with the phrase "accuracy of evaluating the risk of the development or the probability of being affected".

< <Aspect 30> >

Aspect 30 of the present invention relates to a computer program for allowing a computer to execute a method for acquiring data for diagnosing a disease or symptom caused by reduction in intestinal barrier function in a subject. Hereinafter, "a disease or symptom caused by reduction in intestinal barrier function" may be referred to as "the disease or symptom".

The method that a computer is allowed to execute by the computer program according to Aspect 30 includes the steps of:

(S401) acquiring a measurement value for a biomarker in a specimen derived from the subject; and
(S402) comparing the measurement value acquired in step S401 with a reference value to acquire a comparison result as the data for diagnosing the disease or symptom caused by reduction in intestinal barrier function in the subject, wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 5 is applied to Aspect 30. Unless otherwise specified, the description of Aspect 27 is applied to Aspect 30. In the application, "step S101" is replaced with "step S401", "step S102" is replaced with "step S402", the phrase "evaluating intestinal barrier function in the subject" is replaced with the phrase "acquiring data for diagnosing the disease or symptom in the subject", and the phrase "accuracy of evaluating intestinal barrier function in the subject" is replaced with the phrase "accuracy of diagnosing the disease or symptom in the subject".

< <Aspect 31>>

Aspect 31 of the present invention relates to a computer program for allowing a computer to execute a method for screening for a substance or composition having an improving effect on intestinal barrier function or a preventing or ameliorating effect on a disease or symptom caused by reduction in intestinal barrier function. Hereinafter, the phrase "a preventing or ameliorating effect on a disease or symptom caused by reduction in intestinal barrier function" may be referred to as "the preventing or ameliorating effect on the disease or symptom".

The method that a computer is allowed to execute by the computer program according to Aspect 31 includes the steps of:

(S501) acquiring a measurement value for a biomarker in a specimen obtained from a subject having reduced intestinal barrier function after administration of a candidate substance or candidate composition to the subject;
(S502) comparing the measurement value acquired in step S501 with a reference value to acquire a comparison result; and
(S503) evaluating the candidate substance or candidate composition for the improving effect on intestinal barrier function or the preventing or ameliorating effect on the disease or symptom on the basis of the comparison result acquired in step S502,
wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 6 is applied to Aspect 31. Unless otherwise specified, the description of Aspect 27 is applied to Aspect 31. In the application, "step S101" is replaced with "step S501", "step S102" is replaced with "step S502", "step S103" is replaced with "step S503", the phrase "evaluating intestinal barrier function in the subject" is replaced with the phrase "evaluating the candidate substance or candidate composition for the improving effect on intestinal barrier function or the preventing or ameliorating effect on the disease or symptom", and the phrase "accuracy of evaluating intestinal barrier function in the subject" is replaced with the phrase "accuracy of evaluating the candidate substance or candidate composition for the improving effect on intestinal barrier function or the preventing or ameliorating effect on the disease or symptom".

< <Aspect 32> >

Aspect 32 of the present invention relates to a computer program for allowing a computer to execute a method for evaluating a candidate substance or candidate composition for an improving effect on intestinal barrier function or a preventing or ameliorating effect on a disease or symptom caused by reduction in intestinal barrier function. Hereinafter, "a preventing or ameliorating effect on a disease or symptom caused by reduction in intestinal barrier function" may be referred to as "the preventing or ameliorating effect on the disease or symptom".

The method that a computer is allowed to execute by the computer program according to Aspect 32 includes the steps of:

(S601) acquiring a measurement value for a biomarker in a specimen obtained from a subject having reduced intestinal barrier function after administration of a candidate substance or candidate composition to the subject;
(S602) comparing the measurement value acquired in step S601 with a reference value to acquire a comparison result; and
(S603) evaluating the candidate substance or candidate composition for the improving effect on intestinal barrier function or the preventing or ameliorating effect on the disease or symptom on the basis of the comparison result acquired in step S602,
wherein the specimen is dry feces or wet feces, wherein when the specimen is dry feces, the biomarker includes one or more substances selected from Group A, and wherein when the specimen is wet feces, the biomarker includes one or more substances selected from Group B.

Unless otherwise specified, the description of Aspect 7 is applied to Aspect 32. Unless otherwise specified, the description of Aspect 27 is applied to Aspect 32. In the application, "step S101" is replaced with "step S601", "step S102" is replaced with "step S602", "step S103" is replaced with "step S603", the phrase "evaluating intestinal barrier function in the subject" is replaced with the phrase "evaluating the candidate substance or candidate composition for the improving effect on intestinal barrier function or the preventing or ameliorating effect on the disease or symptom", and the phrase "accuracy of evaluating intestinal barrier function in the subject" is replaced with the phrase "accuracy of evaluating the candidate substance or candidate composition for the improving effect on intestinal barrier function or the preventing or ameliorating effect on the disease or symptom".

< <Aspect 33> >

Aspect 33 of the present invention relates to a computer-readable storage medium storing the computer program according to any one of Aspects 27 to 32.

Examples of the computer-readable storage medium storing the program include a non-temporary storage medium such as a ROM, a floppy disk (R), a hard disk, an optical disk, a magneto-optical disk, a CD-ROM, magnetic tape, and a nonvolatile memory card.

EXAMPLES

Hereinafter, the present invention will be described on the basis of Examples.

Lactulose/mannitol test, a known testing method for intestinal barrier function, was carried out for 108 test subjects. The lactulose/mannitol test was carried out as follows. Each test subject was instructed to finish his or her meal by 9 p.m. on the day before testing, and allowed to drink water before bedtime. On the day of testing, each test subject, without being allowed to eat or drink water, was subjected to sugar tolerance test. Each test subject was instructed to ingest a sugar solution (prepared by dissolving 10 g of lactulose, 5 g of mannitol, and 20 g of sucrose in 100 mL of water). During each of periods 2 to 3 hours, 3 to 4 hours, 4 to 5 hours, and 5 to 6 hours after ingestion of the sugar solution, each test subject was instructed to ingest 150 mL or more of water. The whole of urine excreted during the period of 0 to 6 hours after ingestion of water was collected, and the amounts of lactulose and mannitol in the urine were measured by liquid chromatography-tandem mass spectrometry (LC-MS/MS). The LC-MS/MS was performed as follows.

Each measurement sample to be used for the LC-MS/MS was prepared from urine as follows. To 50 $\mu$L of urine, 400 $\mu$L of cooled acetonitrile was added, and the resultant was sufficiently stirred and left to stand on ice for 1 hour. The resulting mixture was further stirred, 800 $\mu$L of 75% by mass acetonitrile was added, the mixture was sufficiently stirred and filtered through a Millex FH 0.45-$\mu$m filter, and the resultant was used as a measurement sample. No internal standard was used.

Measurement by LC-MS/MS and the subsequent data analysis were performed by using a QTRAP 4500 LC-MS/MS system (Thermo Fisher Scientific). An Agilent AdvanceBio MS Spent Media 120 Å 2.1 x 100 mm (Agilent Technologies) was used as a column. The conditions for the measurement by LC-MS/MS were as follows.

Mobile phase A: 10 mM ammonium formate (pH 10.5)
Mobile phase B: acetonitrile (90%)/100 mM ammonium formate (pH 10.5) (10%)
Mobile condition: 0-15 min B 97%-89%, 15-18 min B 89%
Flow: 0.4 mL/min
Injection volume: 5 $\mu$L

Temperature: 35°C
IonSpray Voltage: -4500 V

After measuring the amounts of lactulose and mannitol in urine (hereinafter, referred to as "excretions") by LC-MS/MS, the excretion rates (%) of them were calculated with the following expression:

Excretion rate = excretion ($\mu$mol/mL) $\times$ molecular weight ($\mu$g/$\mu$mol) $\times$ urine volume (mL) / amount of ingested sugar ($\mu$g) $\times$ 100.

After calculating the excretion rates (%), the lactulose/mannitol ratio was calculated with the following expression:

Lactulose/mannitol ratio = lactulose excretion rate/mannitol excretion rate.

Twenty-seven test subjects with lactulose/mannitol ratios of 0.025 or more were included in a positive group, and twenty-seven test subjects with lactulose/mannitol ratios of 0.015 or less were included in a negative group.
Wet feces and serum were obtained from the negative group and the positive group, and used in the following comparative example and example.
Serum was obtained as follows. On the day of the lactulose/mannitol test, 20 mL of blood was collected from each test subject, and serum was separated from part of the blood and stored in a deep freezer (-80°C) until use.
Wet feces were obtained as follows. At any time from 2 days before the lactulose/mannitol test to the day of the test, each test subject was instructed to take an approximately 10-g portion from feces immediately after being excreted in a fecal sampling container (Faeces tube, with blade, screw cap (manufactured by SARSTEDT AG & Co. KG)), and store it in a freezer until submission. On the day of the test, each test subject was instructed to bring and submit the fecal sampling container containing the feces, with the fecal sampling container placed in a cooling container in order to keep the feces frozen. After the submission, the cooling containers were stored in a deep freezer (-80°C) until use.

<<Comparative Example 1>>

For each serum obtained from the negative group and the positive group, serum zonulin concentration was measured by ELISA. The ELISA was performed with a Zonuline ELISA kit (Immundiagnostik AG, Germany) in accordance with a protocol from the manufacturer.
An ROC (Receiver Operating Characteristic) curve was prepared by using the serum zonulin concentrations from the negative group and the positive group, and an area under the ROC curve (AUC: area under the curve) and a cutoff value were determined from the ROC curve prepared. The ROC curve, AUC, and cutoff value were determined by using the statistical analysis software "Excel Statistics" (manufactured by Social Survey Research Information Co., Ltd.). The ROC curve is a curve drawn by plotting the serum zonulin concentrations from the negative group and the positive group with an ordinate representing sensitivity and an abscissa representing (1 - specificity). The AUC is the area under the ROC curve. A point on the ROC curve at which the distance from the upper left corner of the ROC curve was minimized was employed as the cutoff value. Table 15 shows the results.

[Table 15]

Table 15

| Substance Name | Measurement method | AUC | Cutoff value |
|---|---|---|---|
| Zonulin | ELISA | 0.638 | 39.70 |

<<Example 1>>

(1) Metabolomic analysis for wet feces

For each wet feces obtained from the negative group and the positive group, metabolomic analysis was performed to comprehensively measure metabolites in the wet feces. The metabolomic analysis was performed by capillary electrophoresis-Fourier transform mass spectrometry (CE-FTMS) and liquid chromatography-tandem mass spectrometry (LC-MS/MS).

Each measurement sample to be used for CE-FTMS was prepared from wet feces as follows. To 30 to 50 mg of wet feces, 500 $\mu$L of ultrapure water containing an internal standard (20 $\mu$M) was added, and the resultant was vortexed, and centrifuged under centrifugation conditions to cause complete precipitation of feces without any collapse of precipitates. A 350-$\mu$L portion of the supernatant was transferred to an ultrafiltration unit, and subjected to centrifugal filtration (4°C, 9100 $\times$ g, 1 to 2 hours) for protein removal until the solution in the filter cup had almost completely disappeared. The resulting processed wet feces product was preserved at -80°C; when being analyzed, the processed wet feces product was thawed, a 80-$\mu$L portion was then taken therefrom, to which 20 $\mu$L of Milli-Q water was added, and the resultant was stirred and used as a measurement sample. The internal standard used was an internal standard (H3304-1002; HMT) from Human Metabolome Technologies, Inc. (HMT).

Measurement by CE-FTMS and the subsequent data analysis were performed by using an Agilent CE system (Agilent Technologies). Capillary electrophoresis was performed by using a Fused silica capillary. The conditions for the measurement by CE-FTMS were as follows.

[Cationic metabolites (cation mode)]

Run buffer: Cation Buffer Solution (p/n: H 3301-1001)
Rinse buffer: Cation Buffer Solution (p/n: H 3301-1001)
Sample injection: Pressure injection 50 mbar, 10 sec
CE voltage: Positive, 30 kV
MS ionization: ESI Positive
MS capillary voltage: 4,000 V
MS scan range: m/z 60-900
Sheath liquid: HMT Sheath Liquid (p/n: I 3301-1040)

[Anionic metabolites (anion mode)]

Run buffer: Anion Buffer Solution (p/n: H 3302-1023)
Rinse buffer: Anion Buffer Solution (p/n: H 3302-1023)
Sample injection: Pressure injection 50 mbar, 22 sec
CE voltage: Positive, 30 kV
MS ionization: ESI Negative
MS capillary voltage: 3,500 V
MS scan range: m/z 70-1,050
Sheath liquid: HMT Sheath Liquid (p/n: I 3301-1040)

Each measurement sample to be used for LC-MS/MS was prepared from wet feces as follows. Per 100 mg of wet feces, 1600 $\mu$L of cooled methanol containing 0.15% by mass formic acid and 300 $\mu$L of MilliQ water were added, and the resultant was sufficiently stirred and left to stand on ice for 1 hour. The resulting mixture was centrifuged (13000 rpm, 15 min, 4°C), and a 200-$\mu$L portion was taken from the supernatant and brown with nitrogen gas for exsiccation, and the resultant was dissolved in 200 $\mu$L of 10% by mass methanol (containing 0.1% by mass formic acid) for measurement with an octadecylsilyl (ODS) column, or dissolved in 200 $\mu$L of 50% by mass acetonitrile for measurement with a hydrophilic interaction liquid chromatography (HILIC) column. For measurement with an ODS column, a product obtained by filtering through a GL Chromatodisk 4A aqueous 0.45-$\mu$m filter was used as a measurement sample. For measurement with a HILIC column, a product obtained by filtering through a Millex FH 0.45-$\mu$m filter was used as a measurement sample. No internal standard was used. In order to comprehensively analyze metabolites, two columns (ODS column and HILIC column) were used to detect divers substances. The ODS column is more suitable for measurement of hydrophobic substances than the HILIC column, and the HILIC column is more suitable for measurement of hydrophilic substances than the ODS column.

Measurement by LC-MS/MS and the subsequent data analysis were performed by using a Q Exactive Plus Orbitrab LC-MS/MS system (Thermo Fisher Scientific). A Thermo Scientific Hypersil Gold C18 (150 mm L., 2.1 mm I.D., 1.9 $\mu$m) (Thermo Fisher Scientific) was used as an ODS column, and a Merck SeQuant ZIC pHILIC (150 mm L., 2.1 mm I.D., 5 $\mu$m) (Merck Millipore) was used as a HILIC column. The conditions for the measurement by LC-MS/MS were as follows.

[Measurement with ODS column]

Mobile phase A: aqueous solution containing 0.1% by mass formic acid Mobile phase B: methanol containing 0.1% by mass formic acid
Mobile condition: 0-3 min B 0.5%, 3-10 min B 0.5-50%, 10-12 min B 50-98%, 12-32 min B 98%, 32-33 min B 98-0.5%, 33-40 min B 0.5%
Flow: 0-3 min 0.1 mL/min, 3-40 min 0.25 mL/min
Injection volume: 2 $\mu$L
Temperature: 55°C
MS ionization: Swiching mode (ESI Positive, ESI Negative)
MS capillary voltage: 2.5 kV
MS scan range: m/z 70-1050

[Measurement with HILIC column]

Mobile phase A: 10 mM aqueous solution of ammonium acetate (pH 9.6) Mobile phase B: acetonitrile
Mobile condition: 0-3.5 min B 90%, 3.5-18 min B 90-30%, 18-21 min B 30%, 21-22 min B 30-90%, 22-30 min B 90%
Flow: 0.25 mL/min
Injection volume: 2 $\mu$L
Temperature: 20°C
MS ionization: Swiching mode (ESI Positive, ESI Negative)
MS capillary voltage: 2.5 kV
MS scan range: m/z 70-1050

For each wet feces obtained from the negative group and the positive group, peaks derived from different substances were specified from peaks detected in CE-FTMS with reference to the m/Z values for the substances, and the peak areas for the substances were determined. The m/Z values for the substances are shown in Table 16. In determining the peak areas, only peaks with signal/noise (S/N) ratios of 3 or more were extracted from peaks detected in CE-FTMS to determine the peak areas with use of the automatic integration software MasterHands ver. 2.17.5.19 (developed by Keio University). The relative area value (relative concentration) for each substance in each wet feces was determined from the following expression:

Relative area value for substance in wet feces = (peak area value for substance)/((peak area value for internal standard) $\times$ (mass of wet feces)).

Wet feces obtained from the negative group (27 individuals) (measurement samples No. 1 to No. 27) and wet feces obtained from the positive group (27 individuals) (measurement samples No. 28 to No. 54) were sequentially subjected to measurement by LC-MS/MS. At that time, the relative area value of each substance in each wet feces was calculated, with correction of the peak area value for the substance in the measurement sample on the basis of a regression curve prepared from the peak area values for the substances in a mixture (pooled sample) of wet feces obtained from the negative group (27 individuals) (measurement samples No. 1 to No. 27) and wet feces obtained from the positive group (27 individuals) (measurement samples No. 28 to No. 54) by using the Normalize Area node of the small molecule structure identification software Compound Discoverer 3.1 (Thermo Fisher Scientific).
Specifically, in addition to measurement for the measurement samples of No. 1 to No. 54, measurement for the pooled sample was performed every 15 samples, and the subsequent data analysis was then performed to determine the peak area for each substance in the pooled sample in the same manner as described above (i.e., the sequence "measurement for measurement samples of No. 1 to No. 15 -> measurement for pooled sample -> measurement for measurement samples of No. 16 to No. 30 -> measurement for pooled sample ..." was continued until completion of measurement for the sample of No. 54, and the subsequent data analysis was then performed to determine the peak area for each substance in the pooled sample in the same manner as described above). The relative area value of each substance in each wet feces was calculated with correction of the peak area value for the substance in the measurement sample in the same manner as described above.

The correction with use of the pooled sample was carried out for the purpose of correcting errors, for example, caused by the influence of the temporal attenuation of the peak area values for the substances.

An ROC (Receiver Operating Characteristic) curve was prepared with use of the relative area values for each substance in the wet feces, and an area under the ROC curve (AUC: area under the curve) and a cutoff value were determined from the ROC curve prepared. The ROC curve, AUC, and cutoff value were determined by using the statistical analysis software "Excel Statistics" (manufactured by Social Survey Research Information Co., Ltd.). The ROC curve is a curve drawn by plotting the relative area values for a substance in the wet feces with an ordinate representing sensitivity and an abscissa representing (1 - specificity). The AUC is the area under the ROC curve. A point on the ROC curve at which the distance from the upper left corner of the ROC curve was minimized was employed as the cutoff value. Table 16 shows the results. Values of AUC closer to 1 indicate higher accuracy of evaluating intestinal barrier function. Cases with a value of AUC of 0.6 or less can be classified as low evaluation accuracy, cases with a value of AUC of more than 0.6 and 0.7 or less as slightly high evaluation accuracy, cases with a value of AUC of more than 0.7 and 0.8 or less as high evaluation accuracy, and cases with a value of AUC of more than 0.8 as very high evaluation accuracy.

Among the substances comprehensively measured, Table 16 shows results only for substances with AUC of 0.65 or more (the substances of No. 1 to No. 99 in Group B).

For each of the substances of No. 1 to No. 99 in Group B, the positive group average value, the negative group average value, and the ratio of the positive group average value to the negative group average value (positive group average value / negative group average value) were determined by using the relative area values for the substance in the wet feces. Table 17 shows the results. Table 17 also shows tendencies in the positive group. The entry "increase" indicates the presence of a tendency to increase in the positive group, and the entry "decrease" indicates the presence of a tendency to decrease in the positive group.

The results shown in Tables 16 and 17 revealed that in the case of a specimen of wet feces, intestinal barrier function in a subject can be evaluated with high accuracy by using one or more substances selected from Group B as a biomarker. As AUC closer to 1 indicates higher accuracy of evaluating intestinal barrier function, the substances of No. 1 to No. 4 in Group B (formylanthranilic acid, glycerol, formiminoglutamic acid, and 5,6-dihydroxyindole) and the substances of No. 46 to No. 49 in Group B (6-methylquinoline, N1-[4-(1,3-oxazol-5-yl)phenyl]cyclopropane-1-carboxamide, 2-hydroxyquinoline, and 3-methyladipic acid) give particularly high accuracy of evaluating intestinal barrier function.

[Table 16-1]

| Table 16 (Group B) | | | | | |
|---|---|---|---|---|---|
| No. | Substance Name | Measurement method | m/z | AUC | Cutoff value |
| 1 | Formylanthranilic acid | CE-FTMS | 164.04 | 0.8098 | 1.735E-05 |
| 2 | Glycerol | CE-FTMS | 93.05 | 0.7479 | 4.030E-02 |
| 3 | Formiminoglutamic acid | CE-FTMS | 173.06 | 0.7464 | 8.270E-05 |
| 4 | 5,6-Dihydroxyindole | CE-FTMS | 150.05 | 0.7393 | 1.671E-05 |
| 5 | 3-Aminopropane-1,2-diol | CE-FTMS | 92.07 | 0.7108 | 1.000E-07 |
| 6 | 4-Guanidinobutyric acid | CE-FTMS | 146.09 | 0.7094 | 5.009E-04 |
| 7 | 2'-Deoxyadenosine and 5'-Deoxyadenosine | CE-FTMS | 252.11 | 0.7080 | 7.649E-06 |
| 8 | Acetanilide | CE-FTMS | 136.08 | 0.7051 | 1.877E-04 |
| 9 | Pyrophosphate | CE-FTMS | 176.94 | 0.7044 | 3.778E-03 |
| 10 | Myristoleic acid | CE-FTMS | 225.19 | 0.7016 | 2.628E-05 |
| 11 | Diethanolamine | CE-FTMS | 106.09 | 0.7009 | 2.412E-04 |
| 12 | Thiaproline | CE-FTMS | 134.03 | 0.6923 | 6.139E-05 |
| 13 | Hydroxyindole | CE-FTMS | 134.06 | 0.6909 | 4.647E-04 |
| 14 | 2,6-Diaminopimelic acid | CE-FTMS | 191.10 | 0.6880 | 5.705E-04 |
| 15 | p-Aminobenzoic acid | CE-FTMS | 138.06 | 0.6880 | 2.246E-04 |
| 16 | Lipoamide | CE-FTMS | 206.07 | 0.6880 | 1.842E-04 |
| 17 | Phenylhydrazine | CE-FTMS | 109.08 | 0.6838 | 8.537E-06 |
| 18 | 3-Guanidinopropionic acid | CE-FTMS | 132.08 | 0.6823 | 1.128E-06 |

(continued)

| Table 16 (Group B) | | | | | |
|---|---|---|---|---|---|
| No. | Substance Name | Measurement method | m/z | AUC | Cutoff value |
| 19 | N-Acetylaspartic acid | CE-FTMS | 174.04 | 0.6809 | 8.233E-04 |
| 20 | 2,4-Diaminobutyric acid | CE-FTMS | 119.08 | 0.6802 | 3.818E-05 |
| 21 | γ-glutamyl-2-aminobutyric acid | CE-FTMS | 233.11 | 0.6781 | 2.016E-05 |
| 22 | Cysteine | CE-FTMS | 122.03 | 0.6752 | 3.874E-06 |
| 23 | Glycerol 3-phosphate | CE-FTMS | 171.01 | 0.6738 | 4.128E-05 |
| 24 | Glucose 6-phosphate | CE-FTMS | 259.02 | 0.6724 | 2.554E-04 |
| 25 | Mucic acid | CE-FTMS | 209.03 | 0.6724 | 5.846E-05 |

[Table 16-2]

| Table 16 (Continued) | | | | | |
|---|---|---|---|---|---|
| No. | Substance Name | Measurement method | m/z | AUC | Cutoff value |
| 26 | Trigonelline | CE-FTMS | 138.06 | 0.6709 | 7.789E-04 |
| 27 | N-Carbamoylaspartic acid | CE-FTMS | 175.04 | 0.6702 | 7.948E-05 |
| 28 | Threonic acid | CE-FTMS | 135.03 | 0.6695 | 9.191E-05 |
| 29 | Glycocholic acid | CE-FTMS | 464.30 | 0.6681 | 3.977E-06 |
| 30 | Isobutyric acid and Butyric acid | CE-FTMS | 87.05 | 0.6667 | 5.515E-02 |
| 31 | 2-Deoxyribose 1-phosphate | CE-FTMS | 213.02 | 0.6645 | 7.729E-06 |
| 32 | Arginine ethyl ester | CE-FTMS | 203.15 | 0.6638 | 2.085E-06 |
| 33 | N-Methylglutamic acid | CE-FTMS | 162.08 | 0.6624 | 2.906E-04 |
| 34 | Pterin | CE-FTMS | 164.06 | 0.6624 | 8.167E-05 |
| 35 | N-Acetylphenylalanine | CE-FTMS | 206.08 | 0.6603 | 3.633E-05 |
| 36 | Ribulose 5-phosphate | CE-FTMS | 229.01 | 0.6588 | 2.299E-05 |
| 37 | Fructose 6-phosphate | CE-FTMS | 259.02 | 0.6567 | 1.348E-05 |
| 38 | Sedoheptulose 7-phosphate | CE-FTMS | 289.03 | 0.6560 | 3.701E-05 |
| 39 | N-Acetylglucosamine 1-phosphate | CE-FTMS | 300.05 | 0.6538 | 6.011E-06 |
| 40 | Ribose 5-phosphate | CE-FTMS | 229.01 | 0.6481 | 1.212E-05 |
| 41 | 3'-Cytidylic acid and 2'-Cytidylic acid | CE-FTMS | 322.04 | 0.6432 | 1.386E-05 |
| 42 | Dihydroxyacetone phosphate | CE-FTMS | 168.99 | 0.6382 | 2.745E-05 |
| 43 | 4-Oxovaleric acid | CE-FTMS | 115.04 | 0.6346 | 1.000E-07 |
| 44 | Propranolol | CE-FTMS | 260.16 | 0.6311 | 1.802E-06 |
| 45 | 2-Deoxyglucose 6-phosphate | CE-FTMS | 243.03 | 0.6296 | 1.252E-05 |
| 46 | 6-Methylquinoline | LC-MS/MS | 143.07 | 0.7792 | 1.011E+07 |
| 47 | N1-[4-(1,3-Oxazol-5-yl)phenyl]cyclopropane-1-carboxamide | LC-MS/MS | 228.09 | 0.7707 | 1.466E+07 |
| 48 | 2-Hydroxyquinoline | LC-MS/MS | 145.05 | 0.7664 | 2.078E+06 |
| 49 | 3-Methyladipic acid | LC-MS/MS | 160.07 | 0.7564 | 2.792E+07 |

[Table 16-3]

| | Table 16 (Continued) | | | | |
|---|---|---|---|---|---|
| No. | Substance Name | Measurement method | m/z | AUC | Cutoff value |
| 50 | L-threo-3-Phenylserine | LC-MS/MS | 181.07 | 0.7536 | 2.634E+07 |
| 51 | 4-(Diethylamino)salicylaldehyde | LC-MS/MS | 193.11 | 0.7536 | 6.543E+06 |
| 52 | 5-Methoxyindole | LC-MS/MS | 147.07 | 0.7393 | 2.955E+05 |
| 53 | 7-(tert-butyl)-4-imino-1,2,3,4,5,6-hexahydropyrido[3,4-d]pyridazine-1,5-dione | LC-MS/MS | 234.11 | 0.7322 | 6.135E+07 |
| 54 | 4-Indolecarbaldehyde | LC-MS/MS | 145.05 | 0.7236 | 3.257E+07 |
| 55 | Carboxyibuprofen | LC-MS/MS | 236.10 | 0.7236 | 1.239E+05 |
| 56 | Piperonylonitrile | LC-MS/MS | 147.03 | 0.7208 | 8.409E+05 |
| 57 | 5-Hydroxyindole | LC-MS/MS | 133.05 | 0.7194 | 9.655E+06 |
| 58 | Orotic acid | LC-MS/MS | 156.02 | 0.7165 | 1.268E+07 |
| 59 | Glucose 1-phosphate | LC-MS/MS | 260.03 | 0.7123 | 6.790E+06 |
| 60 | L-Alanyl-L-proline | LC-MS/MS | 186.10 | 0.7108 | 2.543E+07 |
| 61 | 2-Acetamido-N-β-aspartyl-2-deoxyhexopyranosylamine | LC-MS/MS | 280.11 | 0.7108 | 2.822E+06 |
| 62 | α-Aspartylphenylalanine | LC-MS/MS | 335.13 | 0.7108 | 1.613E+06 |
| 63 | 6-Hydroxymelatonin | LC-MS/MS | 248.12 | 0.7066 | 1.275E+05 |
| 64 | Xanthosine | LC-MS/MS | 284.08 | 0.7037 | 1.155E+05 |
| 65 | Atenolol | LC-MS/MS | 242.09 | 0.7023 | 1.423E+06 |
| 66 | (R)-Equol | LC-MS/MS | 266.16 | 0.7023 | 2.073E+05 |
| 67 | 2,4-dihydroxyheptadec-16-en-1-yl acetate | LC-MS/MS | 328.26 | 0.7023 | 1.486E+06 |
| 68 | Guanine | LC-MS/MS | 151.05 | 0.6994 | 2.314E+07 |
| 69 | 1-(4-methyl-2-pyridyl)pyrrolidine-2,5-dione | LC-MS/MS | 190.07 | 0.6994 | 1.346E+06 |
| 70 | Laurolactam | LC-MS/MS | 197.18 | 0.6966 | 5.552E+07 |
| 71 | Olmesartan | LC-MS/MS | 234.11 | 0.6966 | 5.376E+05 |
| 72 | Adenine | LC-MS/MS | 135.05 | 0.6966 | 8.854E+06 |
| 73 | Uracil | LC-MS/MS | 112.03 | 0.6952 | 1.479E+07 |

[Table 16-4]

| | Table 16 (Continued) | | | | |
|---|---|---|---|---|---|
| No. | Substance Name | Measurement method | m/z | AUC | Cutoff value |
| 74 | 6-Methyl[1,2,4]triazolo[4,3-b]pyridazin-8-ol | LC-MS/MS | 150.05 | 0.6923 | 2.932E+07 |
| 75 | 1-propyl-1H-benzo[d]imidazole hydrobromide | LC-MS/MS | 160.10 | 0.6880 | 9.064E+05 |
| 76 | Pseudouridine | LC-MS/MS | 244.07 | 0.6880 | 6.790E+06 |
| 77 | D-(-)-Quinic acid | LC-MS/MS | 192.06 | 0.6852 | 1.141E+07 |
| 78 | Acetylcholine | LC-MS/MS | 145.11 | 0.6823 | 3.012E+08 |
| 79 | 3-Isopropylmalic acid | LC-MS/MS | 176.07 | 0.6823 | 4.044E+06 |
| 80 | 2-methoxy-5-(1H-1,2,4-triazol-5-yl)-4-(trifluoromethyl)pyridine | LC-MS/MS | 244.06 | 0.6809 | 1.059E+06 |
| 81 | 3-Amino-2-naphthoic acid | LC-MS/MS | 187.06 | 0.6795 | 8.879E+06 |

(continued)

| Table 16 (Continued) | | | | | |
|---|---|---|---|---|---|
| No. | Substance Name | Measurement method | m/z | AUC | Cutoff value |
| 82 | Ornithine | LC-MS/MS | 132.09 | 0.6795 | 2.176E+07 |
| 83 | 2'-Deoxyguanosine | LC-MS/MS | 267.10 | 0.6781 | 1.690E+07 |
| 84 | Bis(methylbenzylidene)sorbitol | LC-MS/MS | 386.17 | 0.6781 | 6.561E+08 |
| 85 | 2-Furoic acid | LC-MS/MS | 273.14 | 0.6781 | 4.896E+05 |
| 86 | 4-(tert-butyl)phenyl 3,5-dimethylisoxazole-4-carboxylate | LC-MS/MS | 112.02 | 0.6781 | 5.936E+07 |
| 87 | Pyridoxamine | LC-MS/MS | 168.09 | 0.6724 | 3.104E+06 |
| 88 | Tridecylic acid | LC-MS/MS | 214.19 | 0.6695 | 1.284E+08 |
| 89 | 3-Methylhistidine | LC-MS/MS | 169.08 | 0.6667 | 1.741E+07 |
| 90 | Hexanoic acid | LC-MS/MS | 116.08 | 0.6667 | 1.900E+07 |
| 91 | Azobenzene | LC-MS/MS | 182.08 | 0.6624 | 2.955E+07 |
| 92 | (-)-Epigallocatechin | LC-MS/MS | 306.07 | 0.6624 | 3.958E+05 |
| 93 | 3-(2-thienyl)-1,2,4-oxadiazole-5-carbohydrazide | LC-MS/MS | 210.02 | 0.6610 | 3.608E+05 |
| 94 | DL-Lactic Acid | LC-MS/MS | 90.03 | 0.6610 | 6.479E+06 |
| 95 | 6-Hydroxypicolinic acid | LC-MS/MS | 139.03 | 0.6595 | 1.176E+06 |
| 96 | Proline | LC-MS/MS | 368.11 | 0.6595 | 2.874E+07 |

[Table 16-5]

| Table 16 (Continued) | | | | | |
|---|---|---|---|---|---|
| No. | Substance Name | Measurement method | m/z | AUC | Cutoff value |
| 97 | 1,3,5-trihydroxy-4-{[(2E)-3-(3-hydroxy-4-methoxyphenyl)prop-2-enoyl]oxy}cyclohexane-1-carboxylic acid | LC-MS/MS | 115.06 | 0.6595 | 1.792E+06 |
| 98 | Prolylglycine | LC-MS/MS | 172.08 | 0.6581 | 9.604E+06 |
| 99 | 4-[(6E)-3-Hydroxy-8,10-dimethyl-2-(methylamino)-6-dodecen-1-yl]phenol | LC-MS/MS | 333.27 | 0.6581 | 1.918E+05 |

[Table 17-1]

| Table 17 (Group B) | | | | | |
|---|---|---|---|---|---|
| No. | Substance Name | Tendency in positive group | Negative group average value | Positive group average value | Positive group average value / Negative group average value |
| 1 | Formylanthranilic acid | Increase | 1.166E-05 | 3.631E-05 | 3.114 |
| 2 | Glycerol | Decrease | 4.339E-02 | 3.698E-02 | 0.852 |
| 3 | Formiminoglutamic acid | Increase | 1.092E-04 | 1.738E-04 | 1.592 |
| 4 | 5,6-Dihydroxyindole | Increase | 1.526E-05 | 4.859E-05 | 3.184 |
| 5 | 3-Aminopropane-1,2-diol | Decrease | 2.235E-04 | 5.462E-05 | 0.244 |

(continued)

| | Table 17 (Group B) | | | | |
|---|---|---|---|---|---|
| No. | Substance Name | Tendency in positive group | Negative group average value | Positive group average value | Positive group average value / Negative group average value |
| 6 | 4-Guanidinobutyric acid | Decrease | 1.297E-03 | 6.972E-04 | 0.538 |
| 7 | 2'-Deoxyadenosine and 5'-Deoxyadenosine | Increase | 1.214E-05 | 2.925E-05 | 2.409 |
| 8 | Acetanilide | Increase | 1.540E-04 | 2.817E-04 | 1.829 |
| 9 | Pyrophosphate | Increase | 3.552E-03 | 5.058E-03 | 1.424 |
| 10 | Myristoleic acid | Decrease | 6.754E-05 | 2.664E-05 | 0.394 |
| 11 | Diethanolamine | Decrease | 2.779E-04 | 2.009E-04 | 0.723 |
| 12 | Thiaproline | Increase | 6.589E-05 | 1.140E-04 | 1.730 |
| 13 | Hydroxyindole | Increase | 1.528E-03 | 4.040E-03 | 2.644 |
| 14 | 2,6-Diaminopimelic acid | Increase | 6.058E-04 | 9.813E-04 | 1.620 |
| 15 | p-Aminobenzoic acid | Increase | 3.081E-04 | 6.833E-04 | 2.218 |
| 16 | Lipoamide | Decrease | 2.322E-04 | 1.150E-04 | 0.495 |
| 17 | Phenylhydrazine | Decrease | 3.173E-05 | 1.398E-05 | 0.441 |
| 18 | 3-Guanidinopropionic acid | Decrease | 5.590E-06 | 2.459E-06 | 0.440 |
| 19 | N-Acetylaspartic acid | Increase | 1.436E-03 | 2.547E-03 | 1.774 |
| 20 | 2,4-Diaminobutyric acid | Increase | 8.768E-05 | 2.667E-04 | 3.042 |
| 21 | γ-glutamyl-2-aminobutyric acid | Increase | 6.220E-05 | 8.365E-05 | 1.345 |
| 22 | Cysteine | Decrease | 1.011E-05 | 7.501E-06 | 0.742 |
| 23 | Glycerol 3-phosphate | Increase | 8.036E-05 | 1.987E-04 | 2.473 |
| 24 | Glucose 6-phosphate | Increase | 5.733E-04 | 1.825E-03 | 3.183 |

[Table 17-2]

| | Table 17 (Continued) | | | | |
|---|---|---|---|---|---|
| No. | Substance Name | Tendency in positive group | Negative group average value | Positive group average value | Positive group average value / Negative group average value |
| 25 | Mucic acid | Decrease | 7.029E-04 | 1.709E-04 | 0.243 |
| 26 | Trigonelline | Decrease | 1.677E-03 | 1.008E-03 | 0.601 |
| 27 | N-Carbamoylaspartic acid | Increase | 3.778E-05 | 6.837E-05 | 1.810 |
| 28 | Threonic acid | Decrease | 3.905E-04 | 1.096E-04 | 0.281 |
| 29 | Glycocholic acid | Decrease | 9.916E-05 | 5.545E-05 | 0.559 |
| 30 | Isobutyric acid and Butyric acid | Increase | 4.562E-02 | 6.179E-02 | 1.354 |
| 31 | 2-Deoxyribose 1-phosphate | Increase | 2.414E-04 | 6.406E-04 | 2.654 |
| 32 | Arginine ethyl ester | Decrease | 1.159E-05 | 9.635E-06 | 0.831 |
| 33 | N-Methylglutamic acid | Increase | 3.858E-04 | 4.045E-04 | 1.048 |
| 34 | Pterin | Increase | 9.595E-05 | 1.305E-04 | 1.360 |

(continued)

| Table 17 (Continued) | | | | | |
|---|---|---|---|---|---|
| No. | Substance Name | Tendency in positive group | Negative group average value | Positive group average value | Positive group average value / Negative group average value |
| 35 | N-Acetylphenylalanine | Decrease | 8.416E-05 | 4.897E-05 | 0.582 |
| 36 | Ribulose 5-phosphate | Increase | 1.927E-04 | 3.983E-04 | 2.067 |
| 37 | Fructose 6-phosphate | Increase | 1.066E-04 | 3.331E-04 | 3.125 |
| 38 | Sedoheptulose 7-phosphate | Increase | 7.972E-05 | 1.985E-04 | 2.490 |
| 39 | N-Acetylglucosamine 1-phosphate | Increase | 1.214E-05 | 4.534E-05 | 3.735 |
| 40 | Ribose 5-phosphate | Increase | 7.874E-05 | 7.900E-05 | 1.003 |
| 41 | 3'-Cytidylic acid and 2'-Cytidylic acid | Increase | 7.014E-06 | 7.349E-05 | 10.478 |
| 42 | Dihydroxyacetone phosphate | Increase | 5.766E-05 | 1.148E-04 | 1.991 |
| 43 | 4-Oxovaleric acid | Decrease | 4.169E-05 | 0 | 0 |
| 44 | Propranolol | Increase | 8.813E-07 | 5.769E-06 | 6.546 |
| 45 | 2-Deoxyglucose 6-phosphate | Increase | 2.110E-06 | 1.268E-05 | 6.009 |
| 46 | 6-Methylquinoline | Increase | 8.996E+06 | 1.536E+07 | 1.707 |

[Table 17-3]

| Table 17 (Continued) | | | | | |
|---|---|---|---|---|---|
| No. | Substance Name | Tendency in positive group | Negative group average value | Positive group average value | Positive group average value / Negative group average value |
| 47 | N1-[4-(1,3-Oxazol-5-yl)phenyl]cyclopropane-1-carboxamide | Increase | 2.014E+07 | 4.494E+07 | 2.231 |
| 48 | 2-Hydroxyquinoline | Decrease | 7.382E+06 | 4.688E+06 | 0.635 |
| 49 | 3-Methyladipic acid | Decrease | 7.588E+07 | 2.623E+07 | 0.346 |
| 50 | L-threo-3-Phenylserine | Increase | 2.553E+07 | 4.028E+07 | 1.577 |
| 51 | 4-(Diethylamino)salicylaldehyde | Increase | 5.389E+06 | 1.248E+07 | 2.315 |
| 52 | 5-Methoxyindole | Increase | 2.138E+05 | 4.264E+06 | 19.942 |
| 53 | 7-(tert-butyl)-4-imino-1,2,3,4,5,6-hexahydropyrido[3,4-d] pyridazine-1,5-dione | Decrease | 9.444E+07 | 5.411E+07 | 0.573 |
| 54 | 4-Indolecarbaldehyde | Increase | 2.641E+07 | 5.571E+07 | 2.110 |
| 55 | Carboxyibuprofen | Decrease | 1.011E+06 | 1.133E+05 | 0.112 |
| 56 | Piperonylonitrile | Increase | 7.092E+05 | 1.491E+06 | 2.102 |
| 57 | 5-Hydroxyindole | Increase | 4.596E+07 | 1.309E+08 | 2.848 |
| 58 | Orotic acid | Increase | 1.881E+07 | 3.340E+07 | 1.776 |
| 59 | Glucose 1-phosphate | Increase | 6.996E+06 | 1.829E+07 | 2.614 |
| 60 | L-Alanyl-L-proline | Increase | 2.565E+07 | 3.264E+07 | 1.272 |
| 61 | 2-Acetamido-N-β-aspartyl-2-deoxyhexopyranosylamine | Decrease | 4.181E+06 | 2.656E+06 | 0.635 |

(continued)

| Table 17 (Continued) | | | | | |
|---|---|---|---|---|---|
| No. | Substance Name | Tendency in positive group | Negative group average value | Positive group average value | Positive group average value / Negative group average value |
| 62 | α-Aspartylphenylalanine | Decrease | 2.812E+06 | 1.680E+06 | 0.598 |
| 63 | 6-Hydroxymelatonin | Increase | 9.732E+04 | 3.440E+06 | 35.350 |
| 64 | Xanthosine | Increase | 3.095E+05 | 9.238E+05 | 2.984 |
| 65 | Atenolol | Decrease | 4.838E+07 | 2.190E+07 | 0.453 |
| 66 | (R)-Equol | Increase | 2.245E+06 | 8.580E+06 | 3.821 |

[Table 17-4]

| Table 17 (Continued) | | | | | |
|---|---|---|---|---|---|
| No. | Substance Name | Tendency in positive group | Negative group average value | Positive group average value | Positive group average value / Negative group average value |
| 67 | 2,4-dihydroxyheptadec-16-en-1-yl acetate | Decrease | 3.964E+06 | 1.793E+06 | 0.452 |
| 68 | Guanine | Decrease | 1.042E+08 | 4.544E+07 | 0.436 |
| 69 | 1-(4-methyl-2-pyridyl)pyrrolidine-2,5-dione | Increase | 4.240E+06 | 6.583E+06 | 1.553 |
| 70 | Laurolactam | Decrease | 6.163E+07 | 5.108E+07 | 0.829 |
| 71 | Olmesartan | Increase | 5.838E+05 | 2.751E+07 | 47.127 |
| 72 | Adenine | Decrease | 2.584E+07 | 5.780E+06 | 0.224 |
| 73 | Uracil | Increase | 2.327E+07 | 4.243E+07 | 1.824 |
| 74 | 6-Methyl[1,2,4]triazolo[4,3-b]pyridazin-8-ol | Increase | 3.408E+07 | 5.174E+07 | 1.518 |
| 75 | 1-propyl-1H-benzo[d]imidazole hydrobromide | Increase | 9.863E+05 | 3.732E+06 | 3.784 |
| 76 | Pseudouridine | Increase | 7.479E+06 | 1.108E+07 | 1.481 |
| 77 | D-(-)-Quinic acid | Decrease | 1.539E+08 | 3.384E+07 | 0.220 |
| 78 | Acetylcholine | Increase | 6.539E+08 | 2.006E+09 | 3.068 |
| 79 | 3-Isopropylmalic acid | Decrease | 1.064E+07 | 6.056E+06 | 0.569 |
| 80 | 2-methoxy-5-(1H-1,2,4-triazol-5-yl)-4-(trifluoromethyl)pyridine | Increase | 9.450E+05 | 2.028E+06 | 2.145 |
| 81 | 3-Amino-2-naphthoic acid | Increase | 7.145E+06 | 9.919E+06 | 1.388 |
| 82 | Ornithine | Increase | 2.572E+07 | 3.444E+07 | 1.339 |
| 83 | 2'-Deoxyguanosine | Decrease | 5.876E+07 | 2.613E+07 | 0.445 |
| 84 | Bis(methylbenzylidene)sorbitol | Decrease | 7.204E+08 | 5.835E+08 | 0.810 |
| 85 | 2-Furoic acid | Decrease | 7.357E+05 | 4.687E+05 | 0.637 |

(continued)

| | No. | Substance Name | Tendency in positive group | Negative group average value | Positive group average value | Positive group average value / Negative group average value |
|---|---|---|---|---|---|---|
| **Table 17 (Continued)** | | | | | | |
| | 86 | 4-(tert-butyl)phenyl 3,5-di-methylisoxazole-4-carboxy-late | Decrease | 8.780E+07 | 5.211E+07 | 0.593 |
| | 87 | Pyridoxamine | Increase | 5.927E+06 | 1.363E+07 | 2.300 |

[Table 17-5]

| | No. | Substance Name | Tendency in positive group | Negative group average value | Positive group average value | Positive group average value / Negative group average value |
|---|---|---|---|---|---|---|
| **Table 17 (Continued)** | | | | | | |
| | 88 | Tridecylic acid | Increase | 1.062E+08 | 1.845E+08 | 1.736 |
| | 89 | 3-Methylhistidine | Decrease | 7.156E+07 | 3.129E+07 | 0.437 |
| | 90 | Hexanoic acid | Increase | 6.679E+07 | 1.407E+08 | 2.106 |
| | 91 | Azobenzene | Increase | 2.718E+07 | 3.916E+07 | 1.440 |
| | 92 | (-)-Epigallocatechin | Decrease | 4.071E+06 | 1.006E+06 | 0.247 |
| | 93 | 3-(2-thienyl)-1,2,4-oxadiazole-5-carbohydrazide | Increase | 4.068E+05 | 1.115E+06 | 2.740 |
| | 94 | DL-Lactic Acid | Increase | 1.084E+07 | 1.594E+07 | 1.471 |
| | 95 | 6-Hydroxypicolinic acid | Decrease | 2.636E+06 | 2.016E+06 | 0.765 |
| | 96 | Proline | Decrease | 8.797E+07 | 3.716E+07 | 0.422 |
| | 97 | 1,3,5-trihydroxy-4-{[(2E)-3-(3-hydroxy-4-methoxyphenyl)prop-2-enoyl]oxy} cyclohexane-1-carboxylic acid | Decrease | 4.351E+06 | 3.475E+06 | 0.799 |
| | 98 | Prolylglycine | Increase | 1.192E+07 | 2.282E+07 | 1.914 |
| | 99 | 4-[(6E)-3-Hydroxy-8,10-dimethyl-2-(methylamino)-6-dodecen-1-yl] phenol | Decrease | 1.527E+06 | 9.092E+05 | 0.595 |

(2) Metabolomic analysis for dry feces

Wet feces obtained from the negative group and the positive group were aliquoted into an approximately 1-g portions in 25-mL centrifuge tubes, and the masses before drying were measured. The wet feces were dried for 2 days by using a freeze-dryer (RFS2000 manufactured by Asahi Kagaku K. K.), and the masses after drying were measured. The moisture contents of the wet feces were calculated from the masses before and after drying, and the solid content fraction of each wet feces was calculated from the moisture content of the wet feces. The relative area value for each substance in the wet feces, wherein the relative area value had been obtained with the wet feces in (1) above, was divided by the solid content fraction of the wet feces, and the resultant was used as the relative area value for the substance in the corresponding dry feces. Note that values for any biomarker in dry feces as given by converting values for the biomarker in wet feces and those given through measurement by subjecting dried feces to analysis are not significantly different.

An ROC (Receiver Operating Characteristic) curve was prepared with use of the relative area values for each substance in the dry feces, and an area under the ROC curve (AUC: area under the curve) and a cutoff value were determined from the ROC curve prepared. The ROC curve, AUC, and cutoff value were determined by using the statistical analysis software "Excel Statistics" (manufactured by Social Survey Research Information Co., Ltd.). The ROC curve is a curve drawn by plotting the relative area values for a substance in the dry feces with an ordinate representing sensitivity and an abscissa representing (1 - specificity). The AUC is the area under the ROC curve. A point on the ROC curve at which the distance from the upper left corner of the ROC curve was minimized was employed as the cutoff value. Table 18 shows the results. Values of AUC closer to 1 indicate higher accuracy of evaluating intestinal barrier function. Cases with a value of AUC of 0.6 or less can be classified as low evaluation accuracy, cases with a value of AUC of more than 0.6 and 0.7 or less as slightly high evaluation accuracy, cases with a value of AUC of more than 0.7 and 0.8 or less as high evaluation accuracy, and cases with a value of AUC of more than 0.8 as very high evaluation accuracy.

Among the substances comprehensively measured, Table 18 shows results only for substances with AUC of 0.65 or more (the substances of No. 1 to No. 128 in Group A).

For each of the substances of No. 1 to No. 128 in Group A, the positive group average value, the negative group average value, and the ratio of the positive group average value to the negative group average value (positive group average value / negative group average value) were determined by using the relative area values for the substance in the dry feces. Table 19 shows the results. Table 19 also shows tendencies in the positive group. The entry "increase" indicates the presence of a tendency to increase in the positive group, and the entry "decrease" indicates the presence of a tendency to decrease in the positive group.

The results shown in Tables 18 and 19 revealed that in the case of a specimen of dry feces, intestinal barrier function in a subject can be evaluated with high accuracy by using one or more substances selected from Group A as a biomarker. As AUC closer to 1 indicates higher accuracy of evaluating intestinal barrier function, the substances of No. 1 to No. 4 in Group A (formylanthranilic acid, diethanolamine, glycerol, and 5,6-dihydroxyindole) and the substances of No. 63 to No. 66 in Group A (3-methyladipic acid, 2-hydroxyquinoline, 7-(tert-butyl)-4-imino-1,2,3,4,5,6-hexahydropyrido[3,4-d] pyridazine-1,5-dione, and 2,4-dihydroxyheptadec-16-en-1-yl acetate) give particularly high accuracy of evaluating intestinal barrier function.

[Table 18-1]

| Table 18 (Group A) | | | | | |
|---|---|---|---|---|---|
| No. | Substance Name | Measurement method | m/z | AUC | Cutoff value |
| 1 | Formylanthranilic acid | CE-FTMS | 164.04 | 0.8084 | 6.987E-05 |
| 2 | Diethanolamine | CE-FTMS | 106.09 | 0.7521 | 9.720E-04 |
| 3 | Glycerol | CE-FTMS | 93.05 | 0.7479 | 1.648E-01 |
| 4 | 5,6-Dihydroxyindole | CE-FTMS | 150.05 | 0.7379 | 4.685E-05 |
| 5 | Formiminoglutamic acid | CE-FTMS | 173.06 | 0.7350 | 3.424E-04 |
| 6 | Leucine | CE-FTMS | 132.10 | 0.7179 | 8.990E-01 |
| 7 | Myristoleic acid | CE-FTMS | 225.19 | 0.7172 | 4.907E-05 |
| 8 | 3-Aminopropane-1,2-diol | CE-FTMS | 92.07 | 0.7151 | 7.083E-04 |
| 9 | 4-Guanidinobutyric acid | CE-FTMS | 146.09 | 0.7137 | 2.246E-03 |
| 10 | Phenlyalanine | CE-FTMS | 166.09 | 0.7108 | 6.270E-01 |
| 11 | 2'-Deoxyadenosine and 5'-Deoxyadenosine | CE-FTMS | 252.11 | 0.7094 | 3.558E-05 |
| 12 | Lipoamide | CE-FTMS | 206.07 | 0.7094 | 7.370E-04 |
| 13 | 3-Guanidinopropionic acid | CE-FTMS | 132.08 | 0.7066 | 4.111E-06 |
| 14 | Acetanilide | CE-FTMS | 136.08 | 0.7037 | 8.770E-04 |
| 15 | Histidine | CE-FTMS | 156.08 | 0.7037 | 1.802E-01 |
| 16 | γ-glutamyl-2-aminobutyric acid | CE-FTMS | 233.11 | 0.6994 | 1.009E-04 |
| 17 | Methionine sulfoxide | CE-FTMS | 166.05 | 0.6966 | 2.533E-02 |
| 18 | Phenylhydrazine | CE-FTMS | 109.08 | 0.6966 | 5.254E-05 |
| 19 | Tryptophan | CE-FTMS | 205.10 | 0.6923 | 9.222E-02 |
| 20 | Tyrosine | CE-FTMS | 182.08 | 0.6909 | 4.119E-01 |
| 21 | Hydroxyindole | CE-FTMS | 134.06 | 0.6909 | 3.308E-03 |

(continued)

| Table 18 (Group A) | | | | | |
|---|---|---|---|---|---|
| No. | Substance Name | Measurement method | m/z | AUC | Cutoff value |
| 22 | Alanine | CE-FTMS | 90.05 | 0.6909 | 2.476E-01 |
| 23 | p-Aminobenzoic acid | CE-FTMS | 138.06 | 0.6895 | 7.844E-04 |
| 24 | Penicillamine | CE-FTMS | 150.06 | 0.6880 | 2.272E-04 |

[Table 18-2]

| Table 18 (Continued) | | | | | |
|---|---|---|---|---|---|
| No. | Substance Name | Measurement method | m/z | AUC | Cutoff value |
| 25 | 2-Cyanopyridine | CE-FTMS | 105.04 | 0.6845 | 1.293E-03 |
| 26 | Glutamine | CE-FTMS | 147.08 | 0.6838 | 1.503E-01 |
| 27 | 2,4-Diaminobutyric acid | CE-FTMS | 119.08 | 0.6830 | 2.029E-04 |
| 28 | Trigonelline | CE-FTMS | 138.06 | 0.6823 | 4.334E-03 |
| 29 | 2-Methylserine | CE-FTMS | 120.07 | 0.6809 | 7.132E-04 |
| 30 | Glycylglycine | CE-FTMS | 133.06 | 0.6795 | 2.770E-03 |
| 31 | N,N-Dimethylhistidine | CE-FTMS | 184.11 | 0.6781 | 6.870E-04 |
| 32 | Valine | CE-FTMS | 118.09 | 0.6766 | 4.135E-01 |
| 33 | N-Acetylphenylalanine | CE-FTMS | 206.08 | 0.6759 | 1.282E-04 |
| 34 | Isoleucine | CE-FTMS | 132.10 | 0.6709 | 5.148E-01 |
| 35 | Methionine | CE-FTMS | 150.06 | 0.6709 | 2.639E-01 |
| 36 | γ-glutamyl-valyl-glycine | CE-FTMS | 304.15 | 0.6695 | 2.608E-04 |
| 37 | N-Acetylaspartic acid | CE-FTMS | 174.04 | 0.6681 | 4.128E-03 |
| 38 | Threonic acid | CE-FTMS | 135.03 | 0.6681 | 3.224E-04 |
| 39 | Alloisoleucine | CE-FTMS | 132.10 | 0.6681 | 4.463E-03 |
| 40 | Arginine ethyl ester | CE-FTMS | 203.15 | 0.6667 | 1.137E-05 |
| 41 | 2,6-Diaminopimelic acid | CE-FTMS | 191.10 | 0.6667 | 2.517E-03 |
| 42 | Glucose 6-phosphate | CE-FTMS | 259.02 | 0.6667 | 1.816E-03 |
| 43 | Adenosine | CE-FTMS | 268.10 | 0.6638 | 6.549E-04 |
| 44 | Mucic acid | CE-FTMS | 209.03 | 0.6638 | 2.589E-04 |
| 45 | Thymidine | CE-FTMS | 243.10 | 0.6624 | 1.176E-02 |
| 46 | Glycocholic acid | CE-FTMS | 464.30 | 0.6624 | 1.449E-05 |
| 47 | 2-Deoxyribose 1-phosphate | CE-FTMS | 213.02 | 0.6617 | 2.548E-05 |

[Table 18-3]

| Table 18 (Continued) | | | | | |
|---|---|---|---|---|---|
| No. | Substance Name | Measurement method | m/z | AUC | Cutoff value |
| 48 | Isoleucyl-prolyl-proline | CE-FTMS | 326.21 | 0.6595 | 8.785E-04 |
| 49 | Sedoheptulose 7-phosphate | CE-FTMS | 289.03 | 0.6588 | 9.164E-05 |
| 50 | Fumaric acid | CE-FTMS | 115.00 | 0.6581 | 3.557E-03 |
| 51 | N-Acetylmethionine | CE-FTMS | 190.05 | 0.6581 | 3.276E-04 |
| 52 | Fructose 6-phosphate | CE-FTMS | 259.02 | 0.6581 | 5.306E-05 |

(continued)

| Table 18 (Continued) | | | | | |
|---|---|---|---|---|---|
| No. | Substance Name | Measurement method | m/z | AUC | Cutoff value |
| 53 | Glutamylglutamic acid | CE-FTMS | 277.10 | 0.6581 | 7.586E-03 |
| 54 | Cysteine | CE-FTMS | 122.03 | 0.6524 | 9.255E-06 |
| 55 | N-Acetylglucosamine 1-phosphate | CE-FTMS | 300.05 | 0.6510 | 2.191E-05 |
| 56 | Ribose 5-phosphate | CE-FTMS | 229.01 | 0.6496 | 2.926E-05 |
| 57 | Glucose 1-phosphate | CE-FTMS | 259.02 | 0.6467 | 2.341E-04 |
| 58 | 3'-Cytidylic acid and 2'-Cytidylic acid | CE-FTMS | 322.04 | 0.6417 | 2.928E-05 |
| 59 | Dihydroxyacetone phosphate | CE-FTMS | 168.99 | 0.6396 | 1.350E-04 |
| 60 | 4-Oxovaleric acid | CE-FTMS | 115.04 | 0.6346 | 1.000E-07 |
| 61 | 2-Deoxyglucose 6-phosphate | CE-FTMS | 243.03 | 0.6282 | 3.123E-05 |
| 62 | Glucosamine 6-phosphate | CE-FTMS | 258.04 | 0.6083 | 3.708E-05 |
| 63 | 3-Methyladipic acid | LC-MS/MS | 160.07 | 0.7849 | 1.104E+08 |
| 64 | 2-Hydroxyquinoline | LC-MS/MS | 145.05 | 0.7792 | 9.537E+06 |
| 65 | 7-(tert-butyl)-4-imino-1,2,3,4,5,6-hexahydropyrido[3,4-d]pyridazine-1,5-dione) | LC-MS/MS | 234.11 | 0.7578 | 3.046E+08 |
| 66 | 2,4-dihydroxyheptadec-16-en-1-yl acetate | LC-MS/MS | 328.26 | 0.7422 | 5.155E+06 |
| 67 | $\alpha$-Aspartylphenylalanine | LC-MS/MS | 280.11 | 0.7350 | 7.367E+06 |
| 68 | N1-[4-(1,3-Oxazol-5-yl)phenyl]cyclopropane-1-carboxamide | LC-MS/MS | 228.09 | 0.7336 | 6.524E+07 |

[Table 18-4]

| Table 18 (Continued) | | | | | |
|---|---|---|---|---|---|
| No. | Substance Name | Measurement method | m/z | AUC | Cutoff value |
| 69 | 1-(2,4-Dihydroxyquinolin-3-yl)ethan-1-one | LC-MS/MS | 203.06 | 0.7279 | 2.399E+06 |
| 70 | 4-(Diethylamino)salicylaldehyde | LC-MS/MS | 193.11 | 0.7265 | 2.603E+07 |
| 71 | Atenolol | LC-MS/MS | 266.16 | 0.7265 | 6.795E+06 |
| 72 | 5-Hydroxyindole | LC-MS/MS | 133.05 | 0.7222 | 4.262E+07 |
| 73 | 6-Methylquinoline | LC-MS/MS | 143.07 | 0.7208 | 4.392E+07 |
| 74 | Guanine | LC-MS/MS | 151.05 | 0.7179 | 1.526E+08 |
| 75 | Carboxyibuprofen | LC-MS/MS | 236.10 | 0.7179 | 5.205E+05 |
| 76 | 3-Isopropylmalic acid | LC-MS/MS | 176.07 | 0.7151 | 2.205E+07 |
| 77 | Bis(methylbenzylidene)sorbitol | LC-MS/MS | 386.17 | 0.7123 | 2.528E+09 |
| 78 | 2-Furoic acid | LC-MS/MS | 112.02 | 0.7123 | 1.689E+06 |
| 79 | Orotic acid | LC-MS/MS | 156.02 | 0.7108 | 3.783E+07 |
| 80 | 2-Acetamido-N-$\beta$-aspartyl-2-deoxyhexopyra-nosylamine | LC-MS/MS | 335.13 | 0.7094 | 1.084E+07 |
| 81 | Xanthosine | LC-MS/MS | 284.08 | 0.7094 | 3.882E+05 |
| 82 | 1-propyl-1H-benzo[d]imidazole hydrobromide | LC-MS/MS | 160.10 | 0.7080 | 4.383E+06 |
| 83 | 4-Phenolsulfonic acid | LC-MS/MS | 174.00 | 0.7066 | 6.123E+06 |
| 84 | Xanthohumol | LC-MS/MS | 354.15 | 0.7066 | 3.905E+06 |

(continued)

| Table 18 (Continued) | | | | | |
|---|---|---|---|---|---|
| No. | Substance Name | Measurement method | m/z | AUC | Cutoff value |
| 85 | Laurolactam | LC-MS/MS | 135.05 | 0.7023 | 2.352E+08 |
| 86 | Adenine | LC-MS/MS | 197.18 | 0.7023 | 2.938E+07 |
| 87 | 2'-Deoxyguanosine | LC-MS/MS | 267.10 | 0.6994 | 8.778E+07 |
| 88 | 6-Hydroxypicolinic acid | LC-MS/MS | 139.03 | 0.6980 | 4.728E+06 |
| 89 | D-(-)-Quinic acid | LC-MS/MS | 192.06 | 0.6952 | 1.695E+07 |
| 90 | 4-{[(4,6-Dimethoxypyrimidin-2-yl)amino] methylidene}-2-phenyl-4,5-dihydro-1,3-oxazol-5-one | LC-MS/MS | 333.27 | 0.6923 | 6.411E+05 |

[Table 18-5]

| Table 18 (Continued) | | | | | |
|---|---|---|---|---|---|
| No. | Substance Name | Measurement method | m/z | AUC | Cutoff value |
| 91 | 4-[(6E)-3-Hydroxy-8,10-dimethyl-2-(methylamino)-6-dodecen-1-yl]phenol | LC-MS/MS | 326.10 | 0.6923 | 8.139E+05 |
| 92 | Ethyl 2-cyano-3-(tetrahydro-3-thiophenylamino)acrylate | LC-MS/MS | 226.08 | 0.6909 | 7.574E+08 |
| 93 | 3-Succinoylpyridine | LC-MS/MS | 179.06 | 0.6909 | 4.654E+06 |
| 94 | Proline | LC-MS/MS | 115.06 | 0.6895 | 1.313E+08 |
| 95 | L-threo-3-Phenylserine | LC-MS/MS | 181.07 | 0.6895 | 1.365E+08 |
| 96 | (2,4-Diamino-5-pyrimidinyl)(3,4,5-trimethoxyphenyl)methanone | LC-MS/MS | 304.12 | 0.6895 | 1.675E+06 |
| 97 | 3-Morpholino-4-tetrahydro-1H-pyrrol-1-ylcyclobut-3-ene-1,2-dione | LC-MS/MS | 236.12 | 0.6880 | 1.917E+07 |
| 98 | 16-Hydroxyhexadecanoic acid | LC-MS/MS | 157.11 | 0.6880 | 2.998E+09 |
| 99 | Syringic acid | LC-MS/MS | 272.24 | 0.6880 | 8.081E+06 |
| 100 | Tranexamic acid | LC-MS/MS | 198.05 | 0.6880 | 6.722E+06 |
| 101 | 4-(tert-butyl)phenyl 3,5-dimethylisoxazole-4-carboxylate | LC-MS/MS | 273.14 | 0.6852 | 1.687E+08 |
| 102 | 3-Methylhistidine | LC-MS/MS | 169.08 | 0.6852 | 1.411E+08 |
| 103 | 5-Methoxyindole | LC-MS/MS | 147.07 | 0.6823 | 1.077E+06 |
| 104 | Perillic acid | LC-MS/MS | 166.10 | 0.6823 | 9.982E+05 |
| 105 | 6-Methyl[1,2,4]triazolo[4,3-b]pyridazin-8-ol | LC-MS/MS | 150.05 | 0.6809 | 1.148E+08 |
| 106 | Piperonylonitrile | LC-MS/MS | 147.03 | 0.6795 | 3.335E+06 |
| 107 | N 1-(5-Chloro-2-hydroxyphenyl)-2-(morpholinomethyl)benzamide | LC-MS/MS | 346.11 | 0.6795 | 2.392E+05 |
| 108 | N-Methylhistamine | LC-MS/MS | 125.10 | 0.6795 | 1.679E+06 |
| 109 | Dibutylone | LC-MS/MS | 235.12 | 0.6781 | 1.569E+07 |
| 110 | (-)-Epigallocatechin | LC-MS/MS | 306.07 | 0.6781 | 1.933E+06 |
| 111 | Myriocin | LC-MS/MS | 401.28 | 0.6766 | 4.618E+05 |

[Table 18-6]

| Table 18 (Continued) | | | | | |
|---|---|---|---|---|---|
| No. | Substance Name | Measurement method | m/z | AUC | Cutoff value |
| 112 | 4-Indolecarbaldehyde | LC-MS/MS | 145.05 | 0.6752 | 1.631E+08 |
| 113 | Uracil | LC-MS/MS | 128.06 | 0.6738 | 6.381E+07 |
| 114 | Dihydrothymine | LC-MS/MS | 112.03 | 0.6738 | 2.718E+06 |
| 115 | 3-Methylglutaric acid | LC-MS/MS | 146.06 | 0.6738 | 4.365E+08 |
| 116 | 1,5-Anhydro-D-glucitol | LC-MS/MS | 164.07 | 0.6724 | 6.253E+07 |
| 117 | Valethamate | LC-MS/MS | 305.24 | 0.6709 | 5.118E+05 |
| 118 | DL-Arginine | LC-MS/MS | 174.11 | 0.6709 | 1.513E+08 |
| 119 | (R)-Equol | LC-MS/MS | 242.09 | 0.6695 | 7.915E+05 |
| 120 | Theobromine | LC-MS/MS | 180.06 | 0.6681 | 5.198E+07 |
| 121 | 1,5-Isoquinolinediol | LC-MS/MS | 161.05 | 0.6667 | 3.424E+06 |
| 122 | 12-Hydroxydodecanoic acid | LC-MS/MS | 216.17 | 0.6667 | 7.949E+07 |
| 123 | 1,3,5-trihydroxy-4-{[(2E)-3-(3-hydroxy-4-methoxyphenyl)prop-2-enoyl]oxy}cyclohexane-1-carboxylic acid | LC-MS/MS | 192.06 | 0.6610 | 8.471E+06 |
| 124 | 3-Hydroxypicolinic acid | LC-MS/MS | 139.03 | 0.6595 | 2.493E+07 |
| 125 | Theophylline | LC-MS/MS | 138.03 | 0.6595 | 1.072E+08 |
| 126 | 3,4-Dihydroxybenzaldehyde | LC-MS/MS | 180.06 | 0.6595 | 2.044E+06 |
| 127 | Acetylcholine | LC-MS/MS | 146.07 | 0.6595 | 1.184E+09 |
| 128 | Tridecylic acid | LC-MS/MS | 145.11 | 0.6595 | 4.559E+08 |

[Table 19-1]

| Table 19 (Group A) | | | | | |
|---|---|---|---|---|---|
| No. | Substance Name | Tendency in positive group | Negative group average value | Positive group average value | Positive group average value / Negative group average value |
| 1 | Formylanthranilic acid | Increase | 4.906E-05 | 1.391E-04 | 2.835 |
| 2 | Diethanolamine | Decrease | 1.193E-03 | 7.829E-04 | 0.656 |
| 3 | Glycerol | Decrease | 1.929E-01 | 1.454E-01 | 0.754 |
| 4 | 5,6-Dihydroxyindole | Increase | 6.777E-05 | 2.062E-04 | 3.043 |
| 5 | Formiminoglutamic acid | Increase | 4.418E-04 | 6.176E-04 | 1.398 |
| 6 | Leucine | Decrease | 1.040E+00 | 7.216E-01 | 0.694 |
| 7 | Myristoleic acid | Decrease | 2.651E-04 | 1.181E-04 | 0.446 |
| 8 | 3-Aminopropane-1,2-diol | Decrease | 1.062E-03 | 2.474E-04 | 0.233 |
| 9 | 4-Guanidinobutyric acid | Decrease | 6.164E-03 | 2.795E-03 | 0.454 |
| 10 | Phenlyalanine | Decrease | 8.162E-01 | 5.119E-01 | 0.627 |
| 11 | 2'-Deoxyadenosine and 5'-Deoxyadenosine | Increase | 4.400E-05 | 9.758E-05 | 2.218 |
| 12 | Lipoamide | Decrease | 1.079E-03 | 4.637E-04 | 0.430 |

(continued)

| | Table 19 (Group A) | | | | | |
|---|---|---|---|---|---|---|
| No. | Substance Name | Tendency in positive group | Negative group average value | Positive group average value | Positive group average value / Negative group average value |
| 13 | 3-Guanidinopropionic acid | Decrease | 2.738E-05 | 9.713E-06 | 0.355 |
| 14 | Acetanilide | Increase | 5.772E-04 | 9.996E-04 | 1.732 |
| 15 | Histidine | Decrease | 1.688E-01 | 1.071E-01 | 0.634 |
| 16 | $\gamma$-glutamyl-2-aminobutyric acid | Increase | 3.037E-04 | 3.807E-04 | 1.254 |
| 17 | Methionine sulfoxide | Decrease | 3.334E-02 | 2.495E-02 | 0.748 |
| 18 | Phenylhydrazine | Decrease | 1.371E-04 | 5.492E-05 | 0.401 |
| 19 | Tryptophan | Decrease | 1.105E-01 | 8.719E-02 | 0.789 |
| 20 | Tyrosine | Decrease | 4.294E-01 | 2.875E-01 | 0.670 |
| 21 | Hydroxyindole | Increase | 5.235E-03 | 1.333E-02 | 2.546 |
| 22 | Alanine | Decrease | 3.573E-01 | 2.803E-01 | 0.784 |
| 23 | p-Aminobenzoic acid | Increase | 1.111E-03 | 2.076E-03 | 1.868 |

[Table 19-2]

| | Table 19 (Continued) | | | | |
|---|---|---|---|---|---|
| No. | Substance Name | Tendency in positive group | Negative group average value | Positive group average value | Positive group average value / Negative group average value |
| 24 | Penicillamine | Decrease | 2.629E-04 | 1.924E-04 | 0.732 |
| 25 | 2-Cyanopyridine | Decrease | 2.344E-03 | 1.181E-03 | 0.504 |
| 26 | Glutamine | Decrease | 1.568E-01 | 1.065E-01 | 0.679 |
| 27 | 2,4-Diaminobutyric acid | Increase | 3.027E-04 | 1.032E-03 | 3.410 |
| 28 | Trigonelline | Decrease | 7.771E-03 | 4.232E-03 | 0.545 |
| 29 | 2-Methylserine | Decrease | 2.685E-03 | 1.139E-03 | 0.424 |
| 30 | Glycylglycine | Decrease | 3.805E-03 | 2.470E-03 | 0.649 |
| 31 | N,N-Dimethylhistidine | Decrease | 8.202E-04 | 4.735E-04 | 0.577 |
| 32 | Valine | Decrease | 5.893E-01 | 4.009E-01 | 0.680 |
| 33 | N-Acetylphenylalanine | Decrease | 3.860E-04 | 2.327E-04 | 0.603 |
| 34 | Isoleucine | Decrease | 6.136E-01 | 4.455E-01 | 0.726 |
| 35 | Methionine | Decrease | 2.681E-01 | 1.872E-01 | 0.698 |
| 36 | $\gamma$-glutamyl-valyl-glycine | Decrease | 7.954E-04 | 4.639E-04 | 0.583 |
| 37 | N-Acetylaspartic acid | Increase | 6.882E-03 | 9.226E-03 | 1.341 |
| 38 | Threonic acid | Decrease | 2.182E-03 | 5.030E-04 | 0.231 |
| 39 | Alloisoleucine | Decrease | 1.132E-02 | 8.788E-03 | 0.776 |
| 40 | Arginine ethyl ester | Decrease | 5.356E-05 | 3.574E-05 | 0.667 |
| 41 | 2,6-Diaminopimelic acid | Increase | 2.524E-03 | 3.621E-03 | 1.435 |

(continued)

| Table 19 (Continued) | | | | | |
|---|---|---|---|---|---|
| No. | Substance Name | Tendency in positive group | Negative group average value | Positive group average value | Positive group average value / Negative group average value |
| 42 | Glucose 6-phosphate | Increase | 1.899E-03 | 8.136E-03 | 4.284 |
| 43 | Adenosine | Decrease | 1.548E-03 | 7.778E-04 | 0.502 |
| 44 | Mucic acid | Decrease | 3.781E-03 | 7.899E-04 | 0.209 |
| 45 | Thymidine | Decrease | 1.343E-02 | 9.900E-03 | 0.737 |

[Table 19-3]

| Table 19 (Continued) | | | | | |
|---|---|---|---|---|---|
| No. | Substance Name | Tendency in positive group | Negative group average value | Positive group average value | Positive group average value / Negative group average value |
| 46 | Glycocholic acid | Decrease | 5.183E-04 | 2.866E-04 | 0.553 |
| 47 | 2-Deoxyribose 1-phosphate | Increase | 8.700E-04 | 1.997E-03 | 2.296 |
| 48 | Isoleucyl-prolyl-proline | Decrease | 1.085E-03 | 7.296E-04 | 0.672 |
| 49 | Sedoheptulose 7-phosphate | Increase | 2.621E-04 | 8.621E-04 | 3.289 |
| 50 | Fumaric acid | Decrease | 5.315E-03 | 3.120E-03 | 0.587 |
| 51 | N-Acetylmethionine | Decrease | 6.046E-04 | 3.144E-04 | 0.520 |
| 52 | Fructose 6-phosphate | Increase | 3.522E-04 | 1.463E-03 | 4.154 |
| 53 | Glutamylglutamic acid | Decrease | 1.263E-02 | 8.559E-03 | 0.678 |
| 54 | Cysteine | Decrease | 5.005E-05 | 3.658E-05 | 0.731 |
| 55 | N-Acetylglucosamine 1-phosphate | Increase | 4.100E-05 | 1.663E-04 | 4.056 |
| 56 | Ribose 5-phosphate | Increase | 2.590E-04 | 2.898E-04 | 1.119 |
| 57 | Glucose 1-phosphate | Increase | 2.033E-04 | 1.094E-03 | 5.380 |
| 58 | 3'-Cytidylic acid and 2'-Cytidylic acid | Increase | 2.590E-05 | 2.609E-04 | 10.072 |
| 59 | Dihydroxyacetone phosphate | Increase | 1.937E-04 | 3.579E-04 | 1.848 |
| 60 | 4-Oxovaleric acid | Decrease | 1.761E-04 | 0 | 0 |
| 61 | 2-Deoxyglucose 6-phosphate | Increase | 6.826E-06 | 4.902E-05 | 7.181 |
| 62 | Glucosamine 6-phosphate | Increase | 1.007E-05 | 4.001E-05 | 3.973 |
| 63 | 3-Methyladipic acid | Decrease | 3.244E+08 | 9.503E+07 | 0.293 |
| 64 | 2-Hydroxyquinoline | Decrease | 3.412E+07 | 2.291E+07 | 0.672 |
| 65 | 7-(tert-butyl)-4-imino-1,2,3,4,5,6-hexahydropyrido[3,4-d] pyridazine-1,5-dione) | Decrease | 4.268E+08 | 2.205E+08 | 0.517 |
| 66 | 2,4-dihydroxyheptadec-16-en-1-yl acetate | Decrease | 1.542E+07 | 7.270E+06 | 0.472 |

[Table 19-4]

| Table 19 (Continued) | | | | | |
|---|---|---|---|---|---|
| No. | Substance Name | Tendency in positive group | Negative group average value | Positive group average value | Positive group average value / Negative group average value |
| 67 | $\alpha$-Aspartylphenylalanine | Decrease | 1.317E+07 | 7.338E+06 | 0.557 |
| 68 | N1-[4-(1,3-Oxazol-5-yl)phe-nyl]cyclopropane-1-carboxa-mide | Increase | 8.458E+07 | 1.382E+08 | 1.634 |
| 69 | 1-(2,4-Dihydroxyquinolin-3-yl) ethan-1-one | Decrease | 4.671E+07 | 1.908E+06 | 0.041 |
| 70 | 4-(Diethylamino)salicylaldehyde | Increase | 2.310E+07 | 4.414E+07 | 1.911 |
| 71 | Atenolol | Decrease | 2.453E+08 | 1.135E+08 | 0.463 |
| 72 | 5-Hydroxyindole | Increase | 1.608E+08 | 4.365E+08 | 2.715 |
| 73 | 6-Methylquinoline | Increase | 3.928E+07 | 5.577E+07 | 1.420 |
| 74 | Guanine | Decrease | 4.546E+08 | 1.784E+08 | 0.392 |
| 75 | Carboxyibuprofen | Decrease | 4.110E+06 | 4.467E+05 | 0.109 |
| 76 | 3-Isopropylmalic acid | Decrease | 5.514E+07 | 2.522E+07 | 0.457 |
| 77 | Bis(methylbenzylidene)sorbitol | Decrease | 3.210E+09 | 2.303E+09 | 0.717 |
| 78 | 2-Furoic acid | Decrease | 3.354E+06 | 1.932E+06 | 0.576 |
| 79 | Orotic acid | Increase | 7.523E+07 | 1.174E+08 | 1.561 |
| 80 | 2-Acetamido-N-$\beta$-aspartyl-2-deoxyhexopyranosylamine | Decrease | 2.061E+07 | 1.187E+07 | 0.576 |
| 81 | Xanthosine | Increase | 1.200E+06 | 2.609E+06 | 2.174 |
| 82 | 1-propyl-1H-benzo[d]imidazole hydrobromide | Increase | 3.845E+06 | 1.250E+07 | 3.251 |
| 83 | 4-Phenolsulfonic acid | Decrease | 3.354E+08 | 2.555E+07 | 0.076 |
| 84 | Xanthohumol | Decrease | 3.625E+07 | 1.938E+07 | 0.535 |
| 85 | Laurolactam | Decrease | 2.735E+08 | 2.005E+08 | 0.733 |
| 86 | Adenine | Decrease | 1.195E+08 | 2.289E+07 | 0.192 |

[Table 19-5]

| Table 19 (Continued) | | | | | |
|---|---|---|---|---|---|
| No. | Substance Name | Tendency in positive group | Negative group average value | Positive group average value | Positive group average value / Negative group average value |
| 87 | 2'-Deoxyguanosine | Decrease | 2.567E+08 | 1.025E+08 | 0.399 |
| 88 | 6-Hydroxypicolinic acid | Decrease | 1.103E+07 | 7.936E+06 | 0.719 |
| 89 | D-(-)-Quinic acid | Decrease | 7.612E+08 | 1.543E+08 | 0.203 |
| 90 | 4-{[(4,6-Dimethoxypyrimidin-2-yl) amino]methylidene}-2-phenyl-4,5-dihydro-1,3-oxazol-5-one | Decrease | 1.558E+07 | 5.978E+05 | 0.038 |

(continued)

| | Table 19 (Continued) | | | | |
|---|---|---|---|---|---|
| No. | Substance Name | Tendency in positive group | Negative group average value | Positive group average value | Positive group average value / Negative group average value |
| 91 | 4-[(6E)-3-Hydroxy-8,10-di-methyl-2-(methylamino)-6-dode-cen-1-yl] phenol | Decrease | 5.198E+06 | 3.086E+06 | 0.594 |
| 92 | Ethyl 2-cyano-3-(tetrahydro-3-thiophenylamino)acrylate | Decrease | 1.173E+09 | 7.820E+08 | 0.667 |
| 93 | 3-Succinoylpyridine | Decrease | 6.562E+06 | 4.675E+06 | 0.712 |
| 94 | Proline | Decrease | 3.959E+08 | 1.386E+08 | 0.350 |
| 95 | L-threo-3-Phenylserine | Increase | 1.004E+08 | 2.531E+08 | 2.522 |
| 96 | (2,4-Diamino-5-pyrimidinyl)(3,4,5-trimethoxyphenyl)methanone | Decrease | 7.003E+06 | 3.359E+06 | 0.480 |
| 97 | 3-Morpholino-4-tetrahydro-1H-pyrrol-1-ylcyclobut-3-ene-1,2-dione | Decrease | 3.256E+07 | 2.131E+07 | 0.654 |
| 98 | 16-Hydroxyhexadecanoic acid | Decrease | 7.826E+09 | 3.410E+09 | 0.436 |
| 99 | Syringic acid | Decrease | 2.939E+07 | 1.004E+07 | 0.342 |
| 100 | Tranexamic acid | Decrease | 5.174E+09 | 1.089E+07 | 0.002 |
| 101 | 4-(tert-butyl)phenyl 3,5-dimethylisoxazole-4-carboxylate | Decrease | 4.257E+08 | 2.475E+08 | 0.581 |
| 102 | 3-Methylhistidine | Decrease | 3.352E+08 | 1.407E+08 | 0.420 |
| 103 | 5-Methoxyindole | Increase | 9.323E+05 | 1.392E+07 | 14.932 |
| 104 | Perillic acid | Decrease | 7.929E+06 | 2.974E+06 | 0.375 |

[Table 19-6]

| | Table 19 (Continued) | | | | |
|---|---|---|---|---|---|
| No. | Substance Name | Tendency in positive group | Negative group average value | Positive group average value | Positive group average value / Negative group average value |
| 105 | 6-Methyl[1,2,4]triazolo[4,3-b]pyridazin-8-ol | Increase | 1.321E+08 | 1.827E+08 | 1.383 |
| 106 | Piperonylonitrile | Increase | 3.044E+06 | 5.686E+06 | 1.868 |
| 107 | N1-(5-Chloro-2-hydroxyphenyl)-2-(morpholinomethyl)benzamide | Decrease | 6.951E+06 | 2.264E+06 | 0.326 |
| 108 | N-Methylhistamine | Decrease | 4.825E+06 | 2.247E+06 | 0.466 |
| 109 | Dibutylone | Decrease | 2.938E+07 | 1.039E+07 | 0.354 |
| 110 | (-)-Epigallocatechin | Decrease | 2.596E+07 | 4.803E+06 | 0.185 |
| 111 | Myriocin | Decrease | 9.383E+06 | 4.406E+06 | 0.470 |
| 112 | 4-Indolecarbaldehyde | Increase | 1.153E+08 | 2.062E+08 | 1.789 |
| 113 | Uracil | Increase | 9.551E+07 | 1.509E+08 | 1.580 |
| 114 | Dihydrothymine | Decrease | 7.509E+06 | 4.249E+06 | 0.566 |

(continued)

| Table 19 (Continued) | | | | | |
|---|---|---|---|---|---|
| No. | Substance Name | Tendency in positive group | Negative group average value | Positive group average value | Positive group average value / Negative group average value |
| 115 | 3-Methylglutaric acid | Decrease | 1.504E+09 | 2.669E+08 | 0.177 |
| 116 | 1,5-Anhydro-D-glucitol | Decrease | 2.744E+08 | 5.579E+07 | 0.203 |
| 117 | Valethamate | Decrease | 1.184E+06 | 9.475E+05 | 0.801 |
| 118 | DL-Arginine | Decrease | 6.854E+08 | 1.494E+08 | 0.218 |
| 119 | (R)-Equol | Increase | 1.002E+07 | 3.364E+07 | 3.356 |
| 120 | Theobromine | Decrease | 9.967E+07 | 5.903E+07 | 0.592 |
| 121 | 1,5-Isoquinolinediol | Decrease | 3.667E+07 | 4.767E+06 | 0.130 |
| 122 | 12-Hydroxydodecanoic acid | Decrease | 1.252E+08 | 8.007E+07 | 0.639 |
| 123 | 1,3,5-trihydroxy-4-{[(2E)-3-(3-hydroxy-4-methoxyphenyl)prop-2-enoyl]oxy}cyclohexane-1-carboxylic acid | Decrease | 1.995E+07 | 1.475E+07 | 0.740 |
| 124 | 3-Hydroxypicolinic acid | Decrease | 6.929E+07 | 5.222E+07 | 0.754 |

[Table 19-7]

| Table 19 (Continued) | | | | | |
|---|---|---|---|---|---|
| No. | Substance Name | Tendency in positive group | Negative group average value | Positive group average value | Positive group average value / Negative group average value |
| 125 | Theophylline | Decrease | 1.682E+08 | 1.174E+08 | 0.698 |
| 126 | 3,4-Dihydroxybenzaldehyde | Decrease | 3.294E+06 | 2.157E+06 | 0.655 |
| 127 | Acetylcholine | Increase | 2.391E+09 | 7.132E+09 | 2.983 |
| 128 | Tridecylic acid | Increase | 4.309E+08 | 6.294E+08 | 1.461 |

(3) Combined variable (statistical analysis value)

(3-1) Substances of No. 1 to No. 4 in Group A

For two or more substances selected from the substances of No. 1 to No. 4 in Group A (formylanthranilic acid, diethanolamine, glycerol, and 5,6-dihydroxyindole), a combined variable was determined by using the relative area values obtained in (2) above with an expression shown below, an ROC curve was prepared on the basis of the combined variable determined, and AUC and a cutoff value were determined from the ROC curve prepared. Table 20 shows the results. The coefficients and constant term were determined through multinomial logistic regression analysis. The coefficients and constant term are as shown in Table 3. In Table 20, "CFD_A" refers to formylanthranilic acid, "CFD_B" refers to diethanolamine, "CFD_C" refers to glycerol, and "CFD_D" refers to 5,6-dihydroxyindole.

Statistical analysis value = (Coefficient 1 × (Concentration of first substance)) + (Coefficient 2 × (Concentration of second substance)) +...+ (Coefficient n × (Concentration of n-th substance)) + Constant term

As demonstrated in Table 20, it was revealed that an ROC curve with increased AUC is successfully given, that is, enhanced accuracy of evaluating intestinal barrier function is successfully achieved by combining two or more substances selected from the substances of No. 1 to No. 4 in Group A.

[Table 20]

| Table 20 (No. 1 to No. 4 in Group A) | | | | | | |
|---|---|---|---|---|---|---|
| Number of substances combined | First substance | Second substance | Third substance | Fourth substance | AUC | Cutoff value |
| 2 | CFD_A | CFD_B | - | - | 0.902 | -0.3002 |
| 2 | CFD_A | CFD_C | - | - | 0.865 | 0.0797 |
| 2 | CFD_A | CFD_D | - | - | 0.818 | 0.2544 |
| 2 | CFD_B | CFD_C | - | - | 0.802 | -0.1633 |
| 2 | CFD_B | CFD_D | - | - | 0.823 | -0.0437 |
| 2 | CFD_C | CFD_D | - | - | 0.840 | -0.3603 |
| 3 | CFD_A | CFD_B | CFD_C | - | 0.905 | 0.1550 |
| 3 | CFD_A | CFD_B | CFD_D | - | 0.897 | 0.2333 |
| 3 | CFD_A | CFD_C | CFD_D | - | 0.893 | -0.0331 |
| 3 | CFD_B | CFD_C | CFD_D | - | 0.875 | 0.2835 |
| 4 | CFD_A | CFD_B | CFD_C | CFD_D | 0.927 | 0.1220 |

(3-2) Substances of No. 63 to No. 66 in Group A

For two or more substances selected from the substances of No. 63 to No. 66 in Group A (3-methyladipic acid, 2-hydroxyquinoline, 7-(tert-butyl)-4-imino-1,2,3,4,5,6-hexahydropyrido[3,4-d]pyridazine-1,5-dione, and 2,4-dihyroxyheptadec-16-en-1-yl acetate), a combined variable was determined by using the relative area values obtained in (2) above in the same manner as in (3-1) above, an ROC curve was prepared on the basis of the combined variable determined, and AUC and a cutoff value were determined from the ROC curve prepared. Table 21 shows the results. The coefficients and constant term were determined through multinomial logistic regression analysis. The coefficients and constant term are as shown in Table 4. In Table 21, "LFD_A" refers to 3-methyladipic acid, "LFD_B" refers to 2-hydroxyquinoline, "LFD_C" refers to 7-(tert-butyl)-4-imino-1,2,3,4,5,6-hexahydropyrido[3,4-d]pyridazine-1,5-dione, and "LFD_D" refers to 2,4-dihydroxyheptadec-16-en-1-yl acetate.

As demonstrated in Table 21, it was revealed that an ROC curve with increased AUC is successfully given, that is, enhanced accuracy of evaluating intestinal barrier function is successfully achieved by combining two or more substances selected from the substances of No. 63 to No. 66 in Group A.

[Table 21]

| Table 21 (No. 63 to No. 66 in Group A) | | | | | | |
|---|---|---|---|---|---|---|
| Number of substances combined | First substance | Second substance | Third substance | Fourth substance | AUC | Cutoff value |
| 2 | LFD_A | LFD_B | - | - | 0.776 | -0.5638 |
| 2 | LFD_A | LFD_C | - | - | 0.872 | -0.2997 |
| 2 | LFD_A | LFD_D | - | - | 0.853 | -0.6363 |
| 2 | LFD_B | LFD_C | - | - | 0.771 | 0.1345 |
| 2 | LFD_B | LFD_D | - | - | 0.805 | -0.1973 |
| 2 | LFD_C | LFD_D | - | - | 0.828 | -0.0680 |
| 3 | LFD_A | LFD_B | LFD_C | - | 0.873 | -0.3814 |
| 3 | LFD_A | LFD_B | LFD_D | - | 0.859 | -0.7610 |
| 3 | LFD_A | LFD_C | LFD_D | - | 0.912 | -0.5532 |
| 3 | LFD_B | LFD_C | LFD_D | - | 0.806 | -0.1248 |

(continued)

| Table 21 (No. 63 to No. 66 in Group A) | | | | | | |
|---|---|---|---|---|---|---|
| Number of substances combined | First substance | Second substance | Third substance | Fourth substance | AUC | Cutoff value |
| 4 | LFD_A | LFD_B | LFD_C | LFD_D | 0.929 | -0.5374 |

(3-3) Substances of No. 1 to No. 4 in Group B

For two or more substances selected from the substances of No. 1 to No. 4 in Group B (formylanthranilic acid, glycerol, formiminoglutamic acid, and 5,6-dihydroxyindole), a combined variable was determined by using the relative area values obtained in (1) above in the same manner as in (3-1) above, an ROC curve was prepared on the basis of the combined variable determined, and AUC and a cutoff value were determined from the ROC curve prepared. Table 22 shows the results. The coefficients and constant term were determined through multinomial logistic regression analysis. The coefficients and constant term are as shown in Table 5. In Table 22, "CFW_A" refers to formylanthranilic acid, "CFW_B" refers to glycerol, "CFW_C" refers to formiminoglutamic acid, and "CFW_D" refers to 5,6-dihydroxyindole.
As demonstrated in Table 22, it was revealed that an ROC curve with increased AUC is successfully given, that is, enhanced accuracy of evaluating intestinal barrier function is successfully achieved by combining two or more substances selected from the substances of No. 1 to No. 4 in Group B.

[Table 22]

| Table 22 (No. 1 to No. 4 in Group B) | | | | | | |
|---|---|---|---|---|---|---|
| Number of substances combined | First substance | Second substance | Third substance | Fourth substance | AUC | Cutoff value |
| 2 | CFW_A | CFW_B | - | - | 0.855 | 0.0409 |
| 2 | CFW_A | CFW_C | - | - | 0.816 | 0.2945 |
| 2 | CFW_A | CFW_D | - | - | 0.829 | 0.6941 |
| 2 | CFW_B | CFW_C | - | - | 0.788 | 0.2897 |
| 2 | CFW_B | CFW_D | - | - | 0.815 | 0.2488 |
| 2 | CFW_C | CFW_D | - | - | 0.788 | 0.2742 |
| 3 | CFW_A | CFW_B | CFW_C | - | 0.859 | 0.2086 |
| 3 | CFW_A | CFW_B | CFW_D | - | 0.879 | 0.2846 |
| 3 | CFW_A | CFW_C | CFW_D | - | 0.826 | 0.2070 |
| 3 | CFW_B | CFW_C | CFW_D | - | 0.880 | 0.1500 |
| 4 | CFW_A | CFW_B | CFW_C | CFW_D | 0.899 | 0.0357 |

(3-4) Substances of No. 46 to No. 49 in Group B

For two or more substances selected from the substances of No. 46 to No. 49 in Group B (6-methylquinoline, N1-[4-(1,3-oxazol-5-yl)phenyl]cyclopropane-1-carboxamide, 2-hydroxyquinoline, and 3-methyladipic acid), a combined variable was determined by using the relative area values obtained in (1) above in the same manner as in (3-1) above, and AUC and a cutoff value were determined from the ROC curve prepared. Table 23 shows the results. The coefficients and constant term were determined through multinomial logistic regression analysis. The coefficients and constant term are as shown in Table 6. In Table 23, "LFW_A" refers to 6-methylquinoline, "LFW_B" refers to N1-[4-(1,3-oxazol-5-yl)phenyl]cyclopropane-1-carboxamide, "LFW_C" refers to 2-hydroxyquinoline, and "LFW_D" refers to 3-methyladipic acid.
As demonstrated in Table 23, it was revealed that an ROC curve with increased AUC is successfully given, that is, enhanced accuracy of evaluating intestinal barrier function is successfully achieved by combining two or more substances selected from the substances of No. 46 to No. 49 in Group B.

[Table 23]

| Table 23 (No. 46 to No. 49 in Group B) | | | | | | |
|---|---|---|---|---|---|---|
| Number of substances combined | First substance | Second substance | Third substance | Fourth substance | AUC | Cutoff value |
| 2 | LFW_A | LFW_B | - | - | 0.776 | 0.2831 |
| 2 | LFW_A | LFW_C | - | - | 0.796 | 0.1288 |
| 2 | LFW_A | LFW_D | - | - | 0.855 | 0.4390 |
| 2 | LFW_B | LFW_C | - | - | 0.748 | 0.0309 |
| 2 | LFW_B | LFW_D | - | - | 0.788 | -0.6398 |
| 2 | LFW_C | LFW_D | - | - | 0.764 | -0.4317 |
| 3 | LFW_A | LFW_B | LFW_C | - | 0.798 | 0.1412 |
| 3 | LFW_A | LFW_B | LFW_D | - | 0.849 | -0.0486 |
| 3 | LFW_A | LFW_C | LFW_D | - | 0.862 | 0.4135 |
| 3 | LFW_B | LFW_C | LFW_D | - | 0.771 | -0.3123 |
| 4 | LFW_A | LFW_B | LFW_C | LFW_D | 0.865 | 0.1091 |

**Claims**

1. A method for evaluating intestinal barrier function in a subject, the method comprising the steps of:

(1a) measuring a biomarker in a specimen derived from the subject to acquire a measurement value for the biomarker;
(1b) comparing the measurement value acquired in step 1a with a reference value to acquire a comparison result; and
(1c) evaluating intestinal barrier function in the subject on the basis of the comparison result acquired in step 1b, wherein the specimen is dry feces or wet feces,
wherein when the specimen is dry feces, the biomarker comprises one or more substances selected from Group A consisting of:

formylanthranilic acid;
diethanolamine;
glycerol;
5,6-dihydroxyindole;
formiminoglutamic acid;
leucine;
myristoleic acid;
3-aminopropane-1,2-diol;
4-guanidinobutyric acid;
phenylalanine;
2'-deoxyadenosine and 5'-deoxyadenosine;
lipoamide;
3-guanidinopropionic acid;
acetanilide;
histidine;
γ-glutamyl-2-aminobutyric acid;
methionine sulfoxide;
phenylhydrazine;
tryptophan;
tyrosine;
hydroxyindole;

alanine;

p-aminobenzoic acid;

penicillamine;

2-cyanopyridine;

glutamine;

2,4-diaminobutyric acid;

trigonelline;

2-methylserine;

glycylglycine;

N,N-dimethylhistidine;

valine;

N-acetylphenylalanine;

isoleucine;

methionine;

γ-glutamyl-valyl-glycine;

N-acetylaspartic acid;

threonic acid;

alloisoleucine;

arginine ethyl ester;

2,6-diaminopimelic acid;

glucose 6-phosphate;

adenosine;

mucic acid;

thymidine;

glycocholic acid;

2-deoxyribose 1-phosphate;

isoleucyl-prolyl-proline;

sedoheptulose 7-phosphate;

fumaric acid;

N-acetylmethionine;

fructose 6-phosphate;

glutamylglutamic acid;

cysteine;

N-acetylglucosamine 1-phosphate;

ribose 5-phosphate;

glucose 1-phosphate;

3'-cytidylic acid and 2'-cytidylic acid;

dihydroxyacetone phosphate;

4-oxovaleric acid;

2-deoxyglucose 6-phosphate;

glucosamine 6-phosphate;

3-methyladipic acid;

2-hydroxyquinoline;

7-(tert-butyl)-4-imino-1,2,3,4,5,6-hexahydropyrido[3,4-d]pyridazine-1,5-dione;

2,4-dihydroxyheptadec-16-en-1-yl acetate;

α-spartylphenylalanine;

N1-[4-(1,3-oxazol-5-yl)phenyl]cyclopropane-1-carboxamide;

1-(2,4-dihydroxyquinolin-3-yl)ethan-1-one;

4-(diethylamino)salicylaldehyde;

atenolol;

5-hydroxyindole;

6-methylquinoline;

guanine;

carboxyibuprofen;

3-isopropylmalic acid;

bis(methylbenzylidene)sorbitol;

2-furoic acid;

orotic acid;

2-acetamido-N-β-aspartyl-2-deoxyhexopyranosylamine;

xanthosine;

1-propyl-1H-benzo[d]imidazole hydrobromide;

4-phenolsulfonic acid;

xanthohumol;

laurolactam;

adenine;

2'-deoxyguanosine;

6-hydroxypicolinic acid;

D-(-)-quinic acid;

4-{[(4,6-dimethoxypyrimidin-2-yl)amino]methylidene}-2-phenyl-4,5-dihydro-1,3-oxazol-5-one;

4-[(6E)-3-hydroxy-8,10-dimethyl-2-(methylamino)-6-dodecen-1-yl]phenol;

ethyl 2-cyano-3-(tetrahydro-3-thiophenylamino)acrylate;

3-succinoylpyridine;

proline;

L-threo-3-phenylserine;

(2,4-diamino-5-pyrimidinyl)(3,4,5-trimethoxyphenyl)methanone;

3-morpholino-4-tetrahydro-1H-pyrrol-1-ylcyclobut-3-ene-1,2-dione;

16-hydroxyhexadecanoic acid;

syringic acid;

tranexamic acid;

4-(tert-butyl)phenyl 3,5-dimethylisoxazole-4-carboxylate;

3-methylhistidine;

5-methoxyindole;

perillic acid;

6-methyl[1,2,4]triazolo[4,3-b]pyridazin-8-ol;

piperonylonitrile;

N1-(5-chloro-2-hydroxyphenyl)-2-(morpholinomethyl)benzamide;

N-methyl histamine;

dibutylone;

(-)-epigallocatechin;

myriocin;

4-indolecarbaldehyde;

uracil;

dihydrothymine;

3-methylglutaric acid;

1,5-anhydro-D-glucitol;

valethamate;

DL-arginine;

(R)-equol;

theobromine;

1,5-isoquinolinediol;

12-hydroxydodecanoic acid;

1,3,5-trihydroxy-4-{[(2E)-3-(3-hydroxy-4-methoxyphenyl)prop-2-enoyl]oxy}cyclohexane-1-carboxylic acid;

3-hydroxypicolinic acid;

theophylline;

3,4-dihydroxybenzaldehyde;

acetylcholine; and

tridecylic acid, and

wherein when the specimen is wet feces, the biomarker comprises one or more substances selected from Group B consisting of:

formylanthranilic acid;

glycerol;

formiminoglutamic acid;

5,6-dihydroxyindole;

3-aminopropane-1,2-diol;

4-guanidinobutyric acid;

2'-deoxyadenosine and 5'-deoxyadenosine;

acetanilide;

pyrophosphate;

myristoleic acid;

diethanolamine;

thiaproline;

hydroxyindole;

2,6-diaminopimelic acid;

p-aminobenzoic acid;

lipoamide;

phenylhydrazine;

3-guanidinopropionic acid;

N-acetylaspartic acid;

2,4-diaminobutyric acid;

γ-glutamyl-2-aminobutyric acid;

cysteine;

glycerol 3-phosphate;

glucose 6-phosphate;

mucic acid;

trigonelline;

N-carbamoylaspartic acid;

threonic acid;

glycocholic acid;

isobutyric acid and butyric acid;

2-deoxyribose 1-phosphate;

arginine ethyl ester;

N-methylglutamic acid;

pterin;

N-acetylphenylalanine;

ribulose 5-phosphate;

fructose 6-phosphate;

sedoheptulose 7-phosphate;

N-acetylglucosamine 1-phosphate;

ribose 5-phosphate;

3'-cytidylic acid and 2'-cytidylic acid;

dihydroxyacetone phosphate;

4-oxovaleric acid;

propranolol;

2-deoxyglucose 6-phosphate;

6-methylquinoline;

N1-[4-(1,3-oxazol-5-yl)phenyl]cyclopropane-1-carboxamide;

2-hydroxyquinoline;

3-methyladipic acid;

L-threo-3-phenylserine;

4-(diethylamino)salicylaldehyde;

5-methoxyindole;

7-(tert-butyl)-4-imino-1,2,3,4,5,6-hexahydropyrido[3,4-d]pyridazine-1,5-dione;

4-indolecarbaldehyde;

carboxyibuprofen;

piperonylonitrile;

5-hydroxyindole;

orotic acid;

glucose 1-phosphate;

L-alanyl-L-proline;

2-acetamido-N-β-aspartyl-2-deoxyhexopyranosylamine;

α-spartylphenylalanine;

6-hydroxymelatonin;

xanthosine;

atenolol;
(R)-equol;
2,4-dihydroxyheptadec-16-en-1-yl acetate;
guanine;
1-(4-methyl-2-pyridyl)pyrrolidine-2,5-dione;
laurolactam;
olmesartan;
adenine;
uracil;
6-methyl[1,2,4]triazolo[4,3-b]pyridazin-8-ol;
1-propyl-1H-benzo[d]imidazole hydrobromide;
pseudouridine;
D-(-)-quinic acid;
acetylcholine;
3-isopropylmalic acid;
2-methoxy-5-(1H-1,2,4-triazol-5-yl)-4-(trifluoromethyl)pyridine;
3-amino-2-naphthoic acid;
ornithine;
2'-deoxyguanosine;
bis(methylbenzylidene)sorbitol;
2-furoic acid;
4-(tert-butyl)phenyl 3,5-dimethylisoxazole-4-carboxylate;
pyridoxamine;
tridecylic acid;
3-methylhistidine;
hexanoic acid;
azobenzene;
(-)-epigallocatechin;
3-(2-thienyl)-1,2,4-oxadiazole-5-carbohydrazide;
DL-lactic acid;
6-hydroxypicolinic acid;
proline;
1,3,5-trihydroxy-4-{[(2E)-3-(3-hydroxy-4-methoxyphenyl)prop-2-enoyl]oxy}cyclohexane-1-carboxylic acid;
prolylglycine; and
4-[(6E)-3-hydroxy-8,10-dimethyl-2-(methylamino)-6-dodecen-1-yl]phenol.

2. A method for diagnosing a disease or symptom caused by reduction in intestinal barrier function in a subject, the method comprising the steps of:

(2a) measuring a biomarker in a specimen derived from the subject to acquire a measurement value for the biomarker;
(2b) comparing the measurement value acquired in step 2a with a reference value to acquire a comparison result; and
(2c) diagnosing the disease or symptom on the basis of the comparison result acquired in step 2b,
wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

3. A method for treating a disease or symptom caused by reduction in intestinal barrier function in a subject, the method comprising the steps of:

(3a) measuring a biomarker in a specimen derived from the subject to acquire a measurement value for the biomarker;
(3b) comparing the measurement value acquired in step 3a with a reference value to acquire a comparison result;
(3c) diagnosing the disease or symptom on the basis of the comparison result acquired in step 3b; and

(3d) administering a substance or composition for treating the disease or symptom to the subject if the subject is diagnosed to be affected by the disease or symptom,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

4. A method for evaluating a subject's risk of developing a disease or symptom caused by reduction in intestinal barrier function, or probability of the subject being affected by the disease or symptom, the method comprising the steps of:

(4a) measuring a biomarker in a specimen derived from the subject to acquire a measurement value for the biomarker;

(4b) comparing the measurement value acquired in step 4a with a reference value to acquire a comparison result; and

(4c) evaluating the risk or the probability on the basis of the comparison result acquired in step 4b,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

5. A method for acquiring data for diagnosing a disease or symptom caused by reduction in intestinal barrier function in a subject, the method comprising the steps of:

(5a) measuring a biomarker in a specimen derived from the subject to acquire a measurement value for the biomarker; and

(5b) comparing the measurement value acquired in step 5a with a reference value to acquire a comparison result as the data,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

6. A method for screening for a substance or composition having an improving effect on intestinal barrier function or a preventing or ameliorating effect on a disease or symptom caused by reduction in intestinal barrier function, the method comprising the steps of:

(6a) administering a candidate substance or candidate composition to a subject having reduced intestinal barrier function;

(6b) measuring a biomarker in a specimen obtained from the subject after administration of the candidate substance or candidate composition to acquire a measurement value for the biomarker;

(6c) comparing the measurement value acquired in step 6b with a reference value to acquire a comparison result; and

(6d) evaluating the candidate substance or candidate composition for the improving effect on intestinal barrier function or the preventing or ameliorating effect on the disease or symptom on the basis of the comparison result acquired in step 6c,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

7. A method for evaluating a candidate substance or candidate composition for an improving effect on intestinal barrier function or a preventing or ameliorating effect on a disease or symptom caused by reduction in intestinal barrier function, the method comprising the steps of:

(7a) administering the candidate substance or candidate composition to a subject having reduced intestinal barrier function;

(7b) measuring a biomarker in a specimen obtained from the subject after administration of the candidate substance or candidate composition to acquire a measurement value for the biomarker;

(7c) comparing the measurement value acquired in step 7b with a reference value to acquire a comparison result; and

(7d) evaluating the candidate substance or candidate composition for the improving effect on intestinal barrier function or the preventing or ameliorating effect on the disease or symptom on the basis of the comparison result acquired in step 7c,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

8. The method according to any one of claims 1 to 7, wherein the intestinal barrier function is barrier function of a small intestine.

9. The method according to any one of claims 1 to 7, wherein when the specimen is dry feces, the biomarker comprises two, three, or four or more substances selected from Group A, and wherein when the specimen is wet feces, the biomarker comprises two, three, or four or more substances selected from Group B.

10. The method according to any one of claims 1 to 7, wherein when the specimen is dry feces, the biomarker comprises one or more substances selected from the group consisting of formylanthranilic acid, diethanolamine, glycerol, and 5,6-dihydroxyindole, and wherein when the specimen is wet feces, the biomarker comprises one or more substances selected from the group consisting of formylanthranilic acid, glycerol, formiminoglutamic acid, and 5,6-dihydroxyindole.

11. The method according to claim 10, wherein the biomarker in the specimen is measured by capillary electrophoresis-mass spectrometry.

12. The method according to any one of claims 1 to 7, wherein when the specimen is dry feces, the biomarker comprises one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker comprises one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

13. The method according to claim 12, wherein the biomarker in the specimen is measured by liquid chromatography-mass spectrometry.

14. A biomarker in a specimen derived from a subject for use in evaluating intestinal barrier function in the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

15. A biomarker in a specimen derived from a subject for use in diagnosing a disease or symptom caused by reduction in intestinal barrier function in the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

16. A biomarker in a specimen derived from a subject for use in evaluating the subject's risk of developing a disease or symptom caused by reduction in intestinal barrier function, or probability of the subject being affected by the disease or symptom,

>   wherein the specimen is dry feces or wet feces,
>   wherein when the specimen is dry feces, the biomarker comprises one or more substances selected from Group A specified in claim 1, and
>   wherein when the specimen is wet feces, the biomarker comprises one or more substances selected from Group B specified in claim 1.

17. A biomarker in a specimen derived from a subject for use in acquiring data for diagnosing a disease or symptom caused by reduction in intestinal barrier function in the subject,

>   wherein the specimen is dry feces or wet feces,
>   wherein when the specimen is dry feces, the biomarker comprises one or more substances selected from Group A specified in claim 1, and
>   wherein when the specimen is wet feces, the biomarker comprises one or more substances selected from Group B specified in claim 1.

18. A biomarker in a specimen for use in screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject,

>   wherein the specimen is dry feces or wet feces,
>   wherein when the specimen is dry feces, the biomarker comprises one or more substances selected from Group A specified in claim 1, and
>   wherein when the specimen is wet feces, the biomarker comprises one or more substances selected from Group B specified in claim 1.

19. A biomarker in a specimen for use in evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject,

>   wherein the specimen is dry feces or wet feces,
>   wherein when the specimen is dry feces, the biomarker comprises one or more substances selected from Group A specified in claim 1, and
>   wherein when the specimen is wet feces, the biomarker comprises one or more substances selected from Group B specified in claim 1.

20. The biomarker according to any one of claims 14 to 19, wherein the intestinal barrier function is barrier function of a small intestine.

21. The biomarker according to any one of claims 14 to 19, wherein when the specimen is dry feces, the biomarker comprises two, three, or four or more substances selected from the Group A, and wherein when the specimen is wet feces, the biomarker comprises two, three, or four or more substances selected from the Group B.

22. The biomarker according to any one of claims 14 to 19, wherein when the specimen is dry feces, the biomarker comprises one or more substances selected from the group consisting of formylanthranilic acid, diethanolamine, glycerol, and 5,6-dihydroxyindole, and wherein when the specimen is wet feces, the biomarker comprises one or more substances selected from the group consisting of formylanthranilic acid, glycerol, formiminoglutamic acid, and 5,6-dihydroxyindole.

23. The biomarker according to claim 22, wherein the biomarker in the specimen is measured by capillary electrophoresis-mass spectrometry.

24. The biomarker according to any one of claims 14 to 19, wherein when the specimen is dry feces, the biomarker comprises one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker comprises one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

25. The biomarker according to claim 24, wherein the biomarker in the specimen is measured by liquid chromatography-mass spectrometry.

26. Use of a biomarker in a specimen derived from a subject for evaluating intestinal barrier function in the subject,

wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

27. Use of a biomarker in a specimen derived from a subject for diagnosing a disease or symptom caused by reduction in intestinal barrier function in the subject,

wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

28. Use of a biomarker in a specimen derived from a subject for evaluating the subject's risk of developing a disease or symptom caused by reduction in intestinal barrier function, or probability of the subject being affected by the disease or symptom,

wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

29. Use of a biomarker in a specimen derived from a subject for acquiring data for diagnosing a disease or symptom caused by reduction in intestinal barrier function in the subject,

wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

30. Use of a biomarker in a specimen for screening for a substance or composition having an improving effect on intestinal barrier function or a preventing or ameliorating effect on a disease or symptom caused by reduction in intestinal barrier function, the specimen obtained from a subject having reduced intestinal barrier function after administration of a candidate substance or candidate composition to the subject,

wherein the specimen is dry feces or wet feces,
wherein when the specimen is dry feces, the biomarker comprises one or more substances selected from Group A specified in claim 1, and
wherein when the specimen is wet feces, the biomarker comprises one or more substances selected from Group B specified in claim 1.

31. Use of a biomarker in a specimen for evaluating a candidate substance or candidate composition for an improving effect on intestinal barrier function or a preventing or ameliorating effect on a disease or symptom caused by reduction in intestinal barrier function, the specimen obtained from a subject having reduced intestinal barrier function after administration of the candidate substance or candidate composition to the subject,

> wherein the specimen is dry feces or wet feces,
> wherein when the specimen is dry feces, the biomarker comprises one or more substances selected from Group A specified in claim 1, and
> wherein when the specimen is wet feces, the biomarker comprises one or more substances selected from Group B specified in claim 1.

32. The use according to any one of claims 26 to 31, wherein the intestinal barrier function is barrier function of a small intestine.

33. The use according to any one of claims 26 to 31, wherein when the specimen is dry feces, the biomarker comprises two, three, or four or more substances selected from the Group A, and wherein when the specimen is wet feces, the biomarker comprises two, three, or four or more substances selected from the Group B.

34. The use according to any one of claims 26 to 31, wherein when the specimen is dry feces, the biomarker comprises one or more substances selected from the group consisting of formylanthranilic acid, diethanolamine, glycerol, and 5,6-dihydroxyindole, and wherein when the specimen is wet feces, the biomarker comprises one or more substances selected from the group consisting of formylanthranilic acid, glycerol, formiminoglutamic acid, and 5,6-dihydroxyindole.

35. The use according to claim 34, wherein the biomarker in the specimen is measured by capillary electrophoresis-mass spectrometry.

36. The use according to any one of claims 26 to 31, wherein when the specimen is dry feces, the biomarker comprises one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker comprises one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

37. The use according to claim 36, wherein the biomarker in the specimen is measured by liquid chromatography-mass spectrometry.

38. A kit for evaluating intestinal barrier function in a subject,

> wherein the kit comprises one or more reagents for measuring a biomarker in a specimen derived from the subject,
> wherein the specimen is dry feces or wet feces,
> wherein when the specimen is dry feces, the biomarker comprises one or more substances selected from Group A specified in claim 1, and
> wherein when the specimen is wet feces, the biomarker comprises one or more substances selected from Group B specified in claim 1.

39. A kit for diagnosing a disease or symptom caused by reduction in intestinal barrier function in a subject,

> wherein the kit comprises one or more reagents for measuring a biomarker in a specimen derived from the subject,
> wherein the specimen is dry feces or wet feces,
> wherein when the specimen is dry feces, the biomarker comprises one or more substances selected from Group A specified in claim 1, and
> wherein when the specimen is wet feces, the biomarker comprises one or more substances selected from Group B specified in claim 1.

40. A kit for evaluating a subject's risk of developing a disease or symptom caused by reduction in intestinal barrier function, or probability of the subject being affected by the disease or symptom,

wherein the kit comprises one or more reagents for measuring a biomarker in a specimen derived from the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

41. A kit for acquiring data for diagnosing a disease or symptom caused by reduction in intestinal barrier function in a subject,

wherein the kit comprises one or more reagents for measuring a biomarker in a specimen derived from the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

42. A kit for screening for a substance or composition having an improving effect on intestinal barrier function or a preventing or ameliorating effect on a disease or symptom caused by reduction in intestinal barrier function,

wherein the kit comprises one or more reagents for measuring a biomarker in a specimen obtained from a subject having reduced intestinal barrier function after administration of a candidate substance or candidate composition to the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

43. A kit for evaluating a candidate substance or candidate composition for an improving effect on intestinal barrier function or a preventing or ameliorating effect on a disease or symptom caused by reduction in intestinal barrier function,

wherein the kit comprises one or more reagents for measuring a biomarker in a specimen obtained from a subject having reduced intestinal barrier function after administration of the candidate substance or candidate composition to the subject,

wherein the specimen is dry feces or wet feces,

wherein when the specimen is dry feces, the biomarker comprises one or more substances selected from Group A specified in claim 1, and

wherein when the specimen is wet feces, the biomarker comprises one or more substances selected from Group B specified in claim 1.

44. The kit according to any one of claims 38 to 43, wherein the intestinal barrier function is barrier function of a small intestine.

45. The kit according to any one of claims 38 to 43, wherein when the specimen is dry feces, the biomarker comprises two, three, or four or more substances selected from the Group A, and wherein when the specimen is wet feces, the biomarker comprises two, three, or four or more substances selected from the Group B.

46. The kit according to any one of claims 38 to 43, wherein when the specimen is dry feces, the biomarker comprises one or more substances selected from the group consisting of formylanthranilic acid, diethanolamine, glycerol, and 5,6-dihydroxyindole, and wherein when the specimen is wet feces, the biomarker comprises one or more substances selected from the group consisting of formylanthranilic acid, glycerol, formiminoglutamic acid, and 5,6-dihydroxyindole.

47. The kit according to claim 46, wherein the biomarker in the specimen is measured by capillary electrophoresis-mass spectrometry.

48. The kit according to any one of claims 38 to 43, wherein when the specimen is dry feces, the biomarker comprises one or more substances selected from the group consisting of 3-methyladipic acid, 2-hydroxyquinoline, 7-(tert-butyl)-4-imino-1,2,3,4,5,6-hexahydropyrido[3,4-d]pyridazine-1,5-dione, and 2,4-dihydroxyheptadec-16-en-1-yl acetate, and wherein when the specimen is wet feces, the biomarker comprises one or more substances selected from the group consisting of 6-methylquinoline, N1-[4-(1,3-oxazol-5-yl)phenyl]cyclopropane-1-carboxamide, 2-hydroxyquinoline, and 3-methyladipic acid.

49. The kit according to claim 48, wherein the biomarker in the specimen is measured by liquid chromatography-mass spectrometry.

50. A method for producing a food product or pharmaceutical composition, the method comprising the step of blending a substance or composition obtained through screening by the method according to claim 6 with one or two or more components to constitute the food product or pharmaceutical composition.

EP 4 679 087 A1

[Figure 1]

119

[Figure 2]

[Figure 3]

```
                    ┌─────────────┐
                    │    START    │
                    └──────┬──────┘
                           │
                           ▼
    ┌──────────────────────────────────────────┐
    │  Acquisition of measurement value for     │──── S101
    │            biomarker                      │
    └──────────────────────┬───────────────────┘
                           │
                           ▼
    ┌──────────────────────────────────────────┐
    │   Acquisition of comparison result between │──── S102
    │   measurement value and reference value    │
    └──────────────────────┬───────────────────┘
                           │
                           ▼
    ┌──────────────────────────────────────────┐
    │  Evaluation of intestinal barrier function │──── S103
    │             in subject                     │
    └──────────────────────┬───────────────────┘
                           │
                           ▼
                    ┌─────────────┐
                    │     END     │
                    └─────────────┘
```

[Figure 4]

[Figure 5]

## INTERNATIONAL SEARCH REPORT

| International application No. |
| --- |
| **PCT/JP2024/009043** |

**A. CLASSIFICATION OF SUBJECT MATTER**

*G01N 33/50*(2006.01)i
FI: G01N33/50 Z

According to International Patent Classification (IPC) or to both national classification and IPC

**B. FIELDS SEARCHED**

Minimum documentation searched (classification system followed by classification symbols)

G01N33/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | US 2019/0383831 A1 (NEWSOUTH INNOVATIONS PTY LTD.) 19 December 2019 (2019-12-19)<br>claim 1, paragraph [0594] | 1-50 |
| A | US 2017/0053081 A1 (THE ARIZONA BOARD OF REGENTS ON BEHALF OF THE UNIVERSITY OF ARIZONA) 23 February 2017 (2017-02-23)<br>claim 1, paragraph [0013] | 1-50 |
| A | US 2022/0003775 A1 (UNIVERSITEIT GENT) 06 January 2022 (2022-01-06)<br>claim 1, paragraph [0029] | 1-50 |
| A | US 2021/0003592 A1 (EVONIK OPERATIONS GMBH) 07 January 2021 (2021-01-07)<br>claim 12 | 1-50 |

☐ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 April 2024** | **16 April 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/009043**

| Patent document cited in search report | | | Publication date (day/month/year) | Patent family member(s) | | | Publication date (day/month/year) |
|---|---|---|---|---|---|---|---|
| US | 2019/0383831 | A1 | 19 December 2019 | WO | 2017/210751 | A1 | |
| | | | | EP | 3469357 | A1 | |
| US | 2017/0053081 | A1 | 23 February 2017 | WO | 2015/168405 | A1 | |
| US | 2022/0003775 | A1 | 06 January 2022 | WO | 2020/104577 | A1 | |
| | | | | EP | 3884062 | A1 | |
| US | 2021/0003592 | A1 | 07 January 2021 | US | 2021/0011027 | A1 | |
| | | | | WO | 2019/166534 | A1 | |
| | | | | WO | 2019/166531 | A1 | |
| | | | | EP | 3759495 | A1 | |
| | | | | EP | 3759496 | A1 | |
| | | | | CN | 111801579 | A | |
| | | | | CN | 111819444 | A | |
| | | | | KR | 10-2020-0128412 | A | |
| | | | | KR | 10-2020-0128413 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FASANA A et al.** a newly discovered modulator of intestinal permeability, and its expression in coeliac disease. *THE LANCET.*, 2000, vol. 355, 1518-1519
- **ARISTO VOJDANI et al.** Fluctuation of zonulin levels in blood vs stability of antibodies. *World Journal of Gastroenterology.*, 2017, vol. 23 (31), 5669-5679

- Metab Brain Dis. *PNAS*, 14 October 2003, vol. 100 (21), 12343-12348
- *Ann Transl Med*, 2019, vol. 7 (16), 1-10, http://dx.doi.org/10.21037/atm.2019.07.102